(19) **Europäisches Patentamt**
**European Patent Office**
**Office européen des brevets**

(11) **EP 3 108 254 B1**

(12) **EUROPEAN PATENT SPECIFICATION**

(45) Date of publication and mention
of the grant of the patent:
**12.12.2018 Bulletin 2018/50**

(51) Int Cl.:
***G01N 33/574*** *(2006.01)*

(21) Application number: **15704319.1**

(22) Date of filing: **16.02.2015**

(86) International application number:
**PCT/EP2015/053196**

(87) International publication number:
**WO 2015/121465 (20.08.2015 Gazette 2015/33)**

(54) **G-ALPHA, INTERACTING VESICLE ASSOCIATED PROTEIN (GIV) AS A PREDICTIVE MARKER IN STAGE II COLORECTAL CANCER**

G-ALPHA, MIT INTERAGIERENDEM VESIKEL ASSOZIIERTES PROTEIN (GIV) ALS PRÄDIKTIVER MARKER BEI DARMKREBS IM STADIUM II

PROTÉINE ASSOCIÉE AU VÉSICULE INTERAGISSANT AVEC G-ALPHA (GIV) UTILISÉE COMME MARQUEUR PRÉDICTIF D'UN CANCER COLORECTAL DE STADE II

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2014 US 201461940705 P**

(43) Date of publication of application:
**28.12.2016 Bulletin 2016/52**

(73) Proprietors:
• **Ventana Medical Systems, Inc.**
  **Tucson, Arizona 85755 (US)**
• **The Regents of the University of California**
  **Oakland, CA 94607 (US)**
• **The United States of America as Represented by the**
  **Deparment of Vetererans Affairs**
  **Washington, D.C. 20420 (US)**
• **F. Hoffmann-La Roche AG**
  **4070 Basel (CH)**

(72) Inventors:
• **LEITH, Katherine**
  **Tucson,**
  **Arizona 85719 (US)**
• **ROHR, Ulrich-Peter**
  **79540 Loerrach (DE)**
• **SINGH, Shalini**
  **Tucson,**
  **Arizona 85750 (US)**

• **GHOSH, Pradipta**
  **San Diego,**
  **California 92130 (US)**
• **HU, Song**
  **Pleasanton,**
  **California 94588 (US)**
• **LAFLEUR, Bonnie**
  **Tucson,**
  **Arizona 85745 (US)**
• **MURANYI, Andrea**
  **Tucson,**
  **Arizona 85718 (US)**
• **SHANMUGAM, Kandavel**
  **Chandler,**
  **Arizona 85226 (US)**

(74) Representative: **Teschemacher, Andrea**
**Isenbruck Bösl Hörschler LLP**
**Patentanwälte**
**Prinzregentenstraße 68**
**81675 München (DE)**

(56) References cited:
• **M. GARCIA-MARCOS ET AL: "Expression of GIV/Girdin, a metastasis-related protein, predicts patient survival in colon cancer", THE FASEB JOURNAL, vol. 25, no. 2, 25 October 2010 (2010-10-25), pages 590-599, XP055184165, ISSN: 0892-6638, DOI: 10.1096/fj.10-167304 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **JUN BYOUNG YEON ET AL: "Expression of girdin in human colorectal cancer and its association with tumor progression", DISEASES OF THE COLON & RECTUM, LIPPINCOTT WILLIAMS & WILKINS, US, vol. 56, no. 1, 1 January 2013 (2013-01-01), pages 51-57, XP008176038, ISSN: 1530-0358, DOI: 10.1097/DCR.0B013E31826B9B7E**

SEQUENCE LISTING

<110> Ventana Medical Systems, Inc. et al.

<120> G-ALPHA, INTERACTING VESICLE ASSOCIATED PROTEIN (GIV) AS A PREDICTIVE MARKER IN STAGE II COLORECTAL CANCER

<130> R69606PC

<150> US61/940,705
<151> 2014-02-17

<160> 2

<170> PatentIn version 3.5

<210> 1
<211> 1870

<212> PRT
<213> Homo sapiens

<400> 1

```
Met Glu Asn Glu Ile Phe Thr Pro Leu Leu Glu Gln Phe Met Thr Ser
1               5                   10                  15

Pro Leu Val Thr Trp Val Lys Thr Phe Gly Pro Leu Ala Ala Gly Asn
            20                  25                  30

Gly Thr Asn Leu Asp Glu Tyr Val Ala Leu Val Asp Gly Val Phe Leu
            35                  40                  45

Asn Gln Val Met Leu Gln Ile Asn Pro Lys Leu Glu Ser Gln Arg Val
        50                  55                  60

Asn Lys Lys Val Asn Asn Asp Ala Ser Leu Arg Met His Asn Leu Ser
65                  70                  75                  80

Ile Leu Val Arg Gln Ile Lys Phe Tyr Tyr Gln Glu Thr Leu Gln Gln
                85                  90                  95

Leu Ile Met Met Ser Leu Pro Asn Val Leu Ile Ile Gly Lys Asn Pro
                100                 105                 110

Phe Ser Glu Gln Gly Thr Glu Glu Val Lys Lys Leu Leu Leu Leu Leu
            115                 120                 125

Leu Gly Cys Ala Val Gln Cys Gln Lys Lys Glu Glu Phe Ile Glu Arg
        130                 135                 140

Ile Gln Gly Leu Asp Phe Asp Thr Lys Ala Ala Val Ala Ala His Ile
145                 150                 155                 160
```

```
Gln Glu Val Thr His Asn Gln Glu Asn Val Phe Asp Leu Gln Trp Met
                165                 170                 175

Glu Val Thr Asp Met Ser Gln Glu Asp Ile Glu Pro Leu Leu Lys Asn
                180                 185                 190

Met Ala Leu His Leu Lys Arg Leu Ile Asp Glu Arg Asp Glu His Ser
                195                 200                 205

Glu Thr Ile Ile Glu Leu Ser Glu Glu Arg Asp Gly Leu His Phe Leu
    210                 215                 220

Pro His Ala Ser Ser Ser Ala Gln Ser Pro Cys Gly Ser Pro Gly Met
225                 230                 235                 240

Lys Arg Thr Glu Ser Arg Gln His Leu Ser Val Glu Leu Ala Asp Ala
                245                 250                 255

Lys Ala Lys Ile Arg Arg Leu Arg Gln Glu Leu Glu Glu Lys Thr Glu
                260                 265                 270

Gln Leu Leu Asp Cys Lys Gln Glu Leu Glu Gln Met Glu Ile Glu Leu
                275                 280                 285

Lys Arg Leu Gln Gln Glu Asn Met Asn Leu Leu Ser Asp Ala Arg Ser
    290                 295                 300

Ala Arg Met Tyr Arg Asp Glu Leu Asp Ala Leu Arg Glu Lys Ala Val
305                 310                 315                 320

Arg Val Asp Lys Leu Glu Ser Glu Val Ser Arg Tyr Lys Glu Arg Leu
                325                 330                 335

His Asp Ile Glu Phe Tyr Lys Ala Arg Val Glu Glu Leu Lys Glu Asp
                340                 345                 350

Asn Gln Val Leu Leu Glu Thr Lys Thr Met Leu Glu Asp Gln Leu Glu
                355                 360                 365

Gly Thr Arg Ala Arg Ser Asp Lys Leu His Glu Leu Glu Lys Glu Asn
    370                 375                 380

Leu Gln Leu Lys Ala Lys Leu His Asp Met Glu Met Glu Arg Asp Met
385                 390                 395                 400

Asp Arg Lys Lys Ile Glu Glu Leu Met Glu Glu Asn Met Thr Leu Glu
                405                 410                 415
```

```
Met Ala Gln Lys Gln Ser Met Asp Glu Ser Leu His Leu Gly Trp Glu
        420             425             430

Leu Glu Gln Ile Ser Arg Thr Ser Glu Leu Ser Glu Ala Pro Gln Lys
        435             440             445

Ser Leu Gly His Glu Val Asn Glu Leu Thr Ser Ser Arg Leu Leu Lys
        450             455             460

Leu Glu Met Glu Asn Gln Ser Leu Thr Lys Thr Val Glu Glu Leu Arg
465             470             475             480

Thr Thr Val Asp Ser Val Glu Gly Asn Ala Ser Lys Ile Leu Lys Met
            485             490             495

Glu Lys Glu Asn Gln Arg Leu Ser Lys Lys Val Glu Ile Leu Glu Asn
        500             505             510

Glu Ile Val Gln Glu Lys Gln Ser Leu Gln Asn Cys Gln Asn Leu Ser
        515             520             525

Lys Asp Leu Met Lys Glu Lys Ala Gln Leu Glu Lys Thr Ile Glu Thr
        530             535             540

Leu Arg Glu Asn Ser Glu Arg Gln Ile Lys Ile Leu Glu Gln Glu Asn
545             550             555             560

Glu His Leu Asn Gln Thr Val Ser Ser Leu Arg Gln Arg Ser Gln Ile
            565             570             575

Ser Ala Glu Ala Arg Val Lys Asp Ile Glu Lys Glu Asn Lys Ile Leu
        580             585             590

His Glu Ser Ile Lys Glu Thr Ser Ser Lys Leu Ser Lys Ile Glu Phe
        595             600             605

Glu Lys Arg Gln Ile Lys Lys Glu Leu Glu His Tyr Lys Glu Lys Gly
        610             615             620

Glu Arg Ala Glu Glu Leu Glu Asn Glu Leu His His Leu Glu Lys Glu
625             630             635             640

Asn Glu Leu Leu Gln Lys Lys Ile Thr Asn Leu Lys Ile Thr Cys Glu
            645             650             655

Lys Ile Glu Ala Leu Glu Gln Glu Asn Ser Glu Leu Glu Arg Glu Asn
        660             665             670
```

```
Arg Lys Leu Lys Lys Thr Leu Asp Ser Phe Lys Asn Leu Thr Phe Gln
    675             680             685

Leu Glu Ser Leu Glu Lys Glu Asn Ser Gln Leu Asp Glu Glu Asn Leu
    690             695             700

Glu Leu Arg Arg Asn Val Glu Ser Leu Lys Cys Ala Ser Met Lys Met
705             710             715             720

Ala Gln Leu Gln Leu Glu Asn Lys Glu Leu Glu Ser Glu Lys Glu Gln
            725             730             735

Leu Lys Lys Gly Leu Glu Leu Leu Lys Ala Ser Phe Lys Lys Thr Glu
            740             745             750

Arg Leu Glu Val Ser Tyr Gln Gly Leu Asp Ile Glu Asn Gln Arg Leu
    755             760             765

Gln Lys Thr Leu Glu Asn Ser Asn Lys Lys Ile Gln Gln Leu Glu Ser
    770             775             780

Glu Leu Gln Asp Leu Glu Met Glu Asn Gln Thr Leu Gln Lys Asn Leu
785             790             795             800

Glu Glu Leu Lys Ile Ser Ser Lys Arg Leu Glu Gln Leu Glu Lys Glu
            805             810             815

Asn Lys Ser Leu Glu Gln Glu Thr Ser Gln Leu Glu Lys Asp Lys Lys
            820             825             830

Gln Leu Glu Lys Glu Asn Lys Arg Leu Arg Gln Gln Ala Glu Ile Lys
            835             840             845

Asp Thr Thr Leu Glu Glu Asn Asn Val Lys Ile Gly Asn Leu Glu Lys
    850             855             860

Glu Asn Lys Thr Leu Ser Lys Glu Ile Gly Ile Tyr Lys Glu Ser Cys
865             870             875             880

Val Arg Leu Lys Glu Leu Glu Lys Glu Asn Lys Glu Leu Val Lys Arg
            885             890             895

Ala Thr Ile Asp Ile Lys Thr Leu Val Thr Leu Arg Glu Asp Leu Val
    900             905             910

Ser Glu Lys Leu Lys Thr Gln Gln Met Asn Asn Asp Leu Glu Lys Leu
```

51

915              920              925

Thr His Glu Leu Glu Lys Ile Gly Leu Asn Lys Glu Arg Leu Leu His
    930          935          940

Asp Glu Gln Ser Thr Asp Asp Arg Tyr Lys Leu Leu Glu Ser Lys Leu
945         950         955         960

Glu Ser Thr Leu Lys Lys Ser Leu Glu Ile Lys Glu Glu Lys Ile Ala
      965          970          975

Ala Leu Glu Ala Arg Leu Glu Glu Ser Thr Asn Tyr Asn Gln Gln Leu
    980          985          990

Arg Gln Glu Leu Lys Thr Val Lys Lys Asn Tyr Glu Ala Leu Lys Gln
    995        1000        1005

Arg Gln Asp Glu Glu Arg Met Val Gln Ser Ser Pro Pro Ile Ser
   1010        1015        1020

Gly Glu Asp Asn Lys Trp Glu Arg Glu Ser Gln Glu Thr Thr Arg
   1025        1030        1035

Glu Leu Leu Lys Val Lys Asp Arg Leu Ile Glu Val Glu Arg Asn
   1040        1045        1050

Asn Ala Thr Leu Gln Ala Glu Lys Gln Ala Leu Lys Thr Gln Leu
   1055        1060        1065

Lys Gln Leu Glu Thr Gln Asn Asn Asn Leu Gln Ala Gln Ile Leu
   1070        1075        1080

Ala Leu Gln Arg Gln Thr Val Ser Leu Gln Glu Gln Asn Thr Thr
   1085        1090        1095

Leu Gln Thr Gln Asn Ala Lys Leu Gln Val Glu Asn Ser Thr Leu
   1100        1105        1110

Asn Ser Gln Ser Thr Ser Leu Met Asn Gln Asn Ala Gln Leu Leu
   1115        1120        1125

Ile Gln Gln Ser Ser Leu Glu Asn Glu Asn Glu Ser Val Ile Lys
   1130        1135        1140

Glu Arg Glu Asp Leu Lys Ser Leu Tyr Asp Ser Leu Ile Lys Asp
   1145        1150        1155

```
His Glu  Lys Leu Glu Leu Leu  His Glu Arg Gln Ala  Ser Glu Tyr
1160             1165              1170

Glu Ser  Leu Ile Ser Lys His  Gly Thr Leu Lys Ser  Ala His Lys
1175             1180              1185

Asn Leu  Glu Val Glu His Arg  Asp Leu Glu Asp Arg  Tyr Asn Gln
1190             1195              1200

Leu Leu  Lys Gln Lys Gly Gln  Leu Glu Asp Leu Glu  Lys Met Leu
1205             1210              1215

Lys Val  Glu Gln Glu Lys Met  Leu Leu Glu Asn Lys  Asn His Glu
1220             1225              1230

Thr Val  Ala Ala Glu Tyr Lys  Lys Leu Cys Gly Glu  Asn Asp Arg
1235             1240              1245

Leu Asn  His Thr Tyr Ser Gln  Leu Leu Lys Glu Thr  Glu Val Leu
1250             1255              1260

Gln Thr  Asp His Lys Asn Leu  Lys Ser Leu Leu Asn  Asn Ser Lys
1265             1270              1275

Leu Glu  Gln Thr Arg Leu Glu  Ala Glu Phe Ser Lys  Leu Lys Glu
1280             1285              1290

Gln Tyr  Gln Gln Leu Asp Ile  Thr Ser Thr Lys Leu  Asn Asn Gln
1295             1300              1305

Cys Glu  Leu Leu Ser Gln Leu  Lys Gly Asn Leu Glu  Glu Glu Asn
1310             1315              1320

Arg His  Leu Leu Asp Gln Ile  Gln Thr Leu Met Leu  Gln Asn Arg
1325             1330              1335

Thr Leu  Leu Glu Gln Asn Met  Glu Ser Lys Asp Leu  Phe His Val
1340             1345              1350

Glu Gln  Arg Gln Tyr Ile Asp  Lys Leu Asn Glu Leu  Arg Arg Gln
1355             1360              1365

Lys Glu  Lys Leu Glu Glu Lys  Ile Met Asp Gln Tyr  Lys Phe Tyr
1370             1375              1380

Asp Pro  Ser Pro Pro Arg Arg  Arg Gly Asn Trp Ile  Thr Leu Lys
1385             1390              1395
```

```
Met Arg Lys Leu Ile Lys Ser  Lys Lys Asp Ile Asn  Arg Glu Arg
    1400             1405              1410

Gln Lys Ser Leu Thr Leu Thr  Pro Thr Arg Ser Asp  Ser Ser Glu
    1415             1420              1425

Gly Phe Leu Gln Leu Pro His  Gln Asp Ser Gln Asp  Ser Ser Ser
    1430             1435              1440

Val Gly Ser Asn Ser Leu Glu  Asp Gly Gln Thr Leu  Gly Thr Lys
    1445             1450              1455

Lys Ser Ser Met Val Ala Leu  Lys Arg Leu Pro Phe  Leu Arg Asn
    1460             1465              1470

Arg Pro Lys Asp Lys Asp Lys  Met Lys Ala Cys Tyr  Arg Arg Ser
    1475             1480              1485

Met Ser Met Asn Asp Leu Val  Gln Ser Met Val Leu  Ala Gly Gln
    1490             1495              1500

Trp Thr Gly Ser Thr Glu Asn  Leu Glu Val Pro Asp  Asp Ile Ser
    1505             1510              1515

Thr Gly Lys Arg Arg Lys Glu  Leu Gly Ala Met Ala  Phe Ser Thr
    1520             1525              1530

Thr Ala Ile Asn Phe Ser Thr  Val Asn Ser Ser Ala  Gly Phe Arg
    1535             1540              1545

Ser Lys Gln Leu Val Asn Asn  Lys Asp Thr Thr Ser  Phe Glu Asp
    1550             1555              1560

Ile Ser Pro Gln Gly Val Ser  Asp Asp Ser Ser Thr  Gly Ser Arg
    1565             1570              1575

Val His Ala Ser Arg Pro Ala  Ser Leu Asp Ser Gly  Arg Thr Ser
    1580             1585              1590

Thr Ser Asn Ser Asn Asn Asn  Ala Ser Leu His Glu  Val Lys Ala
    1595             1600              1605

Gly Ala Val Asn Asn Gln Ser  Arg Pro Gln Ser His  Ser Ser Gly
    1610             1615              1620

Glu Phe Ser Leu Leu His Asp  His Glu Ala Trp Ser  Ser Ser Gly
    1625             1630              1635
```

```
Ser  Ser  Pro  Ile  Gln  Tyr  Leu  Lys  Arg  Gln  Thr  Arg  Ser  Ser  Pro
     1640                     1645                1650

Val  Leu  Gln  His  Lys  Ile  Ser  Glu  Thr  Leu  Glu  Ser  Arg  His  His
     1655                     1660                1665

Lys  Ile  Lys  Thr  Gly  Ser  Pro  Gly  Ser  Glu  Val  Val  Thr  Leu  Gln
     1670                     1675                1680

Gln  Phe  Leu  Glu  Glu  Ser  Asn  Lys  Leu  Thr  Ser  Val  Gln  Ile  Lys
     1685                     1690                1695

Ser  Ser  Ser  Gln  Glu  Asn  Leu  Leu  Asp  Glu  Val  Met  Lys  Ser  Leu
     1700                     1705                1710

Ser  Val  Ser  Ser  Asp  Phe  Leu  Gly  Lys  Asp  Lys  Pro  Val  Ser  Cys
     1715                     1720                1725

Gly  Leu  Ala  Arg  Ser  Val  Ser  Gly  Lys  Thr  Pro  Gly  Asp  Phe  Tyr
     1730                     1735                1740

Asp  Arg  Arg  Thr  Thr  Lys  Pro  Glu  Phe  Leu  Arg  Pro  Gly  Pro  Arg
     1745                     1750                1755

Lys  Thr  Glu  Asp  Thr  Tyr  Phe  Ile  Ser  Ser  Ala  Gly  Lys  Pro  Thr
     1760                     1765                1770

Pro  Gly  Thr  Gln  Gly  Lys  Ile  Lys  Leu  Val  Lys  Glu  Ser  Ser  Leu
     1775                     1780                1785

Ser  Arg  Gln  Ser  Lys  Asp  Ser  Asn  Pro  Tyr  Ala  Thr  Leu  Pro  Arg
     1790                     1795                1800

Ala  Ser  Ser  Val  Ile  Ser  Thr  Ala  Glu  Gly  Thr  Thr  Arg  Arg  Thr
     1805                     1810                1815

Ser  Ile  His  Asp  Phe  Leu  Thr  Lys  Asp  Ser  Arg  Leu  Pro  Ile  Ser
     1820                     1825                1830

Val  Asp  Ser  Pro  Pro  Ala  Ala  Ala  Asp  Ser  Asn  Thr  Thr  Ala  Ala
     1835                     1840                1845

Ser  Asn  Val  Asp  Lys  Val  Gln  Glu  Ser  Arg  Asn  Ser  Lys  Ser  Arg
     1850                     1855                1860

Ser  Arg  Glu  Gln  Gln  Ser  Ser
```

1865                            1870

<210> 2
<211> 1871
<212> PRT
<213> Homo sapiens

<400> 2

```
Met Glu Asn Glu Ile Phe Thr Pro Leu Leu Glu Gln Phe Met Thr Ser
1               5               10              15

Pro Leu Val Thr Trp Val Lys Thr Phe Gly Pro Leu Ala Ala Gly Asn
            20              25              30

Gly Thr Asn Leu Asp Glu Tyr Val Ala Leu Val Asp Gly Val Phe Leu
        35              40              45

Asn Gln Val Met Leu Gln Ile Asn Pro Lys Leu Glu Ser Gln Arg Val
    50              55              60

Asn Lys Lys Val Asn Asn Asp Ala Ser Leu Arg Met His Asn Leu Ser
65              70              75              80

Ile Leu Val Arg Gln Ile Lys Phe Tyr Tyr Gln Glu Thr Leu Gln Gln
            85              90              95

Leu Ile Met Met Ser Leu Pro Asn Val Leu Ile Ile Gly Lys Asn Pro
        100             105             110

Phe Ser Glu Gln Gly Thr Glu Glu Val Lys Lys Leu Leu Leu Leu Leu
    115             120             125

Leu Gly Cys Ala Val Gln Cys Gln Lys Lys Glu Glu Phe Ile Glu Arg
    130             135             140

Ile Gln Gly Leu Asp Phe Asp Thr Lys Ala Ala Val Ala Ala His Ile
145             150             155             160

Gln Glu Val Thr His Asn Gln Glu Asn Val Phe Asp Leu Gln Trp Met
            165             170             175

Glu Val Thr Asp Met Ser Gln Glu Asp Ile Glu Pro Leu Leu Lys Asn
        180             185             190

Met Ala Leu His Leu Lys Arg Leu Ile Asp Glu Arg Asp Glu His Ser
        195             200             205

Glu Thr Ile Ile Glu Leu Ser Glu Glu Arg Asp Gly Leu His Phe Leu
```

```
          210                        215                        220

Pro His Ala Ser Ser Ser Ala Gln Ser Pro Cys Gly Ser Pro Gly Met
225                 230                 235                 240

Lys Arg Thr Glu Ser Arg Gln His Leu Ser Val Glu Leu Ala Asp Ala
                245                 250                 255

Lys Ala Lys Ile Arg Arg Leu Arg Gln Glu Leu Glu Glu Lys Thr Glu
            260                 265                 270

Gln Leu Leu Asp Cys Lys Gln Glu Leu Glu Gln Met Glu Ile Glu Leu
            275                 280                 285

Lys Arg Leu Gln Gln Glu Asn Met Asn Leu Leu Ser Asp Ala Arg Ser
            290                 295                 300

Ala Arg Met Tyr Arg Asp Glu Leu Asp Ala Leu Arg Glu Lys Ala Val
305                 310                 315                 320

Arg Val Asp Lys Leu Glu Ser Glu Val Ser Arg Tyr Lys Glu Arg Leu
            325                 330                 335

His Asp Ile Glu Phe Tyr Lys Ala Arg Val Glu Glu Leu Lys Glu Asp
            340                 345                 350

Asn Gln Val Leu Leu Glu Thr Lys Thr Met Leu Glu Asp Gln Leu Glu
            355                 360                 365

Gly Thr Arg Ala Arg Ser Asp Lys Leu His Glu Leu Glu Lys Glu Asn
            370                 375                 380

Leu Gln Leu Lys Ala Lys Leu His Asp Met Glu Met Glu Arg Asp Met
385                 390                 395                 400

Asp Arg Lys Lys Ile Glu Glu Leu Met Glu Glu Asn Met Thr Leu Glu
            405                 410                 415

Met Ala Gln Lys Gln Ser Met Asp Glu Ser Leu His Leu Gly Trp Glu
            420                 425                 430

Leu Glu Gln Ile Ser Arg Thr Ser Glu Leu Ser Glu Ala Pro Gln Lys
            435                 440                 445

Ser Leu Gly His Glu Val Asn Glu Leu Thr Ser Ser Arg Leu Leu Lys
            450                 455                 460
```

```
Leu Glu Met Glu Asn Gln Ser Leu Thr Lys Thr Val Glu Glu Leu Arg
465                 470             475                 480

Thr Thr Val Asp Ser Val Glu Gly Asn Ala Ser Lys Ile Leu Lys Met
            485                 490                 495

Glu Lys Glu Asn Gln Arg Leu Ser Lys Lys Val Glu Ile Leu Glu Asn
        500                 505                 510

Glu Ile Val Gln Glu Lys Gln Ser Leu Gln Asn Cys Gln Asn Leu Ser
        515                 520                 525

Lys Asp Leu Met Lys Glu Lys Ala Gln Leu Glu Lys Thr Ile Glu Thr
    530                 535                 540

Leu Arg Glu Asn Ser Glu Arg Gln Ile Lys Ile Leu Glu Gln Glu Asn
545                 550                 555                 560

Glu His Leu Asn Gln Thr Val Ser Ser Leu Arg Gln Arg Ser Gln Ile
            565                 570                 575

Ser Ala Glu Ala Arg Val Lys Asp Ile Glu Lys Glu Asn Lys Ile Leu
        580                 585                 590

His Glu Ser Ile Lys Glu Thr Ser Ser Lys Leu Ser Lys Ile Glu Phe
    595                 600                 605

Glu Lys Arg Gln Ile Lys Lys Glu Leu Glu His Tyr Lys Glu Lys Gly
    610                 615                 620

Glu Arg Ala Glu Glu Leu Glu Asn Glu Leu His His Leu Glu Lys Glu
625                 630                 635                 640

Asn Glu Leu Leu Gln Lys Lys Ile Thr Asn Leu Lys Ile Thr Cys Glu
            645                 650                 655

Lys Ile Glu Ala Leu Glu Gln Glu Asn Ser Glu Leu Glu Arg Glu Asn
        660                 665                 670

Arg Lys Leu Lys Lys Thr Leu Asp Ser Phe Lys Asn Leu Thr Phe Gln
    675                 680                 685

Leu Glu Ser Leu Glu Lys Glu Asn Ser Gln Leu Asp Glu Glu Asn Leu
    690                 695                 700

Glu Leu Arg Arg Asn Val Glu Ser Leu Lys Cys Ala Ser Met Lys Met
705                 710                 715                 720
```

```
Ala Gln Leu Gln Leu Glu Asn Lys Glu Leu Glu Ser Glu Lys Glu Gln
            725               730           735

Leu Lys Lys Gly Leu Glu Leu Leu Lys Ala Ser Phe Lys Lys Thr Glu
            740               745           750

Arg Leu Glu Val Ser Tyr Gln Gly Leu Asp Ile Glu Asn Gln Arg Leu
            755               760           765

Gln Lys Thr Leu Glu Asn Ser Asn Lys Lys Ile Gln Gln Leu Glu Ser
        770               775           780

Glu Leu Gln Asp Leu Glu Met Glu Asn Gln Thr Leu Gln Lys Asn Leu
785               790               795               800

Glu Glu Leu Lys Ile Ser Ser Lys Arg Leu Glu Gln Leu Glu Lys Glu
                805               810               815

Asn Lys Ser Leu Glu Gln Glu Thr Ser Gln Leu Glu Lys Asp Lys Lys
            820               825               830

Gln Leu Glu Lys Glu Asn Lys Arg Leu Arg Gln Gln Ala Glu Ile Lys
            835               840               845

Asp Thr Thr Leu Glu Glu Asn Asn Val Lys Ile Gly Asn Leu Glu Lys
        850               855               860

Glu Asn Lys Thr Leu Ser Lys Glu Ile Gly Ile Tyr Lys Glu Ser Cys
865               870               875               880

Val Arg Leu Lys Glu Leu Glu Lys Glu Asn Lys Glu Leu Val Lys Arg
                885               890               895

Ala Thr Ile Asp Ile Lys Thr Leu Val Thr Leu Arg Glu Asp Leu Val
            900               905               910

Ser Glu Lys Leu Lys Thr Gln Gln Met Asn Asn Asp Leu Glu Lys Leu
            915               920               925

Thr His Glu Leu Glu Lys Ile Gly Leu Asn Lys Glu Arg Leu Leu His
        930               935               940

Asp Glu Gln Ser Thr Asp Asp Ser Arg Tyr Lys Leu Leu Glu Ser Lys
945               950               955               960

Leu Glu Ser Thr Leu Lys Lys Ser Leu Glu Ile Lys Glu Glu Lys Ile
                965               970               975
```

```
Ala Ala Leu Glu Ala Arg Leu Glu Glu Ser Thr Asn Tyr Asn Gln Gln
        980                     985                     990

Leu Arg Gln Glu Leu Lys Thr Val  Lys Lys Asn Tyr Glu  Ala Leu Lys
        995                 1000                     1005

Gln Arg  Gln Asp Glu Glu Arg  Met Val Gln Ser Ser  Pro Pro Ile
        1010                1015                1020

Ser Gly  Glu Asp Asn Lys Trp  Glu Arg Glu Ser Gln  Glu Thr Thr
        1025                1030                1035

Arg Glu  Leu Leu Lys Val Lys  Asp Arg Leu Ile Glu  Val Glu Arg
        1040                1045                1050

Asn Asn  Ala Thr Leu Gln Ala  Glu Lys Gln Ala Leu  Lys Thr Gln
        1055                1060                1065

Leu Lys  Gln Leu Glu Thr Gln  Asn Asn Asn Leu Gln  Ala Gln Ile
        1070                1075                1080

Leu Ala  Leu Gln Arg Gln Thr  Val Ser Leu Gln Glu  Gln Asn Thr
        1085                1090                1095

Thr Leu  Gln Thr Gln Asn Ala  Lys Leu Gln Val Glu  Asn Ser Thr
        1100                1105                1110

Leu Asn  Ser Gln Ser Thr Ser  Leu Met Asn Gln Asn  Ala Gln Leu
        1115                1120                1125

Leu Ile  Gln Gln Ser Ser Leu  Glu Asn Glu Asn Glu  Ser Val Ile
        1130                1135                1140

Lys Glu  Arg Glu Asp Leu Lys  Ser Leu Tyr Asp Ser  Leu Ile Lys
        1145                1150                1155

Asp His  Glu Lys Leu Glu Leu  Leu His Glu Arg Gln  Ala Ser Glu
        1160                1165                1170

Tyr Glu  Ser Leu Ile Ser Lys  His Gly Thr Leu Lys  Ser Ala His
        1175                1180                1185

Lys Asn  Leu Glu Val Glu His  Arg Asp Leu Glu Asp  Arg Tyr Asn
        1190                1195                1200

Gln Leu  Leu Lys Gln Lys Gly  Gln Leu Glu Asp Leu  Glu Lys Met
```

```
                 1205                        1210                        1215

         Leu Lys Val Glu Gln Glu Lys Met Leu Leu Glu Asn Lys Asn His
                 1220                        1225                        1230

         Glu Thr Val Ala Ala Glu Tyr Lys Lys Leu Cys Gly Glu Asn Asp
                 1235                        1240                        1245

         Arg Leu Asn His Thr Tyr Ser Gln Leu Leu Lys Glu Thr Glu Val
                 1250                        1255                        1260

         Leu Gln Thr Asp His Lys Asn Leu Lys Ser Leu Leu Asn Asn Ser
                 1265                        1270                        1275

         Lys Leu Glu Gln Thr Arg Leu Glu Ala Glu Phe Ser Lys Leu Lys
                 1280                        1285                        1290

         Glu Gln Tyr Gln Gln Leu Asp Ile Thr Ser Thr Lys Leu Asn Asn
                 1295                        1300                        1305

         Gln Cys Glu Leu Leu Ser Gln Leu Lys Gly Asn Leu Glu Glu Glu
                 1310                        1315                        1320

         Asn Arg His Leu Leu Asp Gln Ile Gln Thr Leu Met Leu Gln Asn
                 1325                        1330                        1335

         Arg Thr Leu Leu Glu Gln Asn Met Glu Ser Lys Asp Leu Phe His
                 1340                        1345                        1350

         Val Glu Gln Arg Gln Tyr Ile Asp Lys Leu Asn Glu Leu Arg Arg
                 1355                        1360                        1365

         Gln Lys Glu Lys Leu Glu Glu Lys Ile Met Asp Gln Tyr Lys Phe
                 1370                        1375                        1380

         Tyr Asp Pro Ser Pro Pro Arg Arg Arg Gly Asn Trp Ile Thr Leu
                 1385                        1390                        1395

         Lys Met Arg Lys Leu Ile Lys Ser Lys Lys Asp Ile Asn Arg Glu
                 1400                        1405                        1410

         Arg Gln Lys Ser Leu Thr Leu Thr Pro Thr Arg Ser Asp Ser Ser
                 1415                        1420                        1425

         Glu Gly Phe Leu Gln Leu Pro His Gln Asp Ser Gln Asp Ser Ser
                 1430                        1435                        1440
```

```
Ser Val  Gly Ser Asn Ser Leu  Glu Asp Gly Gln Thr  Leu Gly Thr
    1445             1450             1455

Lys Lys  Ser Ser Met Val Ala  Leu Lys Arg Leu Pro  Phe Leu Arg
    1460             1465             1470

Asn Arg  Pro Lys Asp Lys Asp  Lys Met Lys Ala Cys  Tyr Arg Arg
    1475             1480             1485

Ser Met  Ser Met Asn Asp Leu  Val Gln Ser Met Val  Leu Ala Gly
    1490             1495             1500

Gln Trp  Thr Gly Ser Thr Glu  Asn Leu Glu Val Pro  Asp Asp Ile
    1505             1510             1515

Ser Thr  Gly Lys Arg Arg Lys  Glu Leu Gly Ala Met  Ala Phe Ser
    1520             1525             1530

Thr Thr  Ala Ile Asn Phe Ser  Thr Val Asn Ser Ser  Ala Gly Phe
    1535             1540             1545

Arg Ser  Lys Gln Leu Val Asn  Asn Lys Asp Thr Thr  Ser Phe Glu
    1550             1555             1560

Asp Ile  Ser Pro Gln Gly Val  Ser Asp Asp Ser Ser  Thr Gly Ser
    1565             1570             1575

Arg Val  His Ala Ser Arg Pro  Ala Ser Leu Asp Ser  Gly Arg Thr
    1580             1585             1590

Ser Thr  Ser Asn Ser Asn Asn  Asn Ala Ser Leu His  Glu Val Lys
    1595             1600             1605

Ala Gly  Ala Val Asn Asn Gln  Ser Arg Pro Gln Ser  His Ser Ser
    1610             1615             1620

Gly Glu  Phe Ser Leu Leu His  Asp His Glu Ala Trp  Ser Ser Ser
    1625             1630             1635

Gly Ser  Ser Pro Ile Gln Tyr  Leu Lys Arg Gln Thr  Arg Ser Ser
    1640             1645             1650

Pro Val  Leu Gln His Lys Ile  Ser Glu Thr Leu Glu  Ser Arg His
    1655             1660             1665

His Lys  Ile Lys Thr Gly Ser  Pro Gly Ser Glu Val  Val Thr Leu
    1670             1675             1680
```

```
Gln Gln Phe Leu Glu Glu Ser Asn Lys Leu Thr Ser Val Gln Ile
    1685                1690                1695

Lys Ser Ser Ser Gln Glu Asn Leu Leu Asp Glu Val Met Lys Ser
    1700                1705                1710

Leu Ser Val Ser Ser Asp Phe Leu Gly Lys Asp Lys Pro Val Ser
    1715                1720                1725

Cys Gly Leu Ala Arg Ser Val Ser Gly Lys Thr Pro Gly Asp Phe
    1730                1735                1740

Tyr Asp Arg Arg Thr Thr Lys Pro Glu Phe Leu Arg Pro Gly Pro
    1745                1750                1755

Arg Lys Thr Glu Asp Thr Tyr Phe Ile Ser Ser Ala Gly Lys Pro
    1760                1765                1770

Thr Pro Gly Thr Gln Gly Lys Ile Lys Leu Val Lys Glu Ser Ser
    1775                1780                1785

Leu Ser Arg Gln Ser Lys Asp Ser Asn Pro Tyr Ala Thr Leu Pro
    1790                1795                1800

Arg Ala Ser Ser Val Ile Ser Thr Ala Glu Gly Thr Thr Arg Arg
    1805                1810                1815

Thr Ser Ile His Asp Phe Leu Thr Lys Asp Ser Arg Leu Pro Ile
    1820                1825                1830

Ser Val Asp Ser Pro Pro Ala Ala Ala Asp Ser Asn Thr Thr Ala
    1835                1840                1845

Ala Ser Asn Val Asp Lys Val Gln Glu Ser Arg Asn Ser Lys Ser
    1850                1855                1860

Arg Ser Arg Glu Gln Gln Ser Ser
    1865                1870
```

**Description**

**FIELD**

Provided herein are methods and kits for analyzing stage II colorectal cancer (CRC) samples (namely those that are mis-match repair proficient, pMMR). Such methods include scoring G-alpha interacting vesicle associated protein (GIV, also known as girdin) full-length (GIV-fl) expression to provide a GIV-fl status for the CRC in combination with determining the lymphovascular invasion (LVI) status of the CRC.

**PARTIES TO JOINT RESEARCH AGREEMENT**

Ventana Medical Systems, Inc. and the University of California, San Diego are parties to a joint research agreement governing inventions disclosed herein.

**BACKGROUND**

Metastasis is a multi-step, complex process that involves migration of tumor cells to distant locations in the body. Cancer cells that migrate or invade utilize chemotactic receptors to sense growth factor gradients and must amplify PI3K-Akt signaling (Muller et al., Nature, 2001. 410(6824):50-6). In human epithelial cancers the PI3K-Akt signaling pathway is frequently hyperactivated during cancer invasion (Larue and Bellacosa, Oncogene, 2005. 24(50):7443-54), and progressive enhancement of PI3K-Akt coupled to efficient cell migration are hallmarks of high metastatic potential (Qiao et al., Cancer Res, 2007. 67(11):5293-9). However, accurate prediction of metastatic potential of tumors using mechanistically identified biological markers has met limited success (Fidler, Nat Rev Cancer, 2003. 3(6):453-8). Despite recent studies showing that the expression of genes/proteins associated with PI3K-Akt signaling, actin remodeling, motility and invasion vary among tumors (Qiao et al., Cell Cycle, 2008. 7(19):2991-6), most of them have failed to make a transition into cancer clinics as biomarkers for prognostication. Also, GIV-fl expression has been suggested in the prediction of survival of colon cancer patients (Garcia-Marcos et al., FASEB J, 2011. 25(2):590-9. However, the search continues for molecules/markers which are proven to play a role during tumor invasion, whose mechanisms of action are well characterized, and which may also serve to stratify the risk of recurrence or metastasis among cancer patients in clinics.

**SUMMARY**

The present application provides methods, as defined in the claims, that allow for classification of a patient with stage II (such as a stage IIa or IIb) colorectal cancer that is mis-match repair proficient (pMMR) as high- or low-risk, that is, whether their CRC is likely to recur or progress. Such methods are useful as patients at high-risk for progression can be selected to receive chemotherapy or biotherapy, while low-risk patients can be selected to not receive chemotherapy or biotherapy. The claimed methods may be followed by treating patients having been identified as having a high-risk with chemotherapy or biotherapy. In addition, the disclosed methods can be used for chemoprediction, that is, identifying CRC pMMR stage II (such as a stage IIa or IIb) tumors that are more likely to respond to chemotherapy or biotherapy (*e.g.*, those classified as high-risk with the disclosed methods).
It is shown herein that determination of expression of full-length G-alpha interacting vesicle associated protein (GIV-fl) (for example by scoring GIV-fl expression), and determination of the lymphovascular invasion (LVI) status of the subject, can predict with high accuracy those stage II CRCs that are likely to recur (for example within at least 2 years or at least 5 years), and those that are not likely to recur. In some examples, the methods also use one or more other characteristics of the subject, such as the age of the subject at diagnosis, number of lymph nodes that are positive for CRC, sex of the subject, state of tumor differentiation, T stage of the cancer; and on which side the colon cancer was present, to determine whether the stage II CRC is likely to recur, and thus is a high-risk tumor that should be treated with chemotherapy or biotherapy.
Thus, the disclosure provides methods for analyzing a stage II pMMR CRC sample obtained from a subject, for example to classify the CRC as one having high- or low-risk for progressing, for example within 1 year, within 2 years, within 3 years, within 4 years, within 5 years, or within 6 years. The method includes contacting a stage II pMMR CRC sample with a G-alpha interacting vesicle associated GIV-fl protein specific binding agent, wherein the GIV-fl protein specific binding agent comprises an antibody specific for GIV-fl (*i.e.,* which cannot detect forms of GIV that are missing the C-terminus, such as the C-terminal 762 or 763 amino acids), or contacting RNA isolated from a sample with a nucleic acid probe specific for G-alpha interacting vesicle associated protein-full length (GIV-f1) mRNA. The method also includes scoring expression of GIV-fl protein or mRNA in the sample to determine a GIV-fl status (*e.g.*, positive or negative) of the sample.

In one example, scoring expression of GIV-fl protein includes determining an extent of positive GIV-fl staining for the sample on a scale of 0 to 3, wherein extent of positive staining is assigned 0 if 0% to 10% of the total area of the sample is stained positive, wherein extent of positive staining is assigned 1 if 11%-35% of the total area of the sample is stained positive, wherein extent of positive staining is assigned 2 if 36%-50% of the total area of the sample is stained positive, and wherein extent of positive staining is assigned 3 if 51%-100% of the total area of the sample is stained positive; determining a GIV-fl intensity of staining for the sample on a scale of 0 (negative), 1 (weak), 2 (moderate), to 3 (strong); summing the extent of positive GIV-fl staining and the GIV-fl intensity of staining, thereby generating a GIV-fl score value from 0 to 6; and determining that the sample is GIV-fl negative if the GIV-fl score value is 0-2 or determining that the sample is GIV-fl positive if the GIV-fl score value is 3-6.

In another example, scoring expression of GIV-fl protein includes determining if a total area of GIV-fl staining for the sample is greater than 10% or determining if the GIV-fl staining intensity for the sample is 3+ (strong). For example, a sample can be scored negative (0) if there is an absence of any detectable signal or pale gray/tan signal which is similar to the intensity on the negative control reagent; a sample can be scored weak (1+) if there is by light brown staining intensity which is more than that seen on the negative control reagent and in the background; a sample can be scored moderate (2+) if there is brown intensity; or a sample can be scored strong (3+) intensity if there is dark brown to black signal intensity. The GIV-fl status is determined to be positive if the total area of GIV-fl staining with any intensity for the sample is greater than 10% or if the GIV-fl staining intensity is strong (3+) with any percent; or the GIV-fl status is determined to be negative if the total area of GIV-fl staining with a staining intensity of 0, 1+, or 2+ for the sample is less than 10%. The method also includes determining the lymphovascular invasion (LVI) status of the subject, for example by determining whether viable CRC cells are present in any of blood vessels or lymphatic vessel(s) are present in the tumor. In some examples, the method further includes determining one or more additional characteristics of the subject, such as the age of the subject at diagnosis, the number of lymph nodes that are positive for CRC, sex of the subject, state of tumor differentiation, T stage of the cancer; and on which side the colon cancer was present. The determined GIV-fl status, LVI status, and optionally one or more of the subject's characteristics are inputted into a computer or algorithm, which then generates an output, thereby analyzing the sample. In some examples, the output is an indication as to whether the CRC is likely to progress (*e.g.*, metastasize). For example, the output can be a notation that the CRC is a high- or low-risk. In some examples the output is the likely progression free survival (PFS) of the subject. This can allow a physician to identify those chemo-naïve subjects likely to respond to treatment with chemotherapy or biotherapy (high risk) from a chemo-naïve subject who does not need chemotherapy or biotherapy (low risk). One or more steps of the disclosed methods can be performed by a suitably-programmed computer.

The claimed method may be followed by treating a subject identified as high-risk with chemotherapy or biotherapy, such as one or more of 5-fluouracil, leucovorin, panitumumab (VECTIBIX®), cetuximab (ERBITUX®), bevacizumab (AVASTIN®), ziv-aflibercept (ZALTRAP®), irinotecan (CAMPTOSAR®), and oxaliplatin (ELOXATIN®).

Also provided are computer-implemented methods. In one example, the method includes generating a GIV-fl protein expression score based at least on measured GIV-fl protein expression within a displayed image depicting a stage II colorectal cancer (CRC) sample detectably labeled with a GIV-fl antibody, wherein the CRC sample is obtained from a subject, and wherein the GIV-fl protein expression score is generated by (i) determining an extent of positive GIV-fl staining for the sample on a scale of 0 to 3, wherein extent of positive staining is assigned 0 if 0% to 10% of the total area of the sample is stained positive, wherein extent of positive staining is assigned 1 if 11%-35% of the total area of the sample is stained positive, wherein extent of positive staining is assigned 2 if 36%-50% of the total area of the sample is stained positive, and wherein extent of positive staining is assigned 3 if 51%-100% of the total area of the sample is stained positive; determining a GIV-fl staining intensity score (*e.g.,* an overall or predominant staining intensity) for the sample on a scale of 0 to 3 (for example, a sample can be scored negative (0) if there is an absence of any detectable signal or pale gray/tan signal which is similar to the intensity on the negative control reagent; a sample can be scored weak (1) if there is by light brown staining intensity which is more than that seen on the negative control reagent and in the background; a sample can be scored moderate (2) if there is brown intensity; or a sample can be scored strong (3) intensity if there is dark brown to black signal intensity), summing the extent of positive GIV-fl staining and the GIV-fl intensity of staining, thereby generating a GIV-fl score value from 0 to 6; and determining that the sample is GIV-fl negative if the GIV-fl score value is 0-2 or determining that the sample is GIV-fl positive if the GIV-fl score value is 3-6; or (ii) determining if a total area of GIV-fl staining for the sample is greater than 10% or determining if the GIV-fl staining intensity is 3+ (strong) (for example, a sample can be scored negative (0) if there is an absence of any detectable signal or pale gray/tan signal which is similar to the intensity on the negative control reagent; a sample can be scored weak (1+) if there is by light brown staining intensity which is more than that seen on the negative control reagent and in the background; a sample can be scored moderate (2+) if there is brown intensity; or a sample can be scored strong (3+) intensity if there is dark brown to black signal intensity), and determining that the sample is GIV-fl positive if the total area of GIV-fl staining with any intensity for the sample is greater than 10% or if the GIV-fl staining intensity is strong (3+) with any percent; or determining that the sample is GIV-fl negative if the total area of GIV-fl staining with a staining intensity of 0, 1+, or 2+ for the sample is less than 10%. The resulting GIV-fl protein expression score for the sample can be outputted from the computer, such as a visual or audible output. In some examples, the computer-implemented methods further include inputting the lymphovascular invasion (LVI) status of the subject into the computer and optionally inputting into the computer one or more characteristics of the subject, wherein the one or more characteristics include age of the subject at diagnosis, number of lymph nodes that are positive for CRC; sex of the subject, state of tumor differentiation, T stage of the cancer; and on which side the colon cancer was present. In some examples, the one or more characteristics of the subject include, the tumor grade, LVI, number of lymph nodes examined, whether there is perineural invasion, whether there is localized perforation, and whether the margins are indeterminate or positive. The computer can then provide an output, such as whether the CRC is likely to progress (*e.g.*, metastasize). For example, the output can be a notation that the CRC is a high- or low-risk, the likely progression free survival (PFS) of the subject, the prognosis for the subject, whether a subject is likely to benefit from chemotherapy or biotherapy, or combinations thereof.

Also disclosed are one or more non-transitory computer-readable media that include computer-executable instructions causing a computing system to perform the methods provided herein.

Systems for analyzing a stage II mis-match repair proficient (pMMR) colorectal cancer (CRC) sample obtained from a subject are also described. Such systems can include a means for measuring a level of GIV-fl in the sample (such as a GIV-fl-specific antibody); and means for determining the LVI status of the subject (such as H&E or antibodies specific for LVI markers such as CD34 and/or lymphatic endothelium). In some examples, the system includes implemented rules for determining the GIV-fl status of the sample (*e.g.*, positive or negative) based on the measured level of GIV-fl. In some examples, the system includes implemented rules for comparing the measured level of GIV-fl to a GIV-fl reference value, such as a GIV-fl positive or negative control. In some examples, the system includes implemented rules for determining the LVI status of the sample (*e.g.*, positive or negative), for example based on the measured level of LVI markers or based on histological markers (*e.g.*, H&E staining). In some examples, the system includes implemented rules for comparing the measured LVI markers to an LVI reference value or sample, such as an LVI positive or negative control. In some examples, the GIV-fl and/or LVI reference values are stored values. In some examples, the GIV-fl and/or LVI reference values are a level of GIV-fl and/or LVI measured from a control sample by said means for measuring. The system can also include one or more means for implementing the rules, whereby an indication of the likely risk of CRC recurrence and/or likely response of the CRC to chemotherapy or biotherapy is provided based on the GIV-fl and LVI status. In some examples, the system also includes a means for determining one or more characteristics of the subject, wherein the one or more characteristics include age of the subject at diagnosis, number of lymph nodes that are positive for CRC; sex of the subject, state of tumor differentiation, T stage of the cancer; and on which side the colon cancer was present; and implemented rules for comparing the measured level of one or more characteristics to a reference value for the one or more characteristics.

The invention also provides kits, that can be used with the methods provided herein. In one example, the kit includes a GIV-fl specific-binding agent, such as antibody clone SP173. The kit includes: a specific-binding agent that permits for a determination of LVI comprising an antibody specific for CD34 or lymphatic endothelium); a mis-match repair protein specific-binding agent comprising an antibody specific for mutL homolog 1 (MLH1); postmeiotic segregation increased 2 (PMS2); MutS protein homolog 2 Msh2 (MSH2), MutS protein homolog 6 (MSH6), or a combination of such antibodies; and optionally microscope slides; labeled secondary antibodies; and buffers for IHC.

In some embodiments, the disclosure includes a method for analyzing a stage II mis-match repair proficient (pMMR) colorectal cancer (CRC) sample obtained from a subject, comprising: isolating RNA from the sample, contacting the sample containing the RNA with a nucleic acid probe specific for G-alpha interacting vesicle associated protein-full length (GIV-fl) mRNA; and determining the amount of the GIV-fl mRNA in the sample.

The foregoing and other objects and features of the disclosure will become more apparent from the following detailed description, which proceeds with reference to the accompanying figures.

## BRIEF DESCRIPTION OF THE DRAWINGS

FIG. 1 is a data flow diagram that describes the steps used in the statistical modeling disclosed herein.

FIGS. 2A and 2B are digital images showing IHC staining of (A) normal and (B) cancerous colon samples with GIV antibody from IBL or the SP173 Ab described in Example 1.

FIG. 2C are digital images showing GIV-fl IHC staining of cancerous cell lines.

FIGS. 3A-3C are digital images showing IHC staining of invasive cancer cell lines (A) MDA MB231; (B) DLD1 and in (C) normal liver with different amounts of the GIV antibody described in Example 1.

FIGS. 4A-4B are digital images showing IHC staining of GIV negative (A) and GIV positive (B) colon cancer cases with different amounts of the GIV antibody described in Example 1.

FIGS. 5A-5D are digital images showing IHC staining using the GIV antibody described in Example 1 in colorectal cancers with a score of (A) 0, (B) 1+, (C) 2+ or (D) 3+.

**FIG. 6** shows Kaplan-Meier curves for all stage II CRC subjects (T3+T4) stratified by mis-match repair proficient (pMMR), mis-match repair deficient (dMMR), pMMR/GIV-positive, pMMR/GIV-negative, using the GIV-fl Extent and Intensity Score scoring algorithm shown in Table 7.

**FIGS. 7A-7B** show Kaplan-Meier survival curves using (A) GIV-fl Extent and Intensity Score from Table 7 or (B) GIV-fl Predictive Score.

**FIG. 8** shows Kaplan-Meier curves for chemo-naive stage II CRC subjects (T3+T4) stratified by mis-match repair proficient (pMMR), mis-match repair deficient (dMMR), pMMR/GIV-positive, pMMR/GIV-negative, using the GIV-fl Predictive Score scoring algorithm shown in Table 7.

**FIG. 9** shows a dot plot of the potential predictor variables ranked by adjusted partial $\chi2$ statistics.

**FIGS. 10A-10B** show AUC plots for four statistical prediction models for (A) 2 year and (B) 5 year progression free survival.

**FIG. 11** shows a predictiveness curve for chemo-naïve stage II T3 CRC patients based on a statistical model including GIV-fl and LVI.

**FIG. 12** shows Kaplan-Meier curves for chemo-naïve (top graph) and chemo-treated (bottom graph) stage II T3 CRC patients from the BioGrid 1 cohort stratified for GIV+LVI and MMR.

**FIG. 13** shows Kaplan-Meier curves for chemo-naïve (top graph) and chemo-treated (bottom graph) stage II T3 CRC patients from the BioGrid 2 cohort stratified for GIV+LVI and MMR.

**SEQUENCE LISTING**

SEQ ID NO: 1 is the protein sequence of GenBank® accession number BAE44387

```
   1 meneiftpll eqfmtsplvt wvktfgplaa gngtnldeyv alvdgvflnq vmlqinpkle
  61 sqrvnkkvnn daslrmhnls ilvrqikfyy qetlqqlimm slpnvliigk npfseqgtee
 121 vkklllllg cavqcqkkee fieriqgldf dtkaavaahi qevthnqenv fdlqwmevtd
 181 msqediepll knmalhlkrl iderdehset iielseerdg lhflphasss aqspcgspgm
 241 krtesrqhls veladakaki rrlrqeleek teqlldckqe leqmeielkr lqqenmnlls
 301 darsarmyrd eldalrekav rvdklesevs rykerlhdie fykarveelk ednqvlletk
 361 tmledqlegt rarsdklhel ekenlqlkak lhdmemerdm drkkieelme enmtlemaqk
 421 qsmdeslhlg weleqisrts elseapqksl ghevneltss rllklemenq sltktveelr
 481 ttvdsvegna skilkmeken qrlskkveil eneivqekqs lqncqnlskd lmkekaqlek
 541 tietlrense rqikileqen ehlnqtvssl rqrsqisaea rvkdiekenk ilhesikets
 601 sklskiefek rqikkelehy kekgeraeel enelhhleke nellqkkitn lkitcekiea
 661 leqenseler enrklkktld sfknltfqle slekensqld eenlelrrnv eslkcasmkm
 721 aqlqlenkel esekeqlkkg lellkasfkk terlevsyqg ldienqrlqk tlensnkkiq
 781 qleselqdle menqtlqknl eelkisskrl eqlekenksl eqetsqlekd kkqlekenkr
 841 lrqqaeikdt tleennvkig nlekenktls keigiykesc vrlkeleken kelvkratid
 901 iktlvtlred lvseklktqq mnndleklth elekiglnke rllhdeqstd dryklleskl
 961 estlkkslei keekiaalea rleestnynq qlrqelktvk knyealkqrq deermvqssp
1021 pisgednkwe resqettrel lkvkdrliev ernnatlqae kqalktqlkq letqnnnlqa
1081 qilalqrqtv slqeqnttlq tqnaklqven stlnsqstsl mnqnaqlliq qsslenenes
1141 vikeredlks lydslikdhe klellherqa seyeslliskh gtlksahknl evehrdledr
1201 ynqllkqkgq ledlekmlkv eqekmllenk nhetvaaeyk klcgendrln htysqllket
1261 evlqtdhknl ksllnnskle qtrleaefsk lkeqyqqldi tstklnnqce llsqlkgnle
1321 eenrhlldqi qtlmlqnrtl leqnmeskdl fhveqrqyid klnelrrqke kleekimdqy
1381 kfydpspprr rgnwitlkmr klikskkdin rerqksltlt ptrsdssegf lqlphqdsqd
1441 sssvgsnsle dgqtlgtkks smvalkrlpf lrnrpkdkdk mkacyrrsms mndlvqsmvl
1501 agqwtgsten levpddistg krrkelgama fsttainfst vnssagfrsk qlvnnkdtts
1561 fedispqgvs ddsstgsrvh asrpasldsg rtstsnsnnn aslhevkaga vnnqsrpqsh
1621 ssgefsllhd heawsssgss piqylkrqtr sspvlqhkis etlesrhhki ktgspgsevv
1681 tlqqfleesn kltsvqikss sqenlldevm kslsvssdfl gkdkpvscgl arsvsgktpg
1741 dfydrrttkp eflrpgprkt edtyfissag kptpgtqgki klvkesslsr qskdsnpyat
1801 lprassvist aegttrrtsi hdfltkdsrl pisvdsppaa adsnttaasn vdkvqesrns
1861 ksrsreqqss
```

SEQ ID NO: 2 is the protein sequence of GenBank® accession number Q3V6T2

```
   1 MENEIFTPLL EQFMTSPLVT WVKTFGPLAA GNGTNLDEYV ALVDGVFLNQ VMLQINPKLE
  61 SQRVNKKVNN DASLRMHNLS ILVRQIKFYY QETLQQLIMM SLPNVLIIGK NPFSEQGTEE
 121 VKKLLLLLLG CAVQCQKKEE FIERIQGLDF DTKAAVAAHI QEVTHNQENV FDLQWMEVTD
 181 MSQEDIEPLL KNMALHLKRL IDERDEHSET IIELSEERDG LHFLPHASSS AQSPCGSPGM
 241 KRTESRQHLS VELADAKAKI RRLRQELEEK TEQLLDCKQE LEQMEIELKR LQQENMNLLS
 301 DARSARMYRD ELDALREKAV RVDKLESEVS RYKERLHDIE FYKARVEELK EDNQVLLETK
 361 TMLEDQLEGT RARSDKLHEL EKENLQLKAK LHDMEMERDM DRKKIEELME ENMTLEMAQK
 421 QSMDESLHLG WELEQISRTS ELSEAPQKSL GHEVNELTSS RLLKLEMENQ SLTKTVEELR
 481 TTVDSVEGNA SKILKMEKEN QRLSKKVEIL ENEIVQEKQS LQNCQNLSKD LMKEKAQLEK
 541 TIETLRENSE RQIKILEQEN EHLNQTVSSL RQRSQISAEA RVKDIEKENK ILHESIKETS
 601 SKLSKIEFEK RQIKKELEHY KEKGERAEEL ENELHHLEKE NELLQKKITN LKITCEKIEA
 661 LEQENSELER ENRKLKKTLD SFKNLTFQLE SLEKENSQLD EENLELRRNV ESLKCASMKM
 721 AQLQLENKEL ESEKEQLKKG LELLKASFKK TERLEVSYQG LDIENQRLQK TLENSNKKIQ
 781 QLESELQDLE MENQTLQKNL EELKISSKRL EQLEKENKSL EQETSQLEKD KKQLEKENKR
 841 LRQQAEIKDT TLEENNVKIG NLEKENKTLS KEIGIYKESC VRLKELEKEN KELVKRATID
 901 IKTLVTLRED LVSEKLKTQQ MNNDLEKLTH ELEKIGLNKE RLLHDEQSTD DSRYKLLESK
 961 LESTLKKSLE IKEEKIAALE ARLEESTNYN QQLRQELKTV KKNYEALKQR QDEERMVQSS
1021 PPISGEDNKW ERESQETTRE LLKVKDRLIE VERNNATLQA EKQALKTQLK QLETQNNNLQ
1081 AQILALQRQT VSLQEQNTTL QTQNAKLQVE NSTLNSQSTS LMNQNAQLLI QQSSLENENE
1141 SVIKEREDLK SLYDSLIKDH EKLELLHERQ ASEYESLISK HGTLKSAHKN LEVEHRDLED
1201 RYNQLLKQKG QLEDLEKMLK VEQEKMLLEN KNHETVAAEY KKLCGENDRL NHTYSQLLKE
1261 TEVLQTDHKN LKSLLNNSKL EQTRLEAEFS KLKEQYQQLD ITSTKLNNQC ELLSQLKGNL
1321 EEENRHLLDQ IQTLMLQNRT LLEQNMESKD LFHVEQRQYI DKLNELRRQK EKLEEKIMDQ
1381 YKFYDPSPPR RRGNWITLKM RKLIKSKKDI NRERQKSLTL TPTRSDSSEG FLQLPHQDSQ
1441 DSSSVGSNSL EDGQTLGTKK SSMVALKRLP FLRNRPKDKD KMKACYRRSM SMNDLVQSMV
1501 LAGQWTGSTE NLEVPDDIST GKRRKELGAM AFSTTAINFS TVNSSAGFRS KQLVNNKDTT
1561 SFEDISPQGV SDDSSTGSRV HASRPASLDS GRTSTSNSNN NASLHEVKAG AVNNQSRPQS
1621 HSSGEFSLLH DHEAWSSSGS SPIQYLKRQT RSSPVLQHKI SETLESRHHK IKTGSPGSEV
1681 VTLQQFLEES NKLTSVQIKS SSQENLLDEV MKSLSVSSDF LGKDKPVSCG LARSVSGKTP
1741 GDFYDRRTTK PEFLRPGPRK TEDTYFISSA GKPTPGTQGK IKLVKESSLS RQSKDSNPYA
1801 TLPRASSVIS TAEGTTRRTS IHDFLTKDSR LPISVDSPPA AADSNTTAAS NVDKVQESRN
1861 SKSRSREQQS S
```

## DETAILED DESCRIPTION

The following explanations of terms and methods are provided to better describe the present disclosure and to guide those of ordinary skill in the art in the practice of the present disclosure. The singular forms "a," "an," and "the" refer to one or more than one, unless the context clearly dictates otherwise. For example, the term "comprising a cell" includes single or plural cells and is considered equivalent to the phrase "comprising at least one cell." The term "or" refers to a single element of stated alternative elements or a combination of two or more elements, unless the context clearly indicates otherwise. As used herein, "comprises" means "includes." Thus, "comprising A or B," means "including A, B, or A and B," without excluding additional elements. Dates of GenBank® Accession Nos. referred to herein are the sequences available at least as early as February 17, 2014.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below. The materials, methods, and examples are illustrative only and not intended to be limiting.

In order to facilitate review of the various embodiments of the disclosure, the following explanations of specific terms are provided:

**Antibody:** Immunoglobulin molecules and immunologically active portions of immunoglobulin molecules, that is, molecules that contain an antigen binding site that specifically binds (immunoreacts with) an antigen (such as GIV-fl, CD34, or MMR proteins). Exemplary antibodies include monoclonal, polyclonal, and humanized antibodies, such as those that are specific for full-length GIV (*e.g.,* can detect the C-terminal amino acids of SEQ ID NO: 1 or 2, such as using the Ab described in Example 1). In some examples, antibodies can be diagnostic, for example used to detect the presence of a protein such as full-length GIV (GIV-fl). In some embodiments, the diagnostic GIV-fl antibody comprises the light chain variable domain and/or heavy chain variable domain of the antibody produced in Example 1. In some examples, a GIV-fl antibody specifically binds to the full-length GIV protein, but not to a GIV protein having a deleted C-terminus (*e.g.,* GIVΔCT, which in some examples lacks the C-terminal 762 or 763 amino acids).

In some examples, an antibody has a high binding affinity for GIV-fl, such as a binding affinity of at least about $1 \times 10^{-8}$ M, at least about $1.5 \times 10^{-8}$, at least about $2.0 \times 10^{-8}$, at least about $2.5 \times 10^{-8}$, at least about $3.0 \times 10^{-8}$, at least

EP 3 108 254 B1

about 3.5 x $10^{-8}$, at least about 4.0 x $10^{-8}$, at least about 4.5 x $10^{-8}$, or at least about 5.0 x $10^{-8}$ M. In certain embodiments, an antibody that binds to full-length GIV has a dissociation constant (Kd) of $\leq$104 nM, $\leq$100 nM, $\leq$10 nM, $\leq$1 nM, $\leq$0.1 nM, $\leq$0.01 nM, or $\leq$0.001 nM (*e.g.,* $10^{-8}$M or less, *e.g.,* from $10^{-8}$M to $10^{-13}$M, *e.g.,* from $10^{-9}$ M to $10^{-13}$ M). In one embodiment, Kd is measured by a radiolabeled antigen binding assay (RIA) performed with the Fab version of an antibody of interest and its antigen (see, *e.g.,* Chen et al., J. Mol. Biol. 293:865-881, 1999). In another example, Kd is measured using surface plasmon resonance assays using a BIACORES-2000 or a BIACORES-3000 (BIAcore, Inc., Piscataway, N.J.) at 25°C with immobilized antigen CM5 chips at about 10 response units (RU). Binding can be measured using a variety of methods standard in the art, including, but not limited to: Western blot, immunoblot, enzyme-linked immunosorbant assay (ELISA), radioimmunoassay (RIA), immunoprecipitation, surface plasmon resonance, chemiluminescence, fluorescent polarization, phosphorescence, immunohistochemical analysis, matrix-assisted laser desorptionlionization time-of-flight mass spectrometry, microcytometry, microarray, microscopy, fluorescence activated cell sorting (FACS), and flow cytometry.

A naturally occurring antibody (such as IgG, IgM, IgD) includes four polypeptide chains, two heavy (H) chains and two light (L) chains interconnected by disulfide bonds. As used herein, the term antibody also includes recombinant antibodies produced by expression of a nucleic acid that encodes one or more antibody chains in a cell (for example see U.S. Patent No. 4,745,055; U.S. Patent No. 4,444,487; WO 88/03565; EP 256,654; EP 120,694; EP 125,023; Faoulkner et al., Nature 298:286, 1982; Morrison, J. Immunol. 123:793, 1979; Morrison et al., Ann Rev. Immunol. 2:239, 1984).

The term antibody also includes an antigen binding fragment of a naturally occurring or recombinant antibody. Specific, non-limiting examples of binding fragments encompassed within the term antibody include Fab, (Fab')$_2$, Fv, and single-chain Fv (scFv). Fab is the fragment that contains a monovalent antigen-binding fragment of an antibody molecule produced by digestion of whole antibody with the enzyme papain to yield an intact light chain and a portion of one heavy chain or equivalently by genetic engineering. Fab' is the fragment of an antibody molecule obtained by treating whole antibody with pepsin, followed by reduction, to yield an intact light chain and a portion of the heavy chain; two Fab' fragments are obtained per antibody molecule. (Fab')$_2$ is the fragment of the antibody obtained by treating whole antibody with the enzyme pepsin without subsequent reduction or equivalently by genetic engineering. F(Ab')$_2$ is a dimer of two FAb' fragments held together by disulfide bonds. Fv is a genetically engineered fragment containing the variable region of the light chain and the variable region of the heavy chain expressed as two chains. Single chain antibody ("SCA") is a genetically engineered molecule containing the variable region of the light chain, the variable region of the heavy chain, linked by a suitable polypeptide linker as a genetically fused single chain molecule. Methods of making these fragments are routine in the art.

**Contact:** To bring one agent into close proximity to another agent, thereby permitting the agents to interact. For example, a GIV-fl antibody can be applied to a microscope slide or other surface containing a biological sample (such as a stage II CRC sample), thereby permitting detection of GIV-fl proteins in the sample that are specifically recognized by the GIV-fl antibody.

**Detect:** To determine if an agent is present or absent. In some examples this can further include quantification. For example, use of an antibody specific for a particular protein (*e.g.,* GIV-fl) permits detection of the protein in a sample, such as a sample containing cancer tissue. In particular examples, an emission signal from a detectable label (such as an increase in the signal if the target is present) is detected. Detection can be in bulk, so that a macroscopic number of molecules can be observed simultaneously. Detection can also include identification of signals from single molecules using microscopy and such techniques as total internal reflection to reduce background noise.

**G-alpha interacting vesicle associated protein (GIV, also known as girdin)** (OMIM 609736): A non-receptor Guanine-nucleotide Exchange Factor (GEF) for Gai. A unique GEF motif in GIV's C terminus is required for activation of Gai (Garcia-Marcos et al., Proc Natl Acad Sci USA, 2009. 106:3178-83). By activating Gai and releasing 'free' G$\beta$y, GIV amplifies Akt signaling via the G$\beta$y-PI3K pathway (Garcia-Marcos et al., Proc Natl Acad Sci USA, 2009. 106:3178-83). A molecular complex comprised of a trimeric G protein, Gai, and GIV is required for growth factors (EGF (Enomoto et al., Dev Cell, 2005. 9:389-402; Ghosh et al., J Cell Biol, 2008. 182:381-93)), IGF (Jiang et al., Cancer Res, 2008. 68(5):1310-8), VEGF (Kitamura et al., Nat Cell Biol, 2008. 10(3):329-37), insulin (Garcia-Marcos et al., Proc Natl Acad Sci USA, 2009. 106:3178-83; Ghosh et al., J Cell Biol, 2008. 182:381-93, Anai et al., J Biol Chem, 2005. 280:18525-35) to enhance Akt, remodel actin and trigger cell migration.

GIV's C-terminus directly binds the autophosphorylated cytoplasmic tails of EGFR, and thereby links G protein to ligand-activated receptors. When GIV's C-terminus is intact a Gai-GIV-EGFR signaling complex is assembled, EGFR autophosphorylation is enhanced, and the receptor's association with the plasma membrane (PM) is prolonged. Accordingly, PM-based signals that trigger motility (PI3K-Akt and PLC$\gamma$1) are amplified, actin is remodeled, and cell migration is triggered (Ghosh et al., Mol. Biol. Cell. 2010. 21:2338-54). Thus, GIV's C-terminus serves as a common platform which links ligand-activated receptors (Ghosh et al., Mol. Biol. Cell. 2010. 21:2338-54) at the leading edge to actin (Enomoto et al., Dev Cell, 2005. 9:389-402), Akt (Enomoto et al., Dev Cell, 2005. 9:389-402; , Anai et al., J Biol Chem, 2005. 280:18525-35) and Gai (Garcia-Marcos et al., Proc Natl Acad Sci USA, 2009. 106:3178-83), three components whose interplay is essential for cell migration.

GIV is a tyrosine phosphoprotein that directly binds to and activates phosphoinositide 3-kinase (PI3K). Upon ligand stimulation of various receptors, GIV is phosphorylated at tyrosine-1764 and tyrosine-1798 by both receptor and non-receptor tyrosine kinases. These phosphorylation events enable direct binding of GIV to the amino- and carboxyl-terminal Src homology 2 domains of p85α, a regulatory subunit of PI3K; stabilize receptor association with PI3K; and enhance PI3K activity at the plasma membrane to trigger cell migration.

The human GIV gene (*CCDC88A*) in some examples encodes a protein of 1870 amino acids (1871 in an isoform of human GIV) with a predicted molecular mass of about 220 kDa. The structure of GIV can be divided into several different regions: a microtubule-binding hood domain (amino acids 1-195), coiled-coil oligomerization domain (amino acids 196-1304), a G-alpha binding domain (amino acids 1343-1424), a phosphoinositide (PI4P)-binding domain (amino acids 1390-1408), an Akt, actin and receptor binding domain (amino acids 1623-1870), an SH2 domain (amino acids 1714-1815) which is about15 aa downstream of the GEF motif (amino acids 1674-1694). GIV mRNA expression is upregulated during metastatic progression in several cancers, including breast, colorectal, lung, malignant melanoma, renal cell carcinoma, gastric carcinomas, pancreatic carcinoma, esophageal carcinoma, and thyroid carcinoma.

GIV sequences are publically available, for example from GenBank® sequence database (*e.g.*, accession numbers NP-001129069 and BAE44387.1 (protein), and AB201172.1 and NM_001135597.1 (nucleic acid)). GenBank® accession number Q3V6T2 provides a 1871 aa GIV-fl variant. One of ordinary skill in the art can identify additional GIV nucleic acid and protein sequences, including GIV variants.

**Hybridization:** To form base pairs between complementary regions of two strands of DNA, RNA, or between DNA and RNA, thereby forming a duplex molecule. Hybridization conditions resulting in particular degrees of stringency will vary depending upon the nature of the hybridization method and the composition and length of the hybridizing nucleic acid sequences. Generally, the temperature of hybridization and the ionic strength (such as the $Na^+$ concentration) of the hybridization buffer will determine the stringency of hybridization. The presence of a chemical which decreases hybridization (such as formamide) in the hybridization buffer will also determine the stringency (Sadhu et al., J. Biosci. 6:817-821, 1984). Calculations regarding hybridization conditions for attaining particular degrees of stringency are discussed in Sambrook et al., (1989) Molecular Cloning, second edition, Cold Spring Harbor Laboratory, Plainview, NY (chapters 9 and 11). Hybridization conditions for ISH are also discussed in Landegent et al., Hum. Genet. 77:366-370, 1987; Lichter et al., Hum. Genet. 80:224-234, 1988; and Pinkel et al., Proc. Natl. Acad. Sci. USA 85:9138-9142, 1988.

**Label:** An agent capable of detection, for example by spectrophotometry, flow cytometry, or microscopy (such as light microscopy). For example, one or more labels can be attached to an antibody, thereby permitting detection of the target protein (such as GIV). Exemplary labels include radioactive isotopes, fluorophores, ligands, chemiluminescent agents, haptens, enzymes, and combinations thereof.

**Lymphovascular invasion (LVI):** The invasion of tumor cells, such as CRC cells, into blood vessels or lymphatic vessels. If tumor cells are present in blood vessels or lymphatic vessels, the LVI status of the subject or tumor is positive. If tumor cells are not present in blood vessels or lymphatic vessels, the LVI status of the subject or tumor is negative. However, if the subject or tumor is LVI positive, this does not necessarily mean that the tumor has spread to the lymph nodes.

**Normal cells or tissue:** Non-tumor, non-malignant cells and tissue.

**Mis-match repair proficient (pMMR):** A tumor, such as a CRC, is said to be pMMR positive if the DNA mismatch repair system (MMR) is proficient, as contrasted with a tumor with a deficient MMR (dMMR) system. Cancers with pMMR can show low-frequency microsatellite instability (MSI). Routine methods can be used to determine the presence or absence of MMR, such as by MSI testing using PCR or analysis of MMR proteins, MLH1, PMS2, MSH2 and MSH6 expression by IHC.

**Primer:** An oligonucleotide which hybridizes with a sequence in the target nucleic acid and is capable of acting as a point of initiation of synthesis along a complementary strand of nucleic acid under conditions suitable for such synthesis. A perfect complementarity is not required for the primer extension to occur. However, a primer with perfect complementarity (especially near the 3'-terminus) will be extended more efficiently than a primer with mismatches, especially mismatches at or near the 3'-terminus.

**Probe:** an oligonucleotide which hybridizes with a sequence in the target nucleic acid and may be detectably labeled. The probe can have modifications, such as a 3'-terminus modification that makes the probe non-extendable by nucleic acid polymerases; and one or more chromophores.

**Quantitative PCR or quantitative RT-PCR:** a nucleic acid amplification reaction wherein the target nucleic acid is quantitatively detected. QPCR is characterized by a "growth curve" which is a graph of a function, where an independent variable is the number of amplification cycles and a dependent variable is an amplification-dependent measurable parameter (such as the amount of fluorescence emitted by a specific probe upon hybridization, or upon the hydrolysis of the probe by the nuclease activity of the nucleic acid polymerase) is measured at each cycle of amplification, *see* Holland et al., (1991) Proc. Natl. Acad. Sci. 88:7276-7280 and U.S. Patent No. 5,210,015. The amplification-dependent measurable parameter reflects among other variables, the initial amount of the target nucleic acid. A growth curve is typically characterized by a "cycles to threshold" value or "$C_t$ value," which is a number of cycles where a predetermined magnitude of the measurable parameter is achieved. A lower or "earlier" $C_t$ value reflects a greater amount of the input target nucleic acid, while the higher or "later" $C_t$ value represents a lower amount of the input target nucleic acid.

**Sample:** A biological specimen containing genomic DNA, RNA (including mRNA), protein, intact cells (*e.g.,* a tissue sample), or combinations thereof, obtained from a subject. Examples include a specimen containing at least one cancer cell (a cancer sample or cancer tissue sample), for example, a surgical resection specimen, a tissue or tumor biopsy, fine needle aspirate, bronchoalveolar lavage, pleural fluid, sputum, surgical specimen, lymph node, a metastasis, or autopsy material. In other examples, a sample includes a control sample, such as a non-cancerous cell or tissue sample. In one example the control is a negative control, such as a sample known to not include detectable GIV-fl protein (hepatocytes in a liver sample do not stain with GIV-fl antibodies). In another example, the control is a positive control, such as a sample known to include detectable GIV-fl (such as a sample of COS-7 cells; ATCC Catalog No. CRL-1651, or non-hepatocytes in a liver sample such as sinusoids, stellate cells).

**Specific binding agent:** An agent that binds substantially or preferentially only to a defined target such as a protein, for example a GIV-fl protein. In some examples, a GIV-fl specific binding agent specifically binds to the full-length GIV protein, but not to a GIV protein having a deleted C-terminus (*e.g.,* GIVΔCT, which in some examples lacks the C-terminal 762 or 763 amino acids).

A GIV-fl protein-specific binding agent binds substantially only to GIV-fl protein, or to a specific region within the GIV-fl protein (such as the C-terminus). For example, a "GIV-fl specific binding agent" includes antibodies and other agents, such as aptamers, that bind substantially to a GIV-fl polypeptide. Antibodies can be monoclonal or polyclonal antibodies that are specific for the polypeptide, as well as immunologically effective portions ("fragments") thereof. The determination that a particular agent binds substantially only to a GIV-fl polypeptide may readily be made by using or adapting routine procedures. One suitable *in vitro* assay makes use of the Western blotting procedure (described in many standard texts, including Harlow and Lane, Using Antibodies: A Laboratory Manual, CSHL, New York, 1999).

**Subject:** Living multi-cellular vertebrate organisms, a category that includes human and non-human mammals, such as veterinary subjects. In a particular example, a subject is one who has or is suspected of having cancer, such as colorectal cancer, for example stage II CRC (such as stage IIa or IIb CRC). In some examples the stage II CRC is T3, T3/4, or T4. In some examples, the subject is chemo-naïve (that is, has not previously received chemotherapy or biotherapy treatment for their CRC). In some examples, the subject is not chemo-naïve.

**Therapeutically effective amount:** A dose sufficient to prevent advancement, delay progression, or to cause regression of a disease, or which is capable of reducing symptoms caused by the disease, such as cancer, for example colorectal cancer (*e.g.,* stage II CRC). In one example, a therapeutically effective amount is an amount of a chemotherapy or biotherapy sufficient to reduce the size or volume of a tumor, or the number of tumors (such as the number of metastases) by at least 10%, at least 20%, at least 50%, at least 70%, or at least 90%, such as reduce the size, volume or number (such as metastases) of CRC tumors by at least 10%, at least 20%, at least 50%, at least 70%, or at least 90%.

**Under conditions sufficient for:** A phrase that is used to describe any environment that permits the desired activity. An example includes contacting an antibody with a biological sample sufficient to allow detection of one or more target proteins (*e.g.,* GIV-fl) in the sample.

**Overview**

The routine management of patients with stage II colorectal cancer (CRC) remains challenging. About 25% of stage II CRC patients receive adjuvant chemotherapy and/or biotherapy (commonly younger patients and those with high risk as defined by clinicopathological characteristics). Ultimately, a small but definite benefit (about 3% absolute improvement in overall survival) is observed with adjuvant chemotherapy, especially in patients with tumors that are mis-match repair proficient (pMMR).

The present application provides methods, as defined in the claims, that allow for classification of a patient with stage II (such as stage IIa or IIb) colorectal cancer that is pMMR as high- or low-risk, that is, whether the CRC is likely to progress or recur. For example, CRC can progress or recur locally (cancer reappears in the same area), to local nodes (*e.g.,* progression to CRC stage III), or to distant nodes or other organs such as the liver or lung (*e.g.,* progression to CRC stage IV). The disclosed methods are useful as patients at high-risk for progression or recurrence are more likely to show a benefit from chemotherapy or biotherapy than those having a low-risk CRC for progression. The claimed methods may be followed by treating patients identified as having a high-risk CRC with chemotherapy or biotherapy. It is shown herein that determination of expression of full-length G-alpha interacting vesicle associated protein (GIV-fl) (for example by scoring GIV-fl expression), and determination of the lymphovascular invasion (LVI) status of the subject, can predict with high accuracy those stage II CRCs that are likely to recur (for example within at least 2 years or at least 5 years), and thus are high-risk, and those that are not likely to recur and thus are low-risk. In some examples, the methods also use one or more other characteristics of the subject, such as the age of the subject at diagnosis, number of lymph nodes that are positive for CRC, sex of the subject, state of tumor differentiation, T stage of the cancer; and on which side the colon cancer was present, to determine whether the stage II CRC is likely to recur, and thus is a high-risk tumor that should be treated with chemotherapy or biotherapy. In some examples, the one or more characteristics of the subject include one or more of: the tumor grade, LVI, number of lymph nodes examined, whether there is perineural invasion, whether there is localized perforation, and whether the margins are indeterminate or positive.

Full-length GIV protein (GIV-fl), and more specifically its C-terminus (such as the C-terminal 762 or 763 amino acids of SEQ ID NO: 1 or SEQ ID NO: 2, respectively), which contains the key motifs (EGFR-binding, Akt/actin-binding and GEF) is dysregulated in breast and colorectal tumor cells by alternative splicing (Ghosh et al., Mol. Biol. Cell. 2010. 21:2338-54). In poorly invasive cancer cells and in pre-invasive, early-staged colorectal carcinomas GIV-fl is replaced by a C-terminally truncated variant, GIVΔCT. In highly invasive cancer cells and late-stage invasive carcinomas, GIV-fl is highly expressed. Consequently, only some tumor cells and tumors, but not all, express GIV-fl which contains a functionally intact C-terminus and GIV-fl expression correlates with metastatic progression.

Despite the breadth of information available on the molecular and biological functions of GIV-fl during cancer invasion and angiogenesis (Garcia-Marcos et al., Proc Natl Acad Sci USA, 2009. 106(9):3178-83; Garcia-Marcos et al., J Biol Chem. 2010. 285:12765-77; Enomoto et al., Dev Cell, 2005. 9(3):389-402; Ghosh et al., J Cell Biol, 2008. 182(2):381-93; Jiang et al., Cancer Res, 2008. 68(5):1310-8; Kitamura et al., Nat Cell Biol, 2008. 10(3):329-37; Anai et al., J Biol Chem, 2005. 280(18):18525-35; Ghosh et al., Mol. Biol. Cell. 2010. 21:2338-54; Enomoto et al., Ann N Y Acad Sci, 2006. 1086:169-84; Weng et al., Cancer Sci. 2010. 101:836-42), and the observation that the presence of the GIV C-terminus can distinguish the highly metastatic from poorly metastatic cancers (colon, breast, and pancreas) and thereby prognosticate survival among colorectal cancer patients (Garcia-Marcos et al., Faseb J. 2011. 25:590-99), it remains undetermined whether expression of GIV-fl can be used in combination with other indicators to determine which stage II colorectal patients having a poor prognosis (*e.g.,* those that are mis-match repair proficient (pMMR)), will benefit from adjuvant chemotherapy or biotherapy.

The present disclosure provides methods for analyzing or characterizing a stage II (such as stage IIa or stage IIb) pMMR CRC, by using a CRC sample obtained from a subject. In some examples, the methods distinguish a subject with stage II pMMR CRC (such as one who is chemo-naïve) who is likely to respond to treatment with chemotherapy or biotherapy from a subject (such as one who is chemo-naïve) with stage II pMMR CRC who is not likely to respond to treatment with chemotherapy or biotherapy. The disclosed methods can also be used to predict the likely progression free survival (PFS) of the subject, for example predicting whether the subject will have a PFS of at least 1 year, at least 2 years, at least 3 years, at least 4 years, at least 5 years, at least 6 years, or at least 7 years without progression or recurrence of the cancer.

The method includes contacting a stage II CRC sample (*e.g.,* that is T3 or T3/4) with specific binding agent that is specific for GIV-fl. Such a specific binding agent, such as an antibody, can specifically bind to GIV-fl, but not C-terminal truncations of GIV (GIVΔCT, such as GIV proteins missing the C-terminal 762 or 763 amino acids). This allows for the determination of the presence of GIV-fl in the sample. In some examples, an automated tissue stainer is used for application of the GIV-fl primary antibodies (and additional antibodies or other IHC reagents).

Expression of the GIV-fl protein is scored, for example by detecting its staining in the sample, to determine the GIV-fl status (*e.g.,* positive or negative) of the sample. In one example, scoring expression of the GIV-fl protein includes examining the sample and characterizing or determining the percent of overall GIV-fl staining and the predominant GIV-fl staining intensity. In some examples, scoring expression of GIV-fl protein includes visual inspection of GIV-fl staining, for example using light microscopy. In some examples, scoring GIV-fl expression includes a visual inspection of the total area of the sample, for example that is present on a microscope slide. In some examples, scoring GIV-fl expression includes an inspection of the total area of the sample, for example using a slide imager. Scoring GIV-fl expression can include direct or indirect detection of binding of the GIV-fl protein specific binding agent to the sample.

In some examples, a microscope slide is processed and/or imaged using a slide scanner. Slide scanners are known in the art, and can include those disclosed in U.S. Patent Nos. 8,625,930; 8,609,023, and 8,290,236 as well as those available from VMSI. In some examples, image analysis is used to evaluate or discern staining patterns, such as GIV-fl expression. For example, automated scoring of GIV-fl expression using digital images of the slides and computer based image analysis can be used, such as those disclosed in U.S. Patent Nos. 8,625,930; 8,537,181; 8,515,683; and 8,428,887; as well as those available from VMSI.

In one example, the method of scoring GIV-fl expression includes determining an extent of positive GIV-fl staining for the sample on a scale of 0 to 3, wherein extent of positive staining is assigned 0 if 0% to 10% of the total area of the sample is stained positive, wherein extent of positive staining is assigned 1 if 11 %-35% of the total area of the sample is stained positive, wherein extent of positive staining is assigned 2 if 36%-50% of the total area of the sample is stained positive, and wherein extent of positive staining is assigned 3 if 51%-100% of the total area of the sample is stained positive. In addition, a GIV-fl intensity of staining for the sample on a scale of 0 (negative), 1 (weak), 2 (moderate), to 3 (strong) is determined. For example, a sample can be scored negative (0) if there is an absence of any detectable signal or pale gray/tan signal which is similar to the intensity on the negative control reagent; a sample can be scored weak (1) if there is by light staining intensity (*e.g.,* light brown) which is more than that seen on the negative control reagent and in the background; a sample can be scored moderate (2) if there is moderate staining intensity (*e.g.,* brown); or a sample can be scored strong (3) intensity if there is dark staining intensity (*e.g.,* brown to black signal intensity). The color detected will depend on the detection system used. In some examples, the extent of positive GIV-fl staining and the GIV-fl intensity of staining are inputted into a computer or algorithm. The extent of positive GIV-fl staining and the GIV-fl intensity of staining are summed, thereby generating a GIV-fl score value from 0 to 6. Based on this value from 0 to 6, it is determined that the sample is GIV-fl negative if the GIV-fl score value is 0-2 or that the sample is GIV-fl positive if the GIV-fl score value is 3-6. In some examples, the GIV-fl score value and/or the GIV-fl status of the sample (*e.g.,* positive or negative) is outputted by a computer or algorithm, for example in a visual or audible output. In some examples, the GIV-fl score value and/or the GIV-fl status of the sample (*e.g.,* positive or negative) is inputted into an algorithm.

In another example, called the GIV-fl Predictive Score, scoring GIV-fl protein expression for the sample includes determining if the total area of GIV-fl staining with any intensity for the sample is greater than 10% and/or determining if the GIV-fl staining intensity is strong (3+) any percent positive. For example, a sample can be scored negative (0) if there is an absence of any detectable signal or pale gray/tan signal which is similar to the intensity on the negative control reagent; a sample can be scored weak (1+) if there is by light staining intensity *(e.g.,* light brown) which is more than that seen on the negative control reagent and in the background; a sample can be scored moderate (2+) if there is moderate staining intensity (*e.g.,* brown); or a sample can be scored strong (3+) intensity if there is dark staining intensity (*e.g.,* brown to black signal intensity). The color detected will depend on the detection system used. Based on this information, the sample is assigned GIV-fl positive if the total area of GIV-fl staining for the sample is greater than 10% with any intensity or if the GIV-fl staining intensity is strong (3+) with any percent positive; or the sample is assigned GIV-fl negative if the total area of GIV-fl staining with a staining intensity of 0, 1+, or 2+ for the sample is less than 10%. In some examples, the GIV-fl status of the sample (*e.g.,* positive or negative) is outputted by a computer or algorithm, for example in a visual or audible output. In some examples, the GIV-fl status of the sample (*e.g.,* positive or negative) value is inputted into an algorithm.

In another example, GIV-fl expression is assessed by quantifying the GIV-fl mRNA. The method includes isolating RNA from the tumor sample and quantitatively detecting the GIV-fl mRNA using a specific nucleotide probe via *e.g.*, quantitative reverse transcription polymerase chain reaction (qRT-PCR). In some examples, relative amount of the GIV-fl mRNA is determined by comparing the absolute amount of the GIV-FL mRNA to that of a housekeeping gene. In yet other examples, the relative amount of the GIV-fl mRNA in a tumor sample is compared to the relative amount of the GIV-fl mRNA in a control, *e.g.*, non-tumor sample. The tumor sample is assigned GIV-fl positive if the relative amount of the GIV-fl mRNA is substantially higher than the relative amount of the GIV-fl mRNA in a control sample.

The method also includes determining the lymphovascular invasion (LVI) status of the subject (*e.g.,* positive or negative), for example by examining the sample to determine if there was invasion of the CRC into blood vessels or lymphatic vessels. Such a determination can be made using routine method, such as microscopy of a sample stained with H&E or antibodies, such as an antibody specific for the lymphatic endothelium (*e.g.,* Ab D2-40) or a CD34 antibody.

The method can optionally include determining one or more characteristics of the subject. In some examples, the one or more characteristics of the subject are outputted by a computer or algorithm, for example in a visual or audible output. In some examples, the one or more characteristics of the subject are inputted into an algorithm. Exemplary characteristics include but are not limited to the age of the subject at diagnosis, number of lymph nodes that are positive for CRC, sex of the subject (male or female), state of tumor differentiation (*e.g.,* moderate, poor or well), T stage of the CRC (*e.g.,* T1, T2, T3, T4, or T3/4, which reflects the size and/or extent of the primary tumor), and on which side the colon cancer was present (*e.g.,* right or left). Other exemplary characteristics of the subject that can be determined an inputted include but are not limited to: the tumor grade, LVI, number of lymph nodes examined, whether there is perineural invasion, whether there is localized perforation, and whether the margins are indeterminate or positive.

The method can include inputting the GIV-fl status, LVI status, and optionally one or more of the subject's characteristics into a computer or algorithm, and then generating an output from the computer or algorithm, thereby analyzing or characterizing the sample. For example, the output can be an indication as to whether the CRC analyzed is one that is high-risk (likely to recur or progress) or whether the CRC analyzed is one that is low-risk (not likely to recur or progress). For example, the output can be a visual or audible "high-risk", "low-risk" or provide an indication that chemotherapy or biotherapy should or should not be administered to the subject.

In some examples, the method includes selecting a subject for treatment with chemotherapy or biotherapy if the subject is identified as a subject (*e.g.*, chemo-naïve subject) who is likely to respond to treatment with chemotherapy or biotherapy. In some examples, such subjects are to be administered a therapeutically effective amount of one or more appropriate chemotherapies or biotherapies, such as 5-fluouracil, leucovorin, panitumumab (Vectibix®), cetuximab (Erbitux®), bevacizumab (Avastin®), ziv-aflibercept (Zaltrap®), irinotecan (Camptosar®), oxaliplatin (Eloxatin®), or combinations thereof. In contrast, if the subject is identified as a subject (*e.g*., chemo-naïve subject) who is not likely to respond to treatment with chemotherapy or biotherapy, such subjects are not to be administered chemotherapy or biotherapy, but can be assigned for close monitoring.

Samples analyzed using the disclosed methods are routine, and can include a tissue biopsy (such as a tissue section) or fine needle aspirate. In some examples, the sample is a fixed sample, such as a formalin fixed, paraffin embedded (FFPE) sample.

One or more steps of the disclosed methods can be performed by a suitably-programmed computer.

Also provided is a computer-implemented method. Such a method can include generating a GIV-fl protein expression score based at least on measured GIV-fl protein expression within a displayed image depicting a stage II pMMR sample detectably labeled with a GIV-fl antibody, wherein the CRC sample has been obtained from a subject. The GIV-fl protein expression score can be generated by (i) determining an extent of positive GIV-fl staining for the sample on a scale of 0 to 3, wherein extent of positive staining is assigned 0 if 0% to 10% of the total area of the sample is stained positive, wherein extent of positive staining is assigned 1 if 11%-35% of the total area of the sample is stained positive, wherein extent of positive staining is assigned 2 if 36%-50% of the total area of the sample is stained positive, and wherein extent of positive staining is assigned 3 if 51%-100% of the total area of the sample is stained positive; a GIV-fl intensity of staining for the sample on a scale of 0 (negative), 1 (weak), 2 (moderate), to 3 (strong) as described above, summing the extent of positive GIV-fl staining and the GIV-fl intensity of staining, thereby generating a GIV-fl score value from 0 to 6, and determining that the sample is GIV negative if the GIV-fl score value is 0-2 or determining that the sample is GIV positive if the GIV-fl score value is 3-6; or (ii) determining if a total area of GIV-fl staining with any intensity for the sample is greater than 10% or determining if GIV-fl staining intensity is strong (3+) using the scoring methods described above; and determining that the sample is GIV positive if the total area of GIV-fl staining for the sample with any intensity is greater than 10% or if the GIV-fl staining intensity is strong (3+) any percent positive; or determining that the sample is GIV negative if the total area of GIV-fl staining for the sample with a staining intensity of 0, 1+, or 2+ is less than 10%. The resulting GIV-fl protein expression score for the sample can be outputted by the computer, for example to a user or to an algorithm.

The computer-implemented method can further include inputting the lymphovascular invasion (LVI) status of the subject and optionally inputting one or more characteristics of the subject, wherein the one or more characteristics include age of the subject at diagnosis, number of lymph nodes that are positive for CRC, sex of the subject, state of tumor differentiation, T stage of the cancer; and on which side the colon cancer was present (other exemplary characteristics of the subject that can be inputted include but are not limited to: the tumor grade, LVI, number of lymph nodes examined, whether there is perineural invasion, whether there is localized perforation, and whether the margins are indeterminate or positive), and outputting a prognosis or diagnosis for the subject. For example, the output can be an indication as to whether the CRC is high-risk or low-risk, and thus the patient should or should not receive chemotherapy/biotherapy, respectively.

Also disclosed are one or more non-transitory computer-readable media that include computer-executable instructions causing a computing system to perform the methods provided herein.

Systems for analyzing a stage II (such as stage IIa or IIb) mis-match repair proficient (pMMR) colorectal cancer (CRC) sample obtained from a subject are also described. Such systems can include a means for measuring a level of GIV-fl in the sample (such as a GIV-fl-specific antibody); and means for determining the LVI status of the subject (such as H&E or antibodies specific for LVI markers such as CD34 and/or lymphatic endothelium). In some examples, such means include a light microscope, automated tissue or slide stainer, computer, or combinations thereof. In some examples, the system includes implemented rules for determining the GIV-fl status of the sample (*e.g.,* positive or negative) based on the measured level of GIV-fl. Methods of scoring GIV-fl expression are discussed herein. In some examples, the system includes implemented rules for comparing the measured level of GIV-fl to a GIV-fl reference value, such as a GIV-fl positive or negative control. In some examples, the system includes implemented rules for determining the LVI status of the sample (*e.g.,* positive or negative), for example based on the measured level of LVI markers or other histological or structural markers (*e.g.,* H&E staining). In some examples, the system includes implemented rules for comparing the measured LVI markers to an LVI reference value or sample, such as an LVI positive or negative control. In some examples, the GIV-fl and/or LVI reference values are stored values or stored digital images. In some examples, the GIV-fl and/or LVI reference values are a level of GIV-fl and/or LVI measured from a control sample by said means for measuring. The system can also include one or more means for implementing the rules (such as a computer or algorithm), whereby an indication of the likely risk of CRC recurrence and/or likely response of the CRC to chemotherapy or biotherapy is provided based on the GIV-fl and LVI status. In some examples, the system also includes a means for determining one or more characteristics of the subject (or means for imputing such characteristics into an algorithm, such as a keyboard or computer program), wherein the one or more characteristics include age of the subject at diagnosis, number of lymph nodes that are positive for CRC; sex of the subject, state of tumor differentiation, T stage of the cancer; and on which side the colon cancer was present; and implemented rules for comparing the measured level of one or more characteristics to a reference value for the one or more characteristics.

The invention also provides kits that can be used with the methods provided herein. In one example, the kit includes a GIV-fl specific-binding agent, such as antibody clone SP173. The kit includes: a specific-binding agent that permits for a determination of LVI comprising an antibody specific for CD34 or lymphatic endothelium; a mis-match repair protein specific-binding agent comprising an antibody specific for mutL homolog 1 (MLH1); postmeiotic segregation increased 2 (PMS2); MutS protein homolog 2 (MSH2), MutS protein homolog 6 MSH6, or a combination of such antibodies; optionally antibodies specific for other tumor markers, such as EGFR, Bax, Bcl, p53, and the like; and optionally microscope slides (such as glass slides and coverslips); labeled secondary antibodies (such as those that include a detectable label and permit detection of primary antibodies in the kit); and buffers for IHC (such as CC1 buffer from VMSI). In another embodiment, the kit contains reagents for measuring GIV-fl mRNA expression. In this embodiment, the kit contains a nucleic acid probe capable of specifically hybridizing to the GIV-fl mRNA sequence. The kit may also contain reagents for reverse-transcription PCR (RT-PCR), including one or more of the oligonucleotide primers specific for the GIV-fl mRNA, oligonucleotide primers and one or more probes specific for the control gene, *e.g.,* a "house-keeping" gene, nucleoside triphosphates, one or more DNA polymerases to provide reverse transcription and DNA replication activities and buffers and cofactors to support the activity of the one or more DNA polymerases.

**Methods of Analyzing Stage II pMMR Colorectal Cancer Samples**

Disclosed herein are methods for analyzing stage II (such as IIa and IIb, also referred to as T3, and T4, respectively) mis-match repair proficient (pMMR) colorectal cancer (CRC) samples obtained from a subject. A stage IIa CRC is generally characterized as a tumor that invades though the muscularis propria into the subserosa, or into non-peritonealized pericoloic or perirectal tissues, while a stage IIb CRC is when the tumor directly invades other organs or structures, and/or perforates visceral peritoneum. In contrast, stage IIc is when the CRC has spread through the serosa of the colon wall but has not spread to nearby organs. In some examples such methods are used to prognosticate a good or bad outcome for the subject from whom the sample was obtained. For example, the methods can determine if it is likely that the CRC will progress at least 1, 2, 3, 4, 5, 6, or even 7 years following the original diagnosis. For example, if the method predicts that the CRC will progress (*e.g.,* likely to recur or metastasize following surgery) this indicates that the CRC is high-risk and the subject should receive chemotherapy or biotherapy. In contrast, if the method predicts that the CRC will not progress (*e.g.,* not likely recur or metastasize following surgery) this indicates that the CRC is low-risk and the subject should not receive chemotherapy or biotherapy (as they will not likely receive a benefit). Thus, the method can be used as a chemopredictor, to identify subjects with stage II pMMR CRC likely to benefit from receiving chemotherapy or biotherapy.

The methods can include determining if the stage II (such as IIa or IIb) pMMR CRC is positive or negative for GIV-fl protein expression. For example, the method can include scoring GIV-fl protein expression. CRC pMMR tumors that are GIV-fl positive generally have a worse prognosis than those that are GIV-fl negative (*e.g.,* cancer more likely to progress within 1, 2, 3, 4, 5, 6, or even 7 years than a subject with a GIV-fl negative CRC), as GIV-fl positive CRCs are more invasive. That is, GIV-fl is an adverse prognosticator. However, not all GIV-fl positive CRCs will respond to chemotherapy or biotherapy. Thus, the inventors identified additional clinical variables that can identify those stage II pMMR GIV-fl positive CRC cancers that will likely respond to chemotherapy or biotherapy. Such methods can be used to identify a subject as having a stage II pMMR CRC that is predicted to respond to treatment with chemotherapy or biotherapy. In some examples, such subjects have been previously been treated with a chemotherapeutic or biotherapeutic for their CRC. In other examples, such subjects have not been previously been treated with a chemotherapeutic or biotherapeutic for their CRC (chemo-naive).

The disclosed methods include detecting or measuring GIV-fl expression, to provide or determine a GIV-fl status of the sample. Such a status can include one or more of a GIV-fl protein score (such as 0, 1, 2, 3, 4, 5 or 6), GIV-fl positive, or GIV-fl negative. Such methods can include contacting a sample that contains CRC cells (such as a sample obtained from a subject) with a GIV-fl protein specific binding agent. Examples of GIV-fl protein specific binding agents include antibodies, such as monoclonal antibodies (*e.g.,* rabbit monoclonal antibody clone SP173, see Example 1), polyclonal antibodies, chimeric antibodies, and fragments thereof, as well as aptamers, which bind to GIV-fl, but not GIVΔCT, with high specificity. The GIV-fl protein specific binding agents can be incubated with the sample under conditions that allow the GIV-fl protein specific binding agents to bind to GIV-fl proteins in the sample. In one example, the GIV-fl protein expression in the CTC sample is detected or measured, and then based on these measurements, a GIV-fl protein expression score is determined for the sample. Based on this score, it is determined if the sample is GIV-fl positive or negative.

In one example, the GIV-fl score for the CRC sample is determined as follows. The extent of positive GIV-fl staining for the sample is scored on a scale of 0 to 3, wherein the extent of positive staining is assigned 0 if 0% to 10% of the total area of the sample stains positive for GIV-fl, wherein the extent of positive staining is assigned 1 if 11%-35% of the total area of the sample stains positive for GIV-fl, wherein the extent of positive staining is assigned 2 if 36%-50% of the total area of the sample stains positive for GIV-fl, and wherein the extent of positive staining is assigned 3 if 51%-100% of the total area of the sample stains positive for GIV-fl. In addition, a GIV-fl intensity of staining for the sample on a scale of 0 (negative), 1 (weak), 2 (moderate), to 3 (strong) is determined or calculated using the methods described above. In some examples, the value for each of the extent of positive GIV-fl staining and the GIV-fl intensity of staining e are inputted into a computer or algorithm. The value for each of the extent of positive GIV-fl staining and the GIV-fl intensity of staining are summed, thereby generating a GIV-fl score value from 0 to 6. Based on this value from 0 to 6, it is determined that the sample is GIV-fl negative if the GIV-fl score value is 0-2 or that the sample is GIV-fl positive if the GIV-fl score value is 3-6.

In another example the GIV-fl score for the CRC sample is determined as follows. The sample is analyzed to determine if the total area of GIV-fl staining for the sample is greater than 10% and/or to determine if GIV-fl staining intensity is strong (3+) using the methods described above. Based on this information, the sample is assigned GIV-fl positive if the total area of GIV-fl staining with any staining intensity for the sample is greater than 10% or if the GIV-fl staining intensity is strong (3+) any percent; or the sample is assigned GIV-fl negative if the total area of GIV-fl staining for the sample with a staining intensity of 0, 1+, or 2+ is less than 10%.

Methods of using antibodies and other protein specific binding agents to detect a target protein in a sample are routine, and the disclosure is not limited to particular methods. In some examples, IHC is utilized, for example in combination with light microscopy. For example, GIV-fl protein expression in the sample can be directly or indirectly detected by binding of the GIV-fl protein specific binding agent to the sample. In some examples, contacting the sample with the GIV-fl protein specific binding agent and detecting the GIV-fl protein expression in the sample are performed with an automated tissue stainer. In some examples, detecting GIV-fl protein expression utilizes visual inspection, for example by utilizing light microscopy.

Methods of detecting mRNA expression using mRNA isolated from a sample are likewise routine. The methods include quantitative reverse transcription PCR (RT-PCR), e.g. with TaqMan® probes, or PCR-free systems such as the Invader® assay (Third Wave Technologies), hybridization to immobilized probes, *e.g.,* as a part of a microarray, or any other method of quantifying mRNA that is or will become available.

The method also relies on knowing the lymphovascular invasion (LVI) status of the subject (*e.g.,* positive or negative). Methods of determining LVI are routine, such as using microscopy. For example, the CRC sample can be analyzed to determine if there was invasion of the CRC into blood vessels or lymphatic vessels, for example using microscopy. The LVI status can be used in combination with the GIV-fl status to determine whether a stage II pMMR CRC is likely or not likely to respond to chemotherapy or biotherapy.

The method can optionally include determining one or more characteristics of the subject. In some examples, the one or more characteristics of the subject are outputted by a computer or algorithm, for example in a visual or audible output. In some examples, the one or more characteristics of the subject are inputted into an algorithm. Exemplary characteristics include but are not limited to the age of the subject at diagnosis, number of lymph nodes that are positive for CRC, sex of the subject (male or female), state of tumor differentiation (*e.g.,* moderate, poor or well), T stage of the CRC (*e.g.,* T1, T2, T3, T4, or T3/4, which reflects the size and/or extent of the primary tumor), and on which side the colon cancer was present (*e.g.,* right or left). The GIV-fl status, in combination with one or more characteristics of the subject, and in some examples also in combination with LVI status, can be used to determine whether a stage II pMMR CRC is likely or not likely to respond to chemotherapy or biotherapy.

In some examples, the disclosed methods are methods of identifying a subject as having a stage II pMMR CRC likely to or predicted to respond to treatment to chemotherapy or biotherapy. For example, the method can include determining that the subject will benefit from treatment with chemotherapy or biotherapy if the tumor sample obtained from the subject is GIV-fl positive and the patient is LVI positive. Such identified subjects can be selected for treatment with chemotherapy. In addition, the method can be followed by administering to such identified and selected subjects a therapeutically effective amount of chemotherapy or biotherapy. In contrast, the method can include determining that the subject will not benefit from treatment with chemotherapy if the tumor sample obtained from the subject is GIV-fl negative and the patient is LVI negative.

In some examples the GIV-fl protein expression in the test sample is compared to a control, such as a positive and/or a negative control. Thus, in some examples the method includes detecting or measuring GIV-fl protein expression in one or more control samples. For example hepatocytes in a liver sample do not endogenously express GIV-fl, and thus should score a 0 for staining intensity or less than 10% for total area stained for GIV-fl protein expression. In contrast, COS-7 cells express GIV-fl and thus should score a 2 or 3 for staining intensity or greater than 50% for total area stained for GIV-fl protein expression.

One or more steps of the disclosed methods can be performed by a system or suitably-programmed computer. For example, GIV-fl protein expression can be detected and scored using an imaging analysis system or a computer. In some examples, the system or computer provides an output of the GIV-fl protein score or value used to calculate the score, such as a score of 0, 1, 2 or 3 for extent of positive GIV-fl staining; a score of 0, 1, 2, or 3 GIV-fl staining intensity; a GIV-fl staining score of 0, 1, 2, 3, 4, 5 or 6, or whether the sample is GIV-fl positive or negative. In addition, LVI status can be detected using an imaging analysis system or a computer. In some examples, the system or computer provides an output of the LVI status, such as LVI positive or negative. In some examples the system or computer provides an output of whether the CRC, is likely to progress (*e.g.,* recur) and/or respond to chemotherapy or biotherapy. In some examples the output is visual or audio, such as a print-out. In some examples, the output is stored, for example on a computer readable medium.

Thus, provided herein is a computer-implemented method for determining whether a stage II pMMR CRC is high or low risk, and thus whether the CRC is likely to respond to chemotherapy or biotherapy. Such method can include generating a GIV-fl expression score based at least on measured GIV-fl protein expression within a displayed image depicting a CRC sample detectably labeled with GIV-fl antibodies, wherein the CRC sample is obtained from a subject. The GIV-fl protein expression score can be generated by detecting expression of GIV-fl protein in the CRC cells. Methods of generating a GIV-fl protein expression score are provided herein. The resulting GIV-fl protein expression score for the sample can be outputted by the computer, for example to a user or to an algorithm. The computer-implemented method can further include inputting the LVI status (e.g., positive or negative) of the subject. Alternatively, the LVI status can be determined based at least on a displayed histology image depicting a CRC sample (such as one stained with H&E or with antibody D2-40, a marker of the lymphatic endothelium, or with a CD34 antibody). The computer-implemented method can optionally inputting one or more characteristics of the subject, wherein the one or more characteristics include age of the subject at diagnosis, number of lymph nodes that are positive for CRC, sex of the subject, state of tumor differentiation, T stage of the cancer; and on which side the colon cancer was present, and outputting a prognosis or diagnosis for the subject. For example, the output can be an indication as to whether the CRC is high-risk or low-risk, and thus the patient should or should not receive chemotherapy or biotherapy, respectively.

Also described are one or more non-transitory computer-readable media that include computer-executable instructions causing a computing system to perform such a method.

## A. Detection of GIV-FL

The stage II pMMR CRC sample obtained from a subject is analyzed to determine if it contains GIV-fl, such as detectable levels of GIV-fl protein in one or more CRC cells. Thus, the sample can be analyzed to detect or measure the presence of GIV-fl protein in the sample, for example a qualitative or semi-quantitative measurement. In particular embodiments, the disclosed methods utilize qualitative measurement of the presence of GIV-fl protein in tumor cells in the sample. Based on the GIV-fl score, the sample is determined to be GIV-fl positive or negative.

In some examples, GIV-fl is 1870 or 1871 amino acids in length, such as SEQ ID NO: 1 or 2, respectively. In contrast, GIV$\Delta$CT lacks the C-terminal 762 or 763 amino acids (e.g., the C-terminal 762 or 763 amino acid of SEQ ID NO: 1 or SEQ ID NO: 2, respectively), which includes the G-protein activating EF domain. The truncation occurs at the end of exon 19, and results in a protein that terminates at Q1108 and contains 3 additional amino acids at the C-terminus (VVI), which result from translation of the intron-19 sequence. Therefore, the C-terminal sequence of the truncated GIV protein is QTQNAKLQVVI. GIV-fl specific binding agents detect GIV-fl, but not GIV$\Delta$CT.

IHC can be used to detect or measure GIV-fl protein present in a sample from the subject. IHC can determine the presence or distribution of an antigen (such as a protein) in a sample (such as a tumor sample, for example, a portion or section of tissue including GIV-fl-expressing CRC cells or tissue) by detecting interaction of the antigen with a specific binding agent, namely antibody. A sample including an antigen (such as GIV-fl) is incubated with a GIV-fl-specific antibody (such as the SP173 Ab described in Example 1) under conditions permitting antibody-antigen binding. Antibody-antigen binding can be detected by means of a detectable label conjugated to the antibody (direct detection) or by means of a detectable label conjugated to a secondary antibody, which is raised against the primary antibody (e.g., indirect detection). In other examples of indirect detection, antibody-antigen binding is detected by means of a detectable label conjugated to a tertiary antibody which is capable of binding to a secondary antibody (e.g., is raised against the secondary antibody or is raised against a molecule conjugated to the secondary antibody, such as a hapten). Exemplary detectable labels that can be used for IHC include, but are not limited to, radioactive isotopes, fluorochromes (such as fluorescein, fluorescein isothiocyanate, and rhodamine), haptens, enzymes (such as horseradish peroxidase or alkaline phosphatase), and chromogens (such as 3,3'-diaminobenzidine (DAB) or Fast Red). In some examples, detection of antigen-antibody binding also includes signal amplification (such as tyramide signal amplification or related methods). The signal amplification method may include methods described in U.S. Pat. Publ. No. 2012/0171668.

The GIV-fl specific binding agent is an antibody, such as a polyclonal or monoclonal antibody, or fragment thereof. Such a GIV-fl-specific binding agent can in some examples be used to distinguish between GIV-fl and GIV$\Delta$CT. Thus, in some examples the GIV-fl antibody does not bind with high affinity to GIV$\Delta$CT (for example does not product detectable signal if only GIV$\Delta$CT is present). If desired, the GIV-fl antibody can include a detectable label to permit detection and in some cases quantification of the GIV-fl protein/antibody complex. In other examples, the GIV-fl antibody is detected with an appropriate labeled secondary antibody. In additional examples, the GIV-fl antibody is detected with an appropriate labeled tertiary antibody.

Antibodies that can be used to detect GIV-fl expression are known and provided herein (such as SP173). A person of ordinary skill in the art will appreciate that other antibodies can be used in the methods provided herein, including those now available or developed in the future. For example, methods of preparing antibodies against a specific target protein are well known in the art. A GIV-fl protein or a fragment or conservative variant thereof (such as unique region of the C-terminus of GIV-fl, such as a region within the C-terminal 700, 600, 500, 400, 300, 200, 100, 50, or 20 amino acids) can be used to produce antibodies which are immunoreactive or specifically bind to an epitope of the GIV-fl protein. Polyclonal antibodies, antibodies which consist essentially of pooled monoclonal antibodies with different epitopic specificities, as well as distinct monoclonal antibody preparations are included. The preparation of polyclonal antibodies is well known to those skilled in the art. See, for example, Green et al., "Production of Polyclonal Antisera," in: Immunochemical Protocols, pages 1-5, Manson, ed., Humana Press, 1992; Coligan et al., "Production of Polyclonal Antisera in Rabbits, Rats, Mice and Hamsters," in: Current Protocols in Immunology, section 2.4.1, 1992. The preparation of monoclonal antibodies likewise is conventional (see, for example, Kohler & Milstein, Nature 256:495, 1975; Coligan et al., sections 2.5.1-2.6.7; and Harlow et al. in: Antibodies: a Laboratory Manual, page 726, Cold Spring Harbor Pub., 1988).

In some examples, a sample has been obtained from a subject (such as a tumor sample that is known or suspected of expressing GIV-fl, such as a stage II pMMR CRC), and processed for IHC. For example, the sample can be fixed and embedded, for example with formalin and paraffin. The sample can then be mounted on a support, such as a glass microscope slide. For example, the sample can be sliced into a series of thin sections (for example, using a microtome), and the sections mounted onto a microscope slide. In some examples, a single slide includes multiple tissue sections from the same cancer sample or sections from the same cancer sample can be placed on different slides. Different sections of the cancer (*e.g.,* CRC) sample can then be individually labeled with different antibodies, for example an anti-GIV-fl antibody and a negative control antibody (for example, an antibody that does not specifically bind to an endogenous antigen in the CRC). That is, one section can be labeled with GIV-fl antibody and another section can be labeled with a negative control antibody (such as an antibody that binds to a target that does not occur endogenously in the sample). In some examples, a separate slide from the same subject is stained with H&E (such as an adjacent or serial section from the same tumor sample). In some examples, additional proteins of interest can be detected in the same or additional tissue samples by labeling with further antibodies (for example other tumor markers, such as antibodies specific for EGFR, Bax, MMR proteins (*e.g.,* MLH1; PMS2; MSH2 and MSH6), pAKT, PTEN, and PI3K mutants). In some examples, an automated slide or tissue stainer (such as VENTANA BENCHMARK instruments, for example BenchMark XT or BenchMark GX instruments) can be used to stain and process the slides.

In some examples, detecting GIV-fl protein in the sample includes indirect detection of binding of the GIV-fl antibody to the sample (for example, the GIV-fl (primary) antibody is not detectably labeled). For example, the sample is contacted with a GIV-fl antibody (such as SP173) under conditions sufficient for the GIV-fl antibody to bind to GIV-fl protein in the sample. The sample is then contacted with a secondary antibody that can specifically bind to the GIV-fl antibody (such as an anti-rabbit antibody, if the GIV-fl antibody is a rabbit antibody or an anti-mouse antibody, if the GIV-fl antibody is a mouse antibody) under conditions sufficient for the secondary antibody to bind to the GIV-fl antibody. The secondary antibody can be detectably labeled. The detectable label can be conjugated to the secondary antibody. In some examples, the detectable label conjugated to the secondary antibody can be directly detected (such as a fluorescent label, or an enzyme, which can produce a detectable reaction product in the presence of suitable substrate). In other examples, the secondary antibody is conjugated to one or more haptens (such as fluorescein, dinitrophenyl, biotin, or 3-hydroxyquinoxaline-2-carboxylic acid (HQ)). The sample is then contacted with a tertiary antibody that can specifically bind the hapten-conjugated secondary antibody (for example, an anti-hapten antibody, such as an anti-HQ antibody) under conditions sufficient for the tertiary antibody to bind to the hapten. In some examples, the tertiary antibody is conjugated to a detectable label, such as an enzyme (for example, horseradish peroxidase (HRP) or alkaline phosphatase (AP)). The sample is then contacted with one or more reagents that produce a detectable reaction product in the presence of the enzyme. In some examples, the sample is contacted with an HRP substrate (such as hydrogen peroxide) and a chromogen (such as DAB) that produces a visually detectable product in the presence of HRP. In some examples, detecting GIV-fl protein in the sample is carried out using the VENTANA OptiView DAB IHC Detection Kit (Ventana Medical Systems, Inc., Tucson, AZ, Catalog No. 760-700) or the VENTANA ultraView Universal DAB Detection Kit (Ventana Medical Systems, Inc., Tucson, AZ, Catalog No. 760-500).

In particular embodiments, detecting GIV-fl protein in the sample includes indirect detection including signal amplification. In some examples, signal amplification allows unequivocal detection of GIV-fl positive specimens which may exhibit only weak staining without signal amplification. Signal amplification methods for IHC are known to one of ordinary skill in the art. In some examples, signal amplification includes CAtalyzed Reporter Deposition (CARD), also known as Tyramide Signal Amplification (TSA™). In one variation of this method an enzyme-conjugated secondary antibody (such as an HRP-conjugated secondary antibody) binds to the primary antibody. Next a substrate of biotinylated tyramide (tyramine is 4-(2- aminoethyl)phenol) is used, which presumably becomes a free radical when interacting with the HRP enzyme. The phenolic radical then reacts quickly with the surrounding material, thus depositing or fixing biotin in the vicinity. This process is repeated by providing more substrate (biotinylated tyramide) and building up more localized biotin. Finally, the "amplified" biotin deposit is detected with streptavidin attached to a fluorescent molecule. Alternatively, the amplified biotin deposit can be detected with avidin-peroxidase complex, which is then contacted with DAB to produce a brown color.

In other examples, signal amplification includes contacting the sample with hydrogen peroxide and a tyramide-HQ conjugate after contacting the sample with an HRP-conjugated tertiary antibody under conditions sufficient for depositing HQ at or near the site of the primary antibody bound to the sample. The sample is then contacted with an enzyme-conjugated antibody (such as an HRP- or AP-conjugated antibody) that specifically binds to HQ. In some examples, this enzyme-conjugated antibody is the same as the HRP-conjugated tertiary antibody. In other examples, the enzyme-conjugated antibody is a different antibody than the HRP-conjugated tertiary antibody. The sample is then contacted with one or more reagents that produce a detectable reaction product in the presence of the enzyme. In some examples, the sample is contacted with an HRP substrate (such as hydrogen peroxide) and a chromogen (such as DAB) that produces a visually detectable product in the presence of HRP. In some examples, signal amplification is carried out using VENTANA OptiView Amplification Kit (Ventana Medical Systems, Inc., Catalog No. 760-099).

**B. Scoring GIV-fl Expression**

To score samples for GIV-fl expression, a CRC sample with detectably-labeled GIV-fl (for example, one or more slides containing sections of the CRC sample, such as 1, 2, 3, 4, or 5 slides) is used. The sample can be labeled with an antibody (or other protein specific binding agent, such as an aptamer) specific for GIV-fl and appropriately labeled secondary and/or tertiary antibodies, for example as described in Section A, above.

One of ordinary skill in the art can identify portions of the sample which are neoplastic (*e.g.,* tumor cells) and portions of the sample which are normal tissue or cells, for example based on morphological and/or histological characteristics. In some examples, the sample is stained with H&E (for example an adjacent tissue section) to assist in identifying tissue and cell morphology.

The anti- GIV-fl labeled sample (or a digital image thereof) can be visually inspected (for example, with or without light microscopy), for example by a pathologist or by a computer. In some examples, an entire sample (such as an entire tissue section) is visually inspected, for example using light microscopy, for example at about 2x-20x magnification. In some examples, the Ventana Image Analysis System (VIAS) is used to digitally quantify staining intensity and total area of staining. High resolution images can be captured and the optical density (OD) of the chromogen associated with GIV-fl obtained relative to a clear area on the same slide. Three or more different areas per specimen can be analyzed. The average OD can be recorded as the digital measure of expression of GIV-fl for that specimen.

The disclosed methods can be used to determine if the CRC sample is GIV-fl positive or negative, for example by first determining a GIV-fl score for the sample. In one example, the GIV-fl score for the CRC sample is determined as follows. The extent of positive GIV-fl staining for the sample is scored on a scale of 0 to 3, wherein the score is assigned 0 if 0% to 10% of the total area of the sample stains positive for GIV-fl (that is, the % positive bin can be assigned a 0), the score is assigned 1 if 11%-35% of the total area of the sample stains positive for GIV-fl (that is, the % positive bin can be assigned a 1), the score is assigned 2 if 36%-50% of the total area of the sample stains positive for GIV-fl (that is, the % positive bin can be assigned a 2), and the score is assigned 3 if 51%-100% of the total area of the sample stains positive for GIV-fl (that is, the % positive bin can be assigned a 3). In addition, a GIV-fl intensity of staining value or score for the sample on a scale of 0 to 3+ (strong) is determined or calculated. General methods of determining staining intensity (for example, semi-quantitative IHC methods) are known to one of ordinary skill in the art. For example, a score of 0 is used if there is no staining above background, 1 or 1+ is used for weak intensity staining, 2 or 2+ is used for moderate intensity staining, and 3 or 3+ is used for strong intensity staining (for example as discussed above). Thus, a GIV-fl intensity of staining bin can be assigned a 0, 1, 2 or 3. In some examples, the value determined for each of the extent of positive GIV-fl staining and the GIV-fl intensity of staining are inputted into a computer or algorithm. The extent of positive GIV-fl staining and the GIV-fl intensity of staining values or scores are summed, thereby generating a GIV-fl score value from 0 to 6. Based on this value from 0 to 6, if the GIV-fl score value is 0-2 the sample is determined to be GIV-fl negative and if the GIV-fl score value is 3-6 the sample is determined to be GIV-fl positive.

In another example the GIV-fl score for the CRC sample is determined as follows. The sample is analyzed to determine if the total area of GIV-fl staining for the sample is greater than 10% and/or to determine if GIV-fl staining intensity is strong (3+) any percent positive cells using the methods described above). Based on this information, the sample is assigned GIV-fl positive if the total area of GIV-fl staining for the sample with any staining intensity is greater than 10% or if the GIV-fl staining intensity is strong (3+) any percent; or the sample is assigned GIV-fl negative if the total area of GIV-fl staining with a staining intensity of 0, 1+, or 2+ or the sample is less than 10%.

In some examples, the scoring method also includes comparing the GIV-fl labeled tumor sample with one or more controls labeled with the GIV-fl specific binding agent (e.g., antibody) (for example, controls assayed in the same IHC run as the test sample). In some examples, the control includes a positive control, such as a sample including cells known to be GIV-fl -positive (for example COS-7 cells). In other examples, the control includes a negative control, such as a sample including cells known to be GIV-fl -negative (for example hepatocytes). In some examples, the positive and/or negative control samples are system-level controls to ensure proper functioning of assay reagents and instruments.

In one example, the controls include both a positive and a negative control.

In other examples, the negative control includes a stage II pMMR CRC sample (such as stage IIa or stage IIb) stained with a negative control antibody. In some examples, the negative control antibody is an antibody that binds specifically to a target antigen that is not endogenously present in the CRC sample. In some examples, the negative control antibody is an immunoglobulin, such as a monoclonal antibody. Staining with the negative control antibody can be used to evaluate the level of background staining in a sample from the subject. In some examples, the sample stained with the negative control antibody is a sample from the same subject (such as an adjacent or serial section from the sample) as the sample stained with the GIV-fl specific binding agent (*e.g.,* antibody). In other examples, the sample stained with the negative control antibody is from a different subject than the sample stained with the GIV-fl specific binding agent (*e.g.,* antibody).

In yet another example, expression of the GIV-fl is measured by quantifying the mRNA. A number of techniques are known for purifying mRNA from tissues, including fresh or formalin-fixed paraffin embedded tissues (FFPET), see *e.g.,* U.S. Patent No. 6,248,535 *"Method for isolation of RNA from formalin-fixed paraffin-embedded tissue specimens."* Quantitative detection of mRNA by RT-PCR of a target gene relative to a housekeeping gene is likewise known in the art, see *e.g.,* U.S. Patent No. 8,586,311. A typical reaction involves a forward and a reverse primer. At least one of the primers, preferably the primer used for the reverse transcription step, spans the junction of two exons of the target gene to avoid amplification of the residual DNA. To detect the full-length transcript (GIV-f1) at least one primer is specific for the last coding exon of the gene. In some embodiments, relative amount of the GIV-fl mRNA is determined by comparison to the detected quantity of mRNA of a gene with a constitutive level of transcription, sometimes referred to as a "housekeeping gene." The comparison could be performed by determining a difference or a ratio of the two measurements. Examples of housekeeping genes include, without limitation, beta-actin, GADPH, transferrin receptor gene or TMEM55B gene. In yet other examples, the relative amount of the GIV-fl mRNA in a tumor sample is compared to the relative amount of the GIV-fl mRNA in a control, *e.g.,* non-tumor sample. The tumor sample is assigned GIV-fl positive if the relative amount of the GIV-fl mRNA is substantially higher than the relative amount of the GIV-fl mRNA in a control sample.

## C. **LVI**

The disclosed methods factor in the lymphovascular invasion (LVI) status of the subject (*e.g.,* positive or negative). Methods of determining LVI are routine, such as using microscopy. For example, the CRC sample can be analyzed to determine if there was invasion of the CRC into blood vessels or lymphatic vessels, for example using microscopy of an image stained with H&E or with an antibody specific for the lymphatic endothelium such as D2-40 or with an antibody specific for CD34. The LVI status can be used in combination with the GIV-fl status to determine whether a stage IIa or IIb pMMR CRC is likely or not likely to respond to chemotherapy or biotherapy.

## D. **Other clinical variables**

The disclosed methods factor in the one or more characteristics of the subject (or inputting known characteristics for the subject into a computer or algorithm). In some examples, the one or more characteristics of the subject are outputted by a computer or algorithm, for example in a visual or audible output. In some examples, the one or more characteristics of the subject are inputted into an algorithm. Exemplary characteristics include but are not limited to the age of the subject age at diagnosis, number of lymph nodes that are positive for CRC, sex of the subject (male or female), state of tumor differentiation (*e.g.,* moderate, poor or well), T stage of the CRC (*e.g.,* T1, T2, T3, T4, or T3/4, which reflects the size and/or extent of the primary tumor), and on which side the colon cancer was present (*e.g.,* right or left). The GIV-fl status, in combination with one or more characteristics of the subject, and in some examples also in combination with LVI status, can be used to determine whether a stage II pMMR CRC is likely or not likely to respond to chemotherapy or biotherapy.

## E. **Samples**

In some examples, the disclosed methods include the step of preparing a sample for analysis (for example fixing the sample, contacting it with GIV-fl antibodies or H&E staining), after the sample has been obtaiend. Methods of obtaining a biological sample, such as a surgical resection specimen, from a subject are known in the art. For example, methods of obtaining tissue, such as colorectal tissue, lymph node tissue, colorectal cells, or lymph node cells, are routine. For example, a sample from a CRC that contains cellular material, can be obtained by surgical excision of all or part of the tumor, by collecting a fine needle aspirate from the tumor, as well as other methods known in the art. In some examples, the sample has been obtained from a subject having or suspected to have stage II CRC (such as stage IIa or IIb). In particular examples, the sample obtained from the subject includes tumor cells, such as at least a portion of a tumor. In some examples, the sample from the subject also includes normal (*e.g.,* non-tumor) tissue or cells. In some examples, the tumor sample is placed in 10% neutral buffered formalin upon removal from the subject.

The sample can be fresh, frozen, or fixed. In some examples, samples are processed post-collection by fixation and in some examples are wax- (*e.g.,* paraffin-) embedded. Fixatives for mounted cell and tissue preparations are well known in the art and include, without limitation, formalin fixative, 95% alcoholic Bouin's fixative; 95% alcohol fixative; B5 fixative, Bouin's fixative, Karnovsky's fixative (glutaraldehyde), Hartman's fixative, Hollande's fixative, Orth's solution (dichromate fixative), and Zenker's fixative (see, *e.g.,* Carson, Histotechology: A Self-Instructional Text, Chicago:ASCP Press, 1997). GIV-fl staining intensity may vary depending on the particular fixatives used (such as 95% alcohol, AFA, B5, or Prefer). In particular examples, the sample is fixed in neutral buffered formalin (such as 10% neutral buffered formalin) or zinc formalin. In some examples, the sample is fixed for at least about 6 hours (for example, about 6-48 hours, 12-24 hours or about 6, 12, 16, 18, 24, 36, or 48 hours). In additional examples, the sample is placed in fixative within about 6 hours of collection (for example, within about 15 minutes, 30 minutes, 1, 2, 3, 4, 5, or 6 hours).

In some examples, the sample can be a fixed, wax-embedded tissue sample, such as a fixed, wax-embedded tissue sample including tumor cells. In some examples, the sample is a tissue section including tumor cells labeled with a primary antibody specific for GIV-fl, which may be labeled directly or indirectly (*e.g.,* with a labeled secondary antibody), which in some examples is further stained with H&E (*e.g.,* using an adjacent or serial section from the same sample).

In some examples, the sample (or a fraction thereof) is present on a solid support. Solid supports bear the biological sample and permit the convenient detection of components (*e.g.,* proteins) in the sample. Exemplary supports or substrates include microscope slides *(e.g.,* glass microscope slides or plastic microscope slides), coverslips (*e.g.,* glass coverslips or plastic coverslips), tissue culture dishes, multi-well plates, membranes (*e.g.,* nitrocellulose or polyvinylidene fluoride (PVDF)) or BIACORE™ chips.

### F. Methods of Treatment (not subject of the claims)

The disclosed methods can further include identifying and/or selecting subjects for treatment with chemotherapy or biotherapy. For example, if the disclosed methods indicate that the CRC is high-risk, the subject can be selected for treatment with chemotherapy or biotherapy, while if the disclosed methods indicate that the CRC is low-risk, then the subject can be selected to abstain from chemotherapy or biotherapy. Additionally, the disclosed methods do not include, but can be followed by administering one or more chemotherapies or biotherapies to the subject if the sample obtained from the subject is concluded to be high risk (*e.g.,* one likely to progress or recur, for example if the sample is GIV-fl positive and LVI positive). In contrast, the disclosed embodiments can further include identifying subjects who will not likely benefit from chemotherapy or biotherapy, for example if the tumor sample obtained from the subject is concluded to be low risk (*e.g.,* one likely to progress or recur, for example if the sample is GIV-fl negative and LVI negative).

### 1. Exemplary CRC Chemotherapies and Biologic Therapies

CRC chemotherapies and biotherapies include therapeutic agents that when administered in therapeutically effective amounts induce the desired response (*e.g.,* treatment of a CRC, for example by reducing the size or volume of the tumor, or reducing the size, volume or number of metastases). Examples of CRC chemotherapies and bio-therapies that can be used include but are not limited to one or more of the following: 5-fluorouracil (*e.g.*, Adrucil®, Efudex®, Fluoroplex®), Avastin® (bevacizumab), Camptosar® (Irinotecan Hydrochloride), capecitabine (*e.g.,* Xeloda®), oxaliplatin (*e.g.,* Eloxatin®), Erbitux® (cetuximab), leucovorin calcium, regorafenib, Stivarga® (Regorafenib), Vectibix® (Panitumumab), Wellcovorin® (Leucovorin Calcium), and Zaltrap® (Ziv-Aflibercept). Examples of drug combinations uses for CRC include but are not limited to: CAPOX (capecitabine and oxaliplatin), FOLFIRI (5-FU, leucovorin, and irinotecan), FOLFIRI-bevacizumab, FOLFIRI-cetuximab, FOLFOX (5-FU, leucovorin, and oxaliplatin) and XELOX.

In one example, a CRC chemotherapy or bio-therapy increases killing of CRC cells (or reduces their viability). Such killing need not result in 100% reduction of CRC cells; for example a CRC chemotherapy that results in reduction in the number of viable CRC cells by at least 10%, at least 20%, at least 30%, at least 40%, at least 50%, at least 75%, at least 90%, or at least 95% (for example as compared to no treatment with the CRC chemotherapy or bio-therapy) can be used in the methods provided herein. For example, the CRC chemotherapy or biotherapy can reduce the growth of CRC cells by at least 10%, at least 20%, at least 30%, at least 40%, at least 50%, at least 75%, at least 90%, or at least 95% (for example as compared to no chemotherapy or bio-therapy).

In one example, a CRC chemotherapy or bio-therapy decreases GIV-fl expression or activity. Such inhibition need not result in 100% reduction of GIV-fl expression or activity; for example CRC chemotherapy that result in reduction in GIV-fl expression or activity by at least 10%, at least 20%, at least 30%, at least 40%, at least 50%, at least 75%, at least 90%, or at least 95% (for example as compared to no treatment with CRC chemotherapy or bio-therapy) can be used in the methods provided herein. For example, the CRC chemotherapy or bio-therapy may interfere with gene expression (transcription, processing, translation, post-translational modification).

In one example, a CRC bio-therapy includes or consists of an antibody, such as a humanized antibody. Such antibodies can be polyclonal, monoclonal, or chimeric antibodies. As noted above, methods of making antibodies specific for a particular target is routine. In some example, the therapeutic antibody is conjugated to a toxin.

Other examples of CRC bio-therapy include inhibitory nucleic acid molecules, such as an antisense oligonucleotide, a siRNA, a microRNA (miRNA), a shRNA or a ribozyme. Such molecules can be used to decrease or eliminate GIV-fl gene expression. Any type of antisense compound that specifically targets and regulates expression of GIV-fl nucleic acid is contemplated for use. An antisense compound is one which specifically hybridizes with and modulates expression of a target nucleic acid molecule (such as GIV-fl). These compounds can be introduced as single-stranded, double-stranded, circular, branched or hairpin compounds and can contain structural elements such as internal or terminal bulges or loops. Double-stranded antisense compounds can be two strands hybridized to form double-stranded compounds or a single strand with sufficient self complementarity to allow for hybridization and formation of a fully or partially double-stranded compound. In some examples, an antisense GIV-fl oligonucleotide is a single stranded antisense compound, such that when the antisense oligonucleotide hybridizes to a GIV-fl mRNA, the duplex is recognized by RNaseH, resulting in cleavage of the mRNA. In other examples, a miRNA is a single-stranded RNA molecule of about 21-23 nucleotides that is at least partially complementary to an mRNA molecule that regulates gene expression through an RNAi pathway. In further examples, a shRNA is an RNA oligonucleotide that forms a tight hairpin, which is cleaved into siRNA. siRNA molecules are generally about 20-25 nucleotides in length and may have a two nucleotide overhang on the 3' ends, or may be blunt ended. Generally, one strand of a siRNA is at least partially complementary to a target nucleic acid. Antisense compounds specifically targeting a GIV-fl gene can be prepared by designing compounds that are complementary to a GIV-fl nucleotide sequence, such as an mRNA sequence. GIV-fl antisense compounds need not be 100% complementary to the GIV-fl nucleic acid molecule to specifically hybridize and regulate expression of GIV-fl. For example, the antisense compound, or antisense strand of the compound if a double-stranded compound, can be at least 75%, at least 80%, at least 85%, at least 90%, at least 95%, at least 99% or 100% complementary to a GIV-fl nucleic acid sequence. Methods of screening antisense compounds for specificity are well known (see, for example, U.S. Publication No. 2003-0228689). In addition, methods of designing, preparing and using inhibitory nucleic acid molecules are within the abilities of one of skill in the art. Furthermore, sequences for GIV-fl are publicly available.

## 2 Administration of Chemo- or Bio-therapy and Other Agents

In some examples, the disclosed methods are followed by providing a therapeutically effective amount of one or more CRC chemotherapies or bio-therapies to a subject having a high-risk stage II pMMR CRC. Methods and therapeutic dosages of such agents and treatments are known to those of ordinary skill in the art, and for example, can be determined by a skilled clinician. In some examples, the disclosed methods are followed by providing surgery and/or radiation therapy to the subject in combination with the chemotherapy or bio-therapy (for example, sequentially, substantially simultaneously, or simultaneously). Administration can be accomplished by single or multiple doses. Methods and therapeutic dosages of such agents and treatments are known to those skilled in the art, and can be determined by a skilled clinician. The dose required will vary from subject to subject depending on the species, age, weight and general condition of the subject, the particular therapeutic agent being used and its mode of administration.

Therapeutic agents, including CRC chemotherapies or bio-therapy, can be administered to a subject in need of treatment using any suitable means known in the art. Methods of administration include, but are not limited to, intradermal, transdermal, intramuscular, intraperitoneal, parenteral, intravenous, subcutaneous, vaginal, rectal, intranasal, inhalation, oral, or by gene gun. Intranasal administration refers to delivery of the compositions into the nose and nasal passages through one or both of the nares and can include delivery by a spraying mechanism or droplet mechanism, or through aerosolization of the therapeutic agent.

Administration of the therapeutic agents, including CRC chemotherapies or bio-therapy, by inhalant can be through the nose or mouth via delivery by spraying or droplet mechanisms. Delivery can be directly to any area of the respiratory system via intubation. Parenteral administration is generally achieved by injection. Injectables can be prepared in conventional forms, either as liquid solutions or suspensions, solid forms suitable for solution of suspension in liquid prior to injection, or as emulsions. Injection solutions and suspensions can be prepared from sterile powders, granules, and tablets. Administration can be systemic or local.

Therapeutic agents, including CRC chemotherapies or bio-therapies, can be administered in any suitable manner, for example with pharmaceutically acceptable carriers. Pharmaceutically acceptable carriers are determined in part by the particular composition being administered, as well as by the particular method used to administer the composition. Accordingly, there is a wide variety of suitable formulations of pharmaceutical compositions of the present disclosure. The pharmaceutically acceptable carriers (vehicles) useful in this disclosure are conventional. Remington's Pharmaceutical Sciences, by E. W. Martin, Mack Publishing Co., Easton, PA, 15th Edition (1975), describes compositions and formulations suitable for pharmaceutical delivery of one or more therapeutic agents

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Formulations for topical administration can include ointments, lotions, creams, gels, drops, suppositories, sprays, liquids and powders. Conventional pharmaceutical carriers, aqueous, powder or oily bases, thickeners and the like may be necessary or desirable.

Therapeutic agents, including CRC chemotherapies or bio-therapy, for oral administration include powders or granules, suspensions or solutions in water or non-aqueous media, capsules, sachets, or tablets. Thickeners, flavorings, diluents, emulsifiers, dispersing aids or binders may be desirable.

Therapeutic agents, including CRC chemotherapies or bio-therapy, can be administered as a pharmaceutically acceptable acid- or base-addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

CRC chemotherapies and bio-therapies can include anti-neoplastic chemotherapeutic agents, antibiotics, alkylating agents and antioxidants, kinase inhibitors, and other agents such as antibodies. Particular examples of additional chemotherapic agents that can be used include alkylating agents, such as nitrogen mustards (for example, chlorambucil, chlormethine, cyclophosphamide, ifosfamide, and melphalan), nitrosoureas (for example, carmustine, fotemustine, lomustine, and streptozocin), platinum compounds (for example, carboplatin, cisplatin, oxaliplatin, and BBR3464), busulfan, dacarbazine, mechlorethamine, procarbazine, temozolomide, thiotepa, and uramustine; folic acid (for example, methotrexate, pemetrexed, and raltitrexed), purine (for example, cladribine, clofarabine, fludarabine, mercaptopurine, and tioguanine), pyrimidine (for example, capecitabine), cytarabine, fluorouracil, and gemcitabine; plant alkaloids, such as podophyllum (for example, etoposide, and teniposide); microtubule binding agents (such as paclitaxel, docetaxel, vinblastine, vindesine, vinorelbine (navelbine) vincristine, the epothilones, colchicine, dolastatin 15, nocodazole, podophyllotoxin, rhizoxin, and derivatives and analogs thereof), DNA intercalators or cross-linkers (such as cisplatin, carboplatin, oxaliplatin, mitomycins, such as mitomycin C, bleomycin, chlorambucil, cyclophosphamide, and derivatives and analogs thereof), DNA synthesis inhibitors (such as methotrexate, 5-fluoro-5'-deoxyuridine, 5-fluorouracil and analogs thereof); anthracycline family members (for example, daunorubicin, doxorubicin, epirubicin, idarubicin, mitoxantrone, and valrubicin); antimetabolites, such as cytotoxic/antitumor antibiotics, bleomycin, rifampicin, hydroxyurea, and mitomycin; topoisomerase inhibitors, such as topotecan and irinotecan; monoclonal antibodies, such as alemtuzumab, bevacizumab, cetuximab, gemtuzumab, rituximab, panitumumab, pertuzumab, and trastuzumab; photosensitizers, such as aminolevulinic acid, methyl aminolevulinate, porfimer sodium, and verteporfin, enzymes, enzyme inhibitors (such as camptothecin, etoposide, formestane, trichostatin and derivatives and analogs thereof), kinase inhibitors (such as imatinib, gefitinib, and erolitinib), gene regulators (such as raloxifene, 5-azacytidine, 5-aza-2'-deoxycytidine, tamoxifen, 4-hydroxytamoxifen, mifepristone and derivatives and analogs thereof); and other agents , such as alitretinoin, altretamine, amsacrine, anagrelide, arsenic trioxide, asparaginase, axitinib, bexarotene, bevacizumab, bortezomib, celecoxib, denileukin diftitox, estramustine, hydroxycarbamide, lapatinib, pazopanib, pentostatin, masoprocol, mitotane, pegaspargase, tamoxifen, sorafenib, sunitinib, vemurafinib, vandetanib, and tretinoin.. Methods and therapeutic dosages of such agents are known to those skilled in the art, and can be determined by a skilled clinician. Other therapeutic agents, for example anti-tumor agents, that may or may not fall under one or more of the classifications above, also are suitable for administration in combination with the described specific binding agents. Selection and therapeutic dosages of such agents are known to those skilled in the art, and can be determined by a skilled clinician.

In some examples, the dose of a GIV-fl inhibitory nucleic acid (such as an antisense molecule, siRNA, shRNA, or miRNA) is about 1 mg to about 1000 mg, about 10 mg to about 500 mg, or about 50 mg to about 100 mg. In some examples, the dose of antisense compound is about 1 mg, about 10 mg, about 50 mg, about 100 mg, about 250 mg, about 500 mg or about 1000 mg. In some embodiments, the dose of an inhibitory nucleic acid is about 1.0 mg/kg to about 100 mg/kg, or about 5.0 mg/kg to about 500 mg/kg, about 10 mg/kg to about 100 mg/kg, or about 25 to about 50 mg/kg. In some examples, the dose of a inhibitory nucleic acid is about 1.0 mg/kg, about 5 mg/kg, about 10 mg/kg, about 12.5 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg or about 100 mg/kg. It will be appreciated that these dosages are examples only, and an appropriate dose can be determined by one of ordinary skill in the art using only routine experimentation.

In some embodiments, the dose of an antibody or antibody conjugate is about 1 mg/kg to about 25 mg/kg, such as about 2 mg/kg to about 15 mg/kg, about 2 mg/kg to about 10 mg/kg, or about 2 mg/kg to about 8 mg/kg. In some examples, the dose of antibody is about 1 mg/kg, about 2 mg/kg, about 4 mg/kg, about 5 mg/kg, about 6 mg/kg, about 8 mg/kg, about 10 mg/kg, about 15 mg/kg, about 20 mg/kg, or about 25 mg/kg. In other embodiments, the dose of antibody is about 50 $mg/m^2$ to about 500 $mg/m^2$, such as about 50 $mg/m^2$ to about 400 $mg/m^2$, about 100 $mg/m^2$ to about 400 $mg/m^2$, or about 250 $mg/m^2$ to about 400 $mg/m^2$. In some examples, the dose is about 50 $mg/m^2$, about 100 $mg/m^2$, about 150 $mg/m^2$, about 200 $mg/m^2$, about 250 $mg/m^2$, about 300 $mg/m^2$, about 400 $mg/m^2$, or about 500 $mg/m^2$. It will be appreciated that these dosages are examples only, and an appropriate dose can be determined by one of ordinary skill in the art using only routine experimentation.

**G. Outputs**

Following the detection of GIV-fl expression and determination of the GIV-fl protein score and GIV-fl status, as well as the LVI status and optionally other patient characteristics, the assay results (such as the GIV-fl status and LVI status), findings, prognosis, predictions and/or treatment recommendations can be recorded and communicated to technicians, physicians and/or patients, for example. In certain embodiments, computers are used to communicate such information to interested parties, such as, patients and/or the attending physicians. Based on the GIV-fl status, LVI status, and optionally other patient characteristics, an output as to whether the CRC is high- or low-risk (such as whether the CRC is likely to progress or recur, and thus likely to respond to chemotherapy or biotherapy), the subject from whom the sample was obtained can be assigned a treatment plan, such as treatment or not with chemotherapy or biotherapy.

In one embodiment, a prognosis, prediction and/or treatment recommendation based on the output (whether the CRC is high- or low-risk) is communicated to interested parties as soon as possible after the assay is completed and the prognosis is generated. The results and/or related information may be communicated to the subject by the subject's treating physician. Alternatively, the results may be communicated directly to interested parties by any means of communication, including writing, such as by providing a written report, electronic forms of communication, such as email, or telephone. Communication may be facilitated by use of a suitably programmed computer, such as in case of email communications. In certain embodiments, the communication containing results of the test and/or conclusions drawn from and/or treatment recommendations based on the test, may be generated and delivered automatically to interested parties using a combination of computer hardware and software which will be familiar to artisans skilled in telecommunications. One example of a healthcare-oriented communications system is described in U.S. Pat. No. 6,283,761; however, the present disclosure is not limited to methods which utilize this particular communications system.

In certain embodiments of the methods of the disclosure, all or some of the method steps, including the assaying of samples, scoring of GIV-fl protein expression, prognosis of the tumor, and communicating of assay results or prognosis, may be carried out in diverse (*e.g.,* foreign) jurisdictions.

**H. Computer-Readable Media**

Any of the computer-readable media herein can be non-transitory (*e.g.,* memory, magnetic storage, optical storage, or the like).

Any of the storing actions described herein can be implemented by storing in one or more computer-readable media (*e.g.,* computer-readable storage media or other tangible media).

Any of the things described as stored can be stored in one or more computer-readable media (*e.g.,* computer-readable storage media or other tangible media).

Any of the methods described herein can be implemented by computer-executable instructions in (*e.g.,* encoded on) one or more computer-readable media (*e.g.,* computer-readable storage media or other tangible media). Such instructions can cause a computer to perform the method. The technologies described herein can be implemented in a variety of programming languages.

Any of the methods described herein can be implemented by computer-executable instructions stored in one or more computer-readable storage devices (*e.g.,* memory, magnetic storage, optical storage, or the like). Such instructions can cause a computer to perform the method.

**I. Statistical Prediction Model**

The methods utilize statistical prediction models. Based on GIV-fl status, LVI status, and optionally other clinical characteristics, the methods are predictive of whether a stage II pMMR CRC will likely respond to chemotherapy or biotherapy. In developing the model, each variable was accounted for while holding another variable constant. The statistical models utilized Cox proportional hazards modeling (Cox, Journal of the Royal Statistical Society, Series B 34 (2): 187-220, 1972).

Mathematically, Cox proportional hazards survival modeling defines a time-to-event outcome in terms of the hazard function h(t), sometimes called the failure rate, which is a rate of the probability density function (numerator) and the survival function (denominator). Potential predictors can be incorporated into this statistical model as follows:

$$h(t|\mathbf{X}) = h_0(t)exp(\beta_1 X_1 + \ldots + \beta_p X_p)$$

In these models, $h_0(t)$ is the baseline hazard (analogous to an intercept in linear models), the hazard function is linear in the p estimated coefficients, $\beta$, and the $p$ predictors are additive through the coefficients, and can be either categorical or continuous. Cox proportional hazards models do not make any parametric assumptions about the baseline hazard, $h_0(t)$, and when the $\beta$s are estimated it is assumed these are proportional over time within the predictors. The estimated coefficients are obtained using partial likelihood methods, a common statistical technique. The open-source package R (www.Rproject.org) and SAS software version 9.3 (SAS Institute, Carey, North Carolina) were used for statistical programming.

The exponentiated, fitted coefficients, $exp(\hat{\beta})$ for each predictor is interpreted as a "hazard ratio" which describes the ratio between hazard rates in the predictors; for categorical predictors, this is the probability of an event (recurrence) occuring in one group compared to another group at any time, t, and for continuous predictors it is the probability of an event occuring over some interval of predictor compared to an adjacent interval (again, at any time, t). Ratios that are less than one indicate that the reference group (denominator) has a higher probability of recurrence, and ratios larger than one indicate that the comparetor category has a higher probability of recurrence. For example, with GIV-fl positivity and LVI, GIV-fl negative and LVI negative groups were used as the reference. The third set of results in Table 11 shows the estimated hazard ratios for GIV-fl positivity (compared to GIV-fl negative) and LVI positive (compared to LVI negative) when both variables are included in a fitted model (2.56 and 2.07, respectively). Because the fitted model is linear on the exponential scale, the two-factor hazard ratio for when both of these variables are positive (*e.g.*, both take on the value 1, so $X_1 = 1$ and $X_2 = 1$) is estimated by the -product of the hazard ratios:

$$HR_{GIV=1,LVI=1} = exp(\hat{\beta}_1 X_1)exp(\hat{\beta}_2 X_2) = (2.56)(2.07) = 5.30$$

When GIV-fl is positive ($X_1 = 1$), and LVI is negative ($X_2 = 0$) the two-factor hazard ratio becomes (note that $exp(0) = 1$):

$$HR_{GIV=1,LVI=0} = exp(\hat{\beta}_1 X_1)exp(\hat{\beta}_2 X_2) = (2.56)(1) = 2.56$$

And further, when both GIV-fl and LVI are negative ($X_1 = 0$ and $X_2 = 0$) the two-factor hazard ratio becomes:

$$HR_{GIV=0,LVI=0} = exp(\hat{\beta}_1 X_1)exp(\hat{\beta}_2 X_2) = (1)(1) = 1$$

Thus, there is a higher probability of recurrence for patients positive for both GIV-fl expression and LVI than either of these variables alone, as compared to patients with neither; those patients with both variables are 5 times more likely to recur compared to patients with neither GIV-fl positivity or LVI. This ratio of risk of recurrence is higher than the 2.56 risk for GIV positive alone, and the 2.07 for LVI alone. Any of the multifactor hazard ratios, from multiple factor models (*e.g.*, model that includes "all" clinical variables), can be obtained in this manner. The hazard ratio is not symmetric, because it is, by definition, a number between 0 and $\infty$; it is not uncommon to examine these estimates in terms of the log of this hazard ratio, because then the ratio is a symmetric number going from $\infty$ to $\infty$. In the latter case, negative log hazard ratios describe when the reference group is more likely to recur, and positive log hazard ratios are when the reference group is less likely to recur, compared to the specified group.

For the classification, a chemo-naive patient population was used to estimate the coefficients, and the linear predictor ($L_p$) is that which gives us the maximum sensitivity and specificity as a cut-point for high and low risk. Specifically, the linear predictor is obtained for each chemo-naive patient for any p-factor model (where p is the number of predictors), $L_p = exp(\beta X_{1i} \ldots X_{pi})$. The cut-point for $L_p$ is obtained where both the sensitivity and specificity is maximized: max(sensitivity, specificity) = max(sensitivity specificity). Once this optimal cut-point is calculated, it can be applied to new values of X in an independent population by simply applying the cut-point to chemo-treated individuals. High risk individuals are those who have $L_p$ > cut-point, and low risk individuals are those who have $L_p$ < cut-point. To validate the statistical prediction, these high and low risk categories are then compared with actual recurrence for each individual in the chemo-treated population. The final $L_p$ can be used to determine individual patient risk, with high risk patients likely to benefit from chemotherapy or biotherapy treatment.

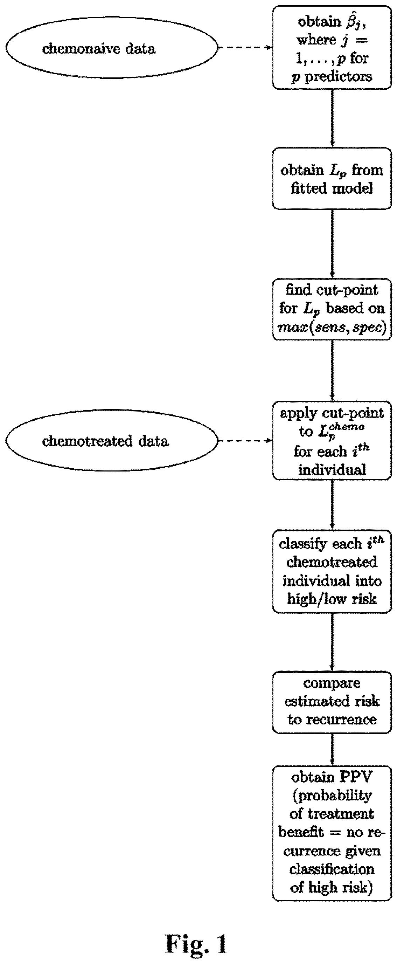

The data flow diagram in Fig. 1 describes the steps used in the statistical modeling. The $\hat{\beta}_p$ and $L_p$ values are inputs that can be provided to clinicians. Clinicians can then use these variables to determine individual risk, based on values of each individual value of the predictors, $X_p$. The process shown in the diagram is not needed for each evaluation; it is a description of the process used to obtain the inputs.

The last step is the application of the statistical modeling to a population of patients who received chemotherapy or biotherapy. This step demonstrates that for patients that were classified as high risk 80% of them actually responded to chemotherapy or biotherapy (Positive Predicted Value = the probability of not having a recurrence given classification of high risk).

The present disclosure is illustrated by the following non-limiting Examples.

## Example 1

### Generation of GIV Antibodies and Optimization of Staining Conditions

This example describes methods used to generate a rabbit monoclonal antibody specific for the full length GIV protein.

A rabbit monoclonal antibody specific for GIV was generated using the last 11 C-terminal amino acids (SKSRSREQQSS, amino acids 1861-1871 of SEQ ID NO: 2) of human GIV. This anti-GIV rabbit monoclonal antibody is clone number SP173 (VMSI Catalog Number M4734).

## Example 2

### Optimization of IHC Staining Conditions

This example describes the evaluation of four GIV antibodies (3 commercially available polyclonal antibodies and the rabbit monoclonal antibody described in Example 1) targeting full length GIV protein by immunohistochemistry (IHC). Each IHC assay was developed on liver cases (normal, fatty and cirrhotic) served as negative control.

The GIV antibody generated in Example 1 (SP173) was compared to three other antibodies raised against the C-terminal part of human GIV, for their ability to detect the full length GIV protein. The three commercially available antibodies tested were: (1) anti-GIV from Sigma Prestige (catalog number HPA038101), which is an affinity purified rabbit polyclonal antibody raised against a 78 C-terminal amino acids (1738-1815) of human girdin; (2) anti-GIV (T-13) from Santa Cruz Biotechnology, Inc. (catalog number sc-133371) which is an affinity purified rabbit polyclonal antibody raised against a peptide mapping at the C-terminus of girdin of human origin; and (3) anti-GIV from Immuno-Biological Laboratories Co. (IBL, catalog number 18979), which is an affinity purified rabbit polyclonal antibody developed against the 19 C-terminal amino acids of human girdin.

The OptiView DAB IHC detection kit (Ventana Medical Systems, Inc. [VMSI], catalog # 760-700) was used for chromogenic detection of the full length GIV protein (GIV-fl). Counterstaining was accomplished by incubating slides with hematoxylin II and Bluing reagent to enhance the DAB signal. The following parameters were examined: antibody titration; primary antibody incubation time and temperature; antigen retrieval conditions and antibody diluent.

A titration series was performed to optimize the primary antibody concentration. Each primary antibody was diluted at various concentrations (0.6 to 60 μg/ml for the antibody from Sigma Prestige; 0.1 to 5 μg/ml for the antibody from Santa Cruz; 0.4 to 16 μg/ml for the antibody from IBL; and 0.1 to 100 μg/ml for the GIV antibody generated in Example 1, SP173) using VMSI Antibody Diluent (catalog number 95119) and applied in a volume of 85 μl on liver and colorectal cancer tissue. GIV antibody from Sigma Prestige showed negative staining at all tested concentrations. As a result, this antibody was excluded from the further analysis.

After selecting an initial titer for antibodies from Santa Cruz (0.67 μg/ml), IBL (8 μg/ml), and SP173 (10 μg/ml), the antigen retrieval conditions were optimized and the primary antibody diluents were screened. The following diluents were tested: VMSI Antibody Diluent catalog numbers 95119; 95028; 90039; 90040; and 90103. Normal and fatty liver cases were used to serve as specificity assessment for the GIV IHC optimization.

The optimal diluent for the antibody from Santa Cruz was 95119, 90039 for IBL (8 μg/ml), and 90040 for the GIV antibody SP173. The optimal pretreatment conditions were 64 minutes incubation at 95°C in CC1 cell conditioning reagent (a milder Tris-based cell condition buffer) for the Santa Cruz and IBL antibodies, and 48 minutes incubation at 95°C in CC1 cell conditioning reagent for the GIV antibody SP173. The optimal amount of time for incubation in primary antibody was 16 minutes at 37°C for the Santa Cruz and IBL antibodies, and 12 minutes at 37°C for the GIV antibody SP173.

**Example 3**

**Staining of Normal Liver Samples with GIV Antibodies**

The identified optimal assay conditions described in Example 2 for each GIV antibody on control liver specimens, namely 0.67 µg/ml Santa Cruz GIV antibody in 95119, 8 µg/ml GIV (IBL) antibody in diluent 90039 and 10 µg/ml GIV Ab SP173 in a diluent 90040, were used to stain liver samples using IHC.

Based on previously determined GIV mRNA level in liver, hepatocytes had to remain negative but all inflammatory cells (Kuppfer macrophages, stellate cells) had to show positive staining in the sinusoidal spaces. GIV immunostaining was evaulated by a pathologist. Two GIV antibody clones (IBL and the SP173 antibody from Example 1) satisfied these criteria. Both antibodies detected sinusoids and stellate cells on normal and fatty liver samples with very similar signal intensity. None of the antibodies resulted staining on hepatocytes (negative control) and on any cirrhotic liver samples. The IBL antibody stained some blood vessels otherwise the staining was negative. Thus, the IHC with the IBL antibody and the SP173 antibody from Example 1 showed positive sinusoids with primarily cytoplasmic staining in non-hepatocytes and was completely negative in hepatocytes. Some normal cells (bile ducts) had nuclear staining. In contrast, the GIV antibody from Santa Cruz resulted in negative staining in non-hepatocytes and focal blush in hepatocytes therefore this antibody was excluded from further analysis.

**Example 4**

**Staining of Liver and Colorectal Cancer Samples with GIV Antibodies**

A set of 36 liver cases from normal, fatty liver and cirrhosis patients (liver cohort) were stained with the optimized staining procedures (Example 2) to serve as specificity assessment for the optimized GIV IHC. Samples were stained with the optimized GIV SP173 antibody from Example 1 IHC assay (Table 1) and with the GIV-IBL antibody IHC assay (Table 2).

Table 1: SP173 Antibody IHC assay

| | |
|---|---|
| 1 | Paraffin [Selected] |
| 2 | Deparaffinization [Selected] |
| 3 | Cell Conditioning [Selected] |
| 4 | CC1 [Selected] |
| 5 | CC1 8 Min [Selected] |
| 6 | CC1 16 Min [Selected] |
| 7 | CC1 24 Min [Selected] |
| 8 | CC1 32 Min [Selected] |
| 9 | CC1 40 Min [Selected] |
| 10 | CC1 46 Min [Selected] |
| 11 | Pre Primary Perosidase Inhibit [Selected] |
| 12 | Primary Antibody [Selected] |
| 13 | Primary Antibody Temperature [Selected] |
| 14 | Warmup Slide to Ab Incubation Temperatures [Primary Antibody] |
| 15 | Apply Coverslip, One Drop of [ANTIBODY 10] (Antibody), and Incubate for [0 Hr 12 Min] |
| 16 | Counterstain [Selected] |
| 17 | Apply One Drop of [HEMATOXYLIN II] (Counterstain), Apply Coverslip, and Incubate for [4 Minutes] |
| 18 | Post Counterstain [Selected] |
| 19 | Apply One Drop of [BLUING REAGENT] ( Post Counterstain), Apply Coverslip, and Incubate for [4 Minutes] |

Table 2: IBL antibody IHC assay

| | |
|---|---|
| 1 | Paraffin [Selected] |
| 2 | Deparaffinization [Selected] |
| 3 | Cell Conditioning [Selected] |

(continued)

| 4 | CC1 [Selected] |
| 5 | CC1 8 Min [Selected] |
| 6 | CC1 16 Min [Selected] |
| 7 | CC1 24 Min [Selected] |
| 8 | CC1 32 Min [Selected] |
| 9 | CC1 40 Min [Selected] |
| 10 | CC1 48 Min [Selected] |
| 11 | CC1 56 Min [Selected] |
| 12 | CC1 64 Min [Selected] |
| 13 | Pre Primary Perosidase inhibit [Selected] |
| 14 | Primary Antibody [Selected] |
| 15 | Primary Antibody Temperature [Selected] |
| 16 | Warmup Slide to Ab Incubation Temperatures [Primary Antibody] |
| 17 | Apply Coversip, One Drop of [ANTIBODY 8] (Antibody), and Incubate for [0 Hr 16 Min] |
| 18 | Counterstain [Selected] |
| 19 | Apply One Drop of [HEMATOXYLIN] (Counterstain). Apply Coverslip, and Incubate for [4 Minutes] |
| 20 | Post Counterstain [Selected] |
| 21 | Apply One Drop of [BLUING REAGENT] (Post Counterstain). Apply Coverslip, and Incubats for [4 Minutes] |

Both GIV-IBL and GIV Ab SP173 antibodies detected GIV protein in all liver samples with moderate to severe fibrosis while GIV remained undetectable in normal livers. Among Hepatitis C patients, the staining with the two antibodies did not show much correlation with inflammation scores, or with hepatic steatosis, but did show the previously observed correlation with their fibrosis severity score.

To test the prognostication ability of the GIV IHC, a 10 patient CRC cohort (Table 3) was stained with the same optimized staining procedures and scored by a pathologist.

**Table 3.** GIV IHC result on CRC cohort

| Case ID | GIV-IBL | GIV-Spring | Overall survival | Patient outcome |
|---------|---------|-----------|------------------|-----------------|
| 9838872 | negative | negative | 0.8 | unknown |
| 9610517 | negative | negative | 14.35 | alive |
| 973446 | negative | negative | 13.47 | alive |
| 012982A5 | negative | negative | 8.93 | septic shock, respiratory failure |
| 012768A7 | negative | negative | 8.98 | massive GI bleeding |
| 96118110 | negative | positive | 14.33 | lost follow-up |
| 071207A8 | negative | positive | N/A | sepsis, cholangitis |
| 012688A5 | negative | positive | 2.93 | metastasis to liver/lung |
| 9835799 | negative | positive | 11.57 | alive |
| 981154 | negative | positive | 2.78 | liver, lung, adrenal metastasis |

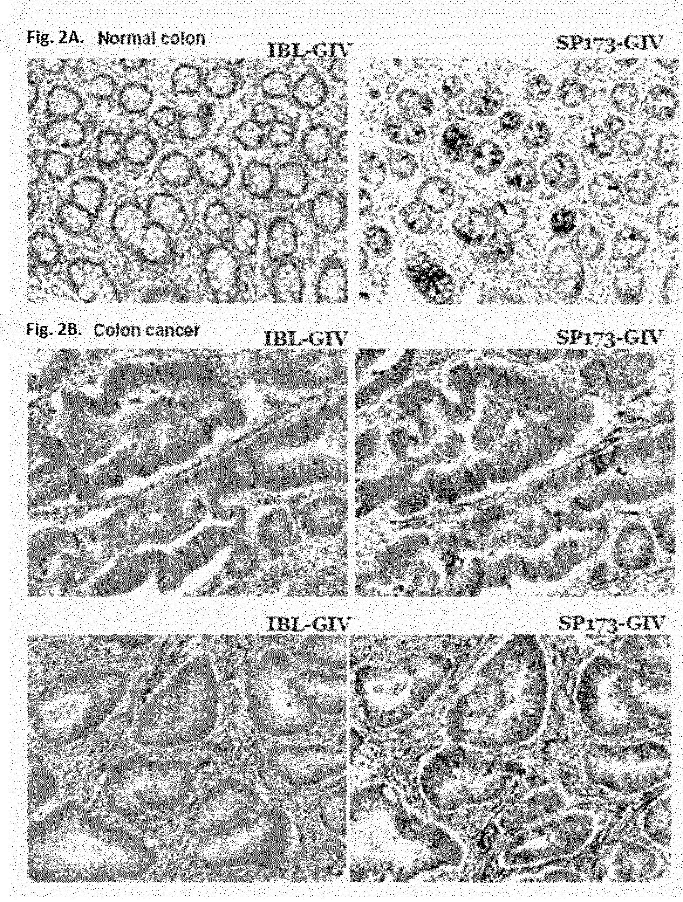

Although the IBL antibody and SP173 antibody had the same specificity on liver cases, the sensitivity and specificity of these antibodies were different on colorectal tissue. The GIV antibody SP173 was more sensitive and resulted in stronger staining than IBL antibody, but also had some non-specific staining on normal colon (possibly chromogenic deposits) (FIG. 2A). However, this non-specific staining did not have an effect on the tumor staining; staining of the tumor area remained very specific (FIG. 2B). GIV IHC with the GIV antibody SP173 identified 5 GIV positive patients and 2 of them had recurrence. GIV IHC with IBL antibody resulted in strong positive staining in stromal cells but weak, mostly negative staining in the cytoplasm of the tumor cells (FIG. 2B). These results demonstrate that GIV antibody SP173 1 is more sensitive as it was able to detect all colorectal cancer cases with recurrence. Therefore this antibody was chosen for further analysis.

**Example 5**

**Staining of Cancer Samples and Mouse GIV-KO Tissues with GIV Antibodies**

This example describes methods used to further optimize GIV IHC staining.

GIV IHC assay using the rabbit monoclonal anti-GIV antibody SP173 was used (see Example 2). The nominal staining conditions for the BenchMark XT automated stainer (VMSI) are listed in Table 4. The primary antibody concentration used was 10 μg/ml in diluent 90040. Counterstaining was accomplished by incubating slides with hematoxylin II and Bluing reagent to enhance the DAB signal. Using the optimized primary antibody formulation along with the established conditions in liver tissue, the non-hepatocytes (sinusoids, stellate cells) were stained positive while the hepatocytes remained negative.

**Table 4.** Assay conditions for GIV IHC

| Procedure | XT OptiView DAB IHC |
|---|---|
| **Antigen** | **Full length GIV protein** |
| Sample Type | Colorectal cancer cases (4 μm section) |
| Paraffin | Selected |
| Deparaffinization | Selected |
| Cell Conditioning | CC1, 48 min |
| Pre Primary Peroxidase Inhibition | Selected |
| Primary Ab Temp | Selected, 37 °C |
| Primary Ab Incubation | 12 min |
| Amplify | Not Selected |
| Counterstain | Hematoxylin II, 4 min |
| | Bluing reagent, 4 min |

Annotated cancer cell lines (the expression of GIV-fl protein was previously determined by RT-PCR) were used to test the specificity and sensitivity of the GIV IHC assay:

- DLD1 colon invasive: detectable GIV-fl mRNA and/or protein,
- HT29 colon poorly invasive: undetectable GIV-fl mRNA and/or protein,
- Ls174T colon poorly invasive: undetectable GIV-fl mRNA and/or protein,
- MDA MB231 breast invasive: detectable GIV-fl mRNA and/or protein,
- MCF7 breast non-invasive: undetectable GIV-fl mRNA and/or protein.

According to the published data, the highly invasive cell lines have 20-50 fold more GIV full length protein level than their noninvasive counterparts. The noninvasive cells contain mostly the C-terminal truncated GIV protein (created by alternatively splicing the full length mRNA) therefore these cell lines should not demonstrate much reactivity with GIV (fl) antibody raised against the C-terminal end (SP173, see Example 1). The GIV IHC assay described above was applied on theses cell lines. Stained slides were evaluated by a pathologist.

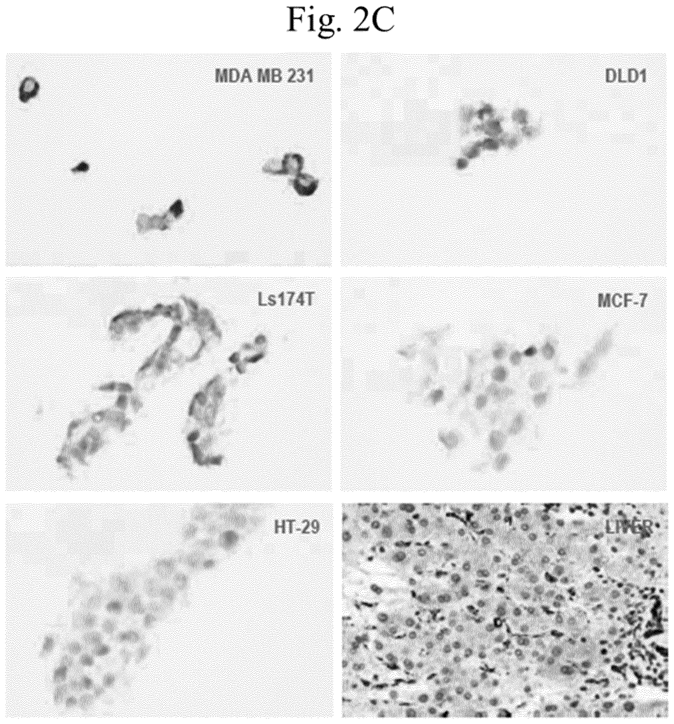

In agreement with the published data, GIV IHC assay resulted in positive staining in invasive breast MDA MB231 and colon DLD1 cell lines while the non-invasive or poorly invasive cell lines were mostly negative for the full length GIV protein (FIG. 2C). No difference was observed in GIV-fl expression of steady state, starved or EGF stimulated cells by IHC.

GIV IHC assay was evaluated on dissected GIV KO mouse organs. Although, heterozygous and homozygous samples showed reduced GIV staining compared to the wild type, complete depletion of GIV protein was not detected by IHC. In order to match homo- (-/-) or heterozygous (-/+) for girdin, the primary antibody concentration was decreased from 10 to 5; 2.5 and 1 μg/ml. KO mice organs showed positive staining even at 1 ug/ml GIV-Ab concentration.

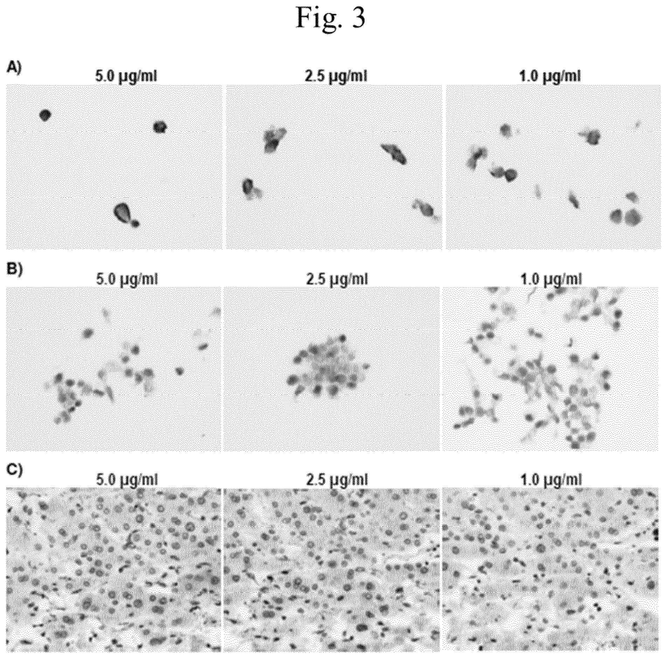

Since complete depletion of GIV was unlikely to occur in mutant mice, the annotated control cell lines and normal liver samples were used to finalize GIV antibody concentration. The GIV IHC assay was repeated with the optimal conditions but using 5, 2.5 and 1 μg/ml of primary antibody. Although the breast cancer cell line remained positive even at 1 μg/ml primary antibody, either significantly reduced signal or complete loss of signal was observed in normal liver and invasive colon cell line at 5 $\mu$g/ml antibody (FIGS. 3A-3C).

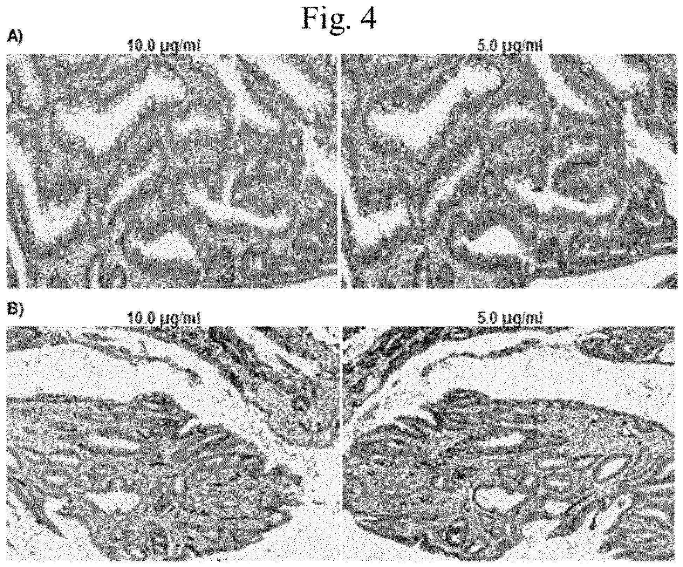

GIV staining with lower antibody concentration was also tested the 10 patient CRC cohort (Table 3) (better and worse outcome). Since 5 $\mu$g/ml GIV titer did not change the GIV status in colon cases (FIGS. 4A-4B) rather decreased the unnecessary background staining therefore the final GIV antibody concentration was set at 7.5 $\mu$g/ml.

**Example 6 (prophetic)**

**Assessing level of GIV-fl gene expression by qRT-PCR**

CRC patient samples are used in this experiment. RNA is isolated from fresh or frozen tissue or FFPET using e.g., the protocol described in Molecular Cloning. A Laboratory Manual by T Maniatis, E F Fritsch and J Sambrook, Cold Spring Harbor Laboratory, New York. 1982, or U.S. Patent No. 6,248,535 respectively.

In this example, the amplification of the GIV-fl target utilizes a forward primer, a reverse primer and the detection probe specific for the GIV-fl mRNA. One of the primers is specific for the last coding exon of the GIV gene. A parallel reaction utilizes a forward primer, a reverse primer and the detection probe specific for the housekeeping gene GADPH. An exemplary reaction mixture comprises 0.075-0.2 $\mu$M of each forward primer, reverse primer and the detection probe, nucleoside triphosphates, 3 mM $MgCl_2$, 50 mM Tricine pH 8.0, 55 mM potassium acetate, 160 -200 $\mu$M each dATP, dCTP, and dGTP, 160 $\mu$M dTTP (or optionally 320 $\mu$M dUTP and 0.2 U/$\mu$L uracil-N-glycosylase), thermostable DNA polymerase with reverse transcriptase activity, 0.1 mM EDTA, 1.25%-2% DMSO, 2.5 mM magnesium acetate and target DNA (template). Amplification and analysis are performed using the Roche LightCycler® 480 instrument (Roche Applied Science, Indianapolis, Ind.) An exemplary temperature profile is: 50°C 5 minutes; 2 cycles of 95°C (10 seconds) to 55-65°C (30 seconds) followed by cycling from 93°C (10 seconds) to 55-65°C (30 seconds) 40-60 times, 1 cycle cool down to 37°C (10 seconds), and 1 cycle cool down to 25°C (10 seconds). Fluorescence data is collected at the start of each 55-65°C step and $C_t$ values are determined for the reaction amplifying the target GIV-fl mRNA and the control GADPH mRNA in each sample. The relative expression of the GIV-fl gene is measured as the difference between the $C_t$ values of the target and control genes.

**Example 7**

**Assay Validation in Colorectal Cancer (CRC)**

This example describes methods used to analytically validate GIV expression by immunohistochemistry (IHC) in a large cohort of stage II CRC samples with known clinical outcomes.

A set of 192 stage II colorectal cancer cases was used to validate GIV expression as a predictive biomarker to further predict the tumor recurrence/prognosis for MMR proficient (pMMR) stage II CRC. Samples were stained with the optimized GIV immunohistochemistry assay (Table 1). For negative staining, rabbit monoclonal negative control Ig was used as primary antibody (VMSI Catalog # 790-4795; Table 5).

**Table 5**: Rabbit monoclonal negative control Ig Immunoassay

| 1 | Paraffin [Selected] |
|---|---|
| 2 | Deparaffinization [Selected] |
| 3 | Cell Conditioning [Selected] |
| 4 | CC1 [Selected] |
| 5 | CC1 8 Min [Selected] |
| 6 | CC1 16 Min [Selected] |
| 7 | CC1 24 Min [Selected] |
| 8 | CC1 32 Min [Selected] |
| 9 | CC1 40 Min [Selected] |
| 10 | CC1 46 Min [Selected] |
| 11 | CC1 56 Min [Selected] |
| 12 | CC1 64 Min [Selected] |
| 13 | Pre Primary Peroxidase inhibit [Selected] |
| 14 | Primary Antibody [Selected] |
| 15 | Primary Antibody Temperature [Selected] |
| 16 | Warmup Slide to Ab Incubation Temperature [Primary Antibody] |

(continued)

17 Apply Coversip, One Drop of [ANTIBODY 30] (Antibody), and Incubate for [0 Hr 16 Min]

18 Counterstain [Selected]

19 Apply One Drop of [HEMATOXYLIN II] (Counterstain). Apply Coverslip, and Incubate for [4 Minutes]

20 Post Counterstain [Selected]

21 Apply One Drop of [BLUING REAGENT] (Post Counterstain). Apply Coverslip, and Incubate for [4 Minutes]

Stained slides were scored for predominant signal intensity and for percentage of positive tumor cell staining (Table 6).

**Table 6.** GIV IHC results on the 192 stage II CRC samples

| Case ID | H&E (tumor present: yes/no) | Negative Control | Predominant Signal Intensity/Gestalt (0-3+) | % positive | Nuclear staining present (yes/no) |
|---|---|---|---|---|---|
| 00A1026-1C | Yes | Acceptable | 2+ | 35 | No |
| 00A6122-I | Yes | Acceptable | 1+ | 15 | No |
| 01A1336-1C | Yes | Acceptable | 1+ | 10 | No |
| 02A5647-1I | Yes | Acceptable | 1+ | 10 | No |
| 04A14059-3B | yes | Acceptable | 0 | 0 | No |
| 04A15855-1B | Yes | Acceptable | 2+ | 50 | No |
| 04A20697-1D | Yes | Acceptable | 1+ | 4 | No |
| 05A10353-D | Yes | Acceptable | 1+ | 1 | No |
| 05A11669-B | Yes | Acceptable | 1+ | 10 | Yes |
| 05A12666-1C | Yes | Acceptable | 2+ | 60 | No |
| 05A13217-B | Yes | Acceptable | 2+ | 85 | No |
| 05A14795-A | Yes | Acceptable | 1+ | 10 | No |
| 05A16650-1E | Yes | Acceptable | 2+ | 10 | No |
| 05A17166-2D | Yes | Acceptable | 1+ | 50 | No |
| 05A19460-D | Yes | Acceptable | 1+ | 20 | No |
| 05A21057-D | Yes | Acceptable | 2+ | 30 | No |
| 05A21534-A | Yes | Acceptable | 2+ | 85 | No |
| 05A2963-1E | Yes | Acceptable | 2+ | 35 | No |
| 06A12643-F | Yes | Acceptable | 1+ | 5 | No |
| 06A13463-L | Yes | Acceptable | 1+ | 40 | No |
| 06A14696-2B | Yes | Acceptable | 1+ | <1 | No |
| 06A15628-E | Yes | Acceptable | 2+ | 2 | No |
| 06A16584-A | Yes | Acceptable | 1+ | 10 | Yes |
| 06A17444-1E | Yes | Acceptable | 2+ | 10 | No |
| 06A18488-E | Yes | Acceptable | 1+ | 40 | No |
| 06A18930-1A | Yes | Acceptable | 2+ | 35 | Yes |
| 06A20990-1C | Yes | Acceptable | 1+ | 3 | No |
| 06A21078-A | Yes | Acceptable | 2+ | 5 | No |

(continued)

| Case ID | H&E (tumor present: yes/no) | Negative Control | Predominant Signal Intensity/Gestalt (0-3+) | % positive | Nuclear staining present (yes/no) |
|---|---|---|---|---|---|
| 06A21661-D | Yes | Acceptable | 2+ | 7 | No |
| 06A22137-1B | Yes | Acceptable | 1+ | 8 | No |
| 07A20580-C | Yes | Acceptable | 1+ | 65 | No |
| 07A21258-B | Yes | Acceptable | 1+ | 35 | No |
| 07A22295-1D | Yes | Acceptable | 0 | 0 | No |
| 07A25123-C | Yes | Acceptable | 2+ | 6 | No |
| 07A25327-1G | Yes | Acceptable | 1+ | 4 | No |
| 07A25823-C | Yes | Acceptable | 1+ | 4 | No |
| 07A26280-B | Yes | Acceptable | 3+ | 60 | Yes (3+; 90%) |
| 07A27529-1B | Yes | Acceptable | 2+ | 80 | No |
| 07A27620-B | Yes | Acceptable | 0 | 0 | No |
| 07A28429-C | Yes | Acceptable | 1+ | 90 | No |
| 07A29535-C | Yes | Acceptable | 1+ | 5 | No |
| 07A30498-1D | Yes | Acceptable | 1+ | 45 | No |
| 07A31038-F | Yes | Acceptable | 2+ | 60 | No |
| 07A32490-C | Yes | Acceptable | 1+ | 7 | No |
| 08A10093-C | Yes | Acceptable | 2+ | 3 | No |
| 08A21989-C | Yes | Acceptable | 1+ | 90 | No |
| 08A23446-1D | Yes | Acceptable | 1+ | 80 | No |
| 08A24383-F | Yes | Acceptable | 1+ | 80 | No |
| 08A26142-B | Yes | Acceptable | 0 | 0 | No |
| 09A619-D | Yes | Acceptable | 0 | 0 | No |
| 02A0517-1C | Yes | Acceptable | 1+ | 10 | No |
| 02A18759-C | Yes | Acceptable | 1+ | 95 | No |
| 02A3045-1C | Yes | Acceptable | 2+ | 20 | No |
| 04A12674-B | Yes | Acceptable | 0 | 0 | No |
| 06A2249-1N | Yes | Acceptable | 2+ | 5 | No |
| 06A3482-1D | Yes | Acceptable | 2+ | 8 | No |
| 06A3578-1G | Yes | Acceptable | 0 | 0 | No |
| 06A4157-1C | Yes | Acceptable | 1+ | 60 | No |
| 06A5014-A | Yes | Acceptable | 2+ | 2 | No |
| 06A6684-1D | Yes | Acceptable | 1+ | 2 | No |
| 06A7316-1C | Yes | Acceptable | 1+ | 35 | No |
| 06A7985-1D | Yes | Acceptable | 1+ | 25 | No |
| 06A9173-B | Yes | Acceptable | 1+ | 20 | No |
| 06A9592-1M | Yes | Acceptable | 0 | 0 | No |
| 07A10014-1E | Yes | Acceptable | 1+ | 7 | No |

(continued)

| Case ID | H&E (tumor present: yes/no) | Negative Control | Predominant Signal Intensity/Gestalt (0-3+) | % positive | Nuclear staining present (yes/no) |
|---|---|---|---|---|---|
| 07A1016-1E | Yes | Acceptable | 1+ | 2 | Yes |
| 07A10357-A | Yes | Acceptable | 1+ | 55 | No |
| 07A1523-1E | Yes | Acceptable | 1+ | 8 | No |
| 07A2051-D | Yes | Acceptable | 1+ | 8 | No |
| 07A2577-D | Yes | Acceptable | 1+ | 20 | No |
| 07A27758-C | Yes | Acceptable | 1+ | 50 | No |
| 07A3796-1D | Yes | Acceptable | 2+ | 20 | No |
| 07A4252-C | Yes | Acceptable | 1+ | 75 | No |
| 07A6444-1B | Yes | Acceptable | 2+ | 25 | No |
| 07A6924-1C | Yes | Acceptable | 1+ | 20 | No |
| 07A8451-B | Yes | Acceptable | 1+ | 5 | No |
| 07A8691-C | Yes | Acceptable | 1+ | 4 | No |
| 07A9065-B | Yes | Acceptable | 0 | 0 | No |
| 07A9116-C | Yes | Acceptable | 2+ | 6 | No |
| 07A9194-E | Yes | Acceptable | 1+ | 5 | No |
| 07A921-1C | Yes | Acceptable | 1+ | 4 | Yes |
| 08A1198-B | Yes | Acceptable | 2+ | 35 | No |
| 08A1577-C | Yes | Acceptable | 2+ | 10 | No |
| 08A3217-D | Yes | Acceptable | 1+ | 5 | No |
| 08A3404-D | Yes | Acceptable | 2+ | 35 | No |
| 08A3746-C | Yes | Acceptable | 2+ | 1 | No |
| 08A4872-D | Yes | Acceptable | 1+ | 99 | No |
| 08A498-C | Yes | Acceptable | 0 | 0 | No |
| 08A5142-C | Yes | Acceptable | 1+ | 30 | No |
| 08A5453-B | Yes | Acceptable | 1+ | 5 | No |
| 08A6122-D | Yes | Acceptable | 1+ | 40 | No |
| 08A7715-1C | Yes | Acceptable | 1+ | 35 | No |
| 08A822-B | Yes | Acceptable | 2+ | 35 | Yes |
| 08A8610-E | Yes | Acceptable | 0 | 0 | No |
| 08A9631-1F | Yes | Acceptable | 2+ | 10 | No |
| 08A9717-C | Yes | Acceptable | 1+ | 6 | Yes |
| 09A155-C | Yes | Acceptable | 2+ | 2 | No |
| 09A96-E | Yes | Acceptable | 2+ | 2 | No |
| 10A23348-E | Yes | Acceptable | 1+ | 3 | No |
| 11A29407-1D | Yes | Acceptable | 0 | 0 | Yes (3+; 75%) |
| 00A3119-E | Yes | Acceptable | 1+ | 90 | No |
| 01A17541-B | Yes | Acceptable | 2+ | 20 | No |

(continued)

| Case ID | H&E (tumor present: yes/no) | Negative Control | Predominant Signal Intensity/Gestalt (0-3+) | % positive | Nuclear staining present (yes/no) |
|---|---|---|---|---|---|
| 02A3063-1D | Yes | Acceptable | 1+ | <1 | No |
| 02A7072-3M | Yes | Acceptable | 1+ | 4 | No |
| 03A3541-1D | Yes | Acceptable | 2+ | 10 | No |
| 03A3673-1B | Yes | Acceptable | 2+ | 40 | No |
| 03A4545-1C | Yes | Acceptable | 1+ | 20 | No |
| 03A7890-1F | Yes | Acceptable | 2+ | 80 | No |
| 03A14704-1C | Yes | Acceptable | 1+ | 5 | No |
| 04A2285-1B | Yes | Acceptable | 0 | 0 | No |
| 04A5521-1A | Yes | Acceptable | 0 | 0 | No |
| 05A2500-1F | Yes | Acceptable | 1+ | 1 | No |
| 05A13714-D | Yes | Acceptable | 1+ | 70 | Yes (2+) |
| 05A19183-B | Yes | Acceptable | 2+ | 40 | No |
| 05A19886-1E | Yes | Acceptable | 1+ | 45 | Yes |
| 06A1251-E | Yes | Acceptable | 0 | 0 | No |
| 06A1299-1C | Yes | Acceptable | 1+ | 90 | No |
| 06A1518-1E | Yes | Acceptable | 0 | 0 | No |
| 06A2200-1D | Yes | Acceptable | 0 | 0 | No |
| 06A2603-1D | Yes | Acceptable | 1+ | 3 | No |
| 06A2792-1F | Yes | Acceptable | 0 | 0 | No |
| 06A3182-E | Yes | Acceptable | 2+ | 35 | No |
| 06A9658-1D | Yes | Acceptable | 1+ | 5 | No |
| 06A10044-2B | Yes | Acceptable | 0 | 0 | Yes (3+; 20%) |
| 06A10707-D | Yes | Acceptable | 1+ | 6 | No |
| 06A12718-F | Yes | Acceptable | 0 | 0 | No |
| 06A13059-1E | Yes | Acceptable | 1+ | 50 | Yes |
| 06A18530-1B | Yes | Acceptable | 3+ | 25 | Yes |
| 06A19265-C | Yes | Acceptable | 1+ | 45 | Yes |
| 06A19803-1E | Yes | Acceptable | 2+ | 55 | Yes |
| 06A20116-B | Yes | Acceptable | 1+ | 5 | No |
| 07A1514-1D | Yes | Acceptable | 0 | 0 | No |
| 07A10191-1C | Yes | Acceptable | 1+ | 10 | No |
| 07A21005-E | Yes | Acceptable | 2+ | 75 | No |
| 07A22217-B | Yes | Acceptable | 1+ | 10 | No |
| 07A24787-D | Yes | Acceptable | 1+ | 15 | No |
| 07A26453-B | Yes | Acceptable | 0 | 0 | No |
| 07A26589-C | Yes | Acceptable | 1+ | 7 | No |
| 07A29013-B | Yes | Acceptable | 1+ | 60 | Yes |

(continued)

| Case ID | H&E (tumor present: yes/no) | Negative Control | Predominant Signal Intensity/Gestalt (0-3+) | % positive | Nuclear staining present (yes/no) |
|---|---|---|---|---|---|
| 07A33075-E | Yes | Acceptable | 1+ | 40 | Yes (60%) |
| 08A20458-D | Yes | Acceptable | 1+ | 35 | No |
| 08A26455-E | Yes | Acceptable | 2+ | 40 | Yes |
| 08A28427-E | Yes | Acceptable | 1+ | 25 | Yes (4+) |
| 09A883-C | Yes | Acceptable | 2+ | 10 | No |
| 09A1181-1D | Yes | Acceptable | 1+ | 1 | No |
| 09A1215-E | Yes | Acceptable | 0 | 0 | No |
| 09A3356-C | Yes | Acceptable | 1+ | 25 | No |
| 09A3848-C | Yes | Acceptable | 2+ | 40 | Yes |
| 10A22093-E | Yes | Acceptable | 2+ | 70 | Yes |
| 6A110044-1C | Yes | Acceptable | 2+ | 40 | No |
| 00-16804-B | Yes | Acceptable | 2+ | 35 | Yes |
| 00-20289-C | Yes | Acceptable | 1+ | 20 | No |
| 01-104530-C | Yes | Acceptable | 1+ | 10 | No |
| 01-15728-D | Yes | Acceptable | 1+ | 35 | No |
| 00A2038-D | Yes | Acceptable | 2+ | 25 | No |
| 01A3315-1B | Yes | Acceptable | 1+ | 7 | No |
| 01A4630-1B | Yes | Acceptable | 2+ | 30 | No |
| 02A6377-1F | Yes | Acceptable | 2+ | 40 | No |
| 02A11503-B | Yes | Acceptable | 1+ | 4 | No |
| 02A17602-E | Yes | Acceptable | 0 | 0 | No |
| 03A2250-1D | Yes | Acceptable | 2+ | 10 | No |
| 03A2334-1E | Yes | Acceptable | 1+ | 20 | No |
| 03A4041-1C | Yes | Acceptable | 2+ | 5 | No |
| 03A14751-E | Yes | Acceptable | 2+ | 25 | No |
| 04A7560-1A | Yes | Acceptable | 1+ | 60 | No |
| 04A13501-C | Yes | Acceptable | 1+ | 7 | No |
| 05A1060-1G | Yes | Acceptable | 3+ | 94 | No |
| 05A1891-1B | Yes | Acceptable | 2+ | 7 | Yes |
| 05A2558-1E | Yes | Acceptable | 3+ | 80 | No |
| 05A4276-1 | Yes | Acceptable | 1+ | 25 | No |
| 05A4629-1C | Yes | Acceptable | 1+ | 3 | No |
| 05A5756-1E | Yes | Acceptable | 0 | 0 | Yes |
| 05A6341-E | Yes | Acceptable | 2+ | 65 | No |
| 05A6414-E | Yes | Acceptable | 1+ | 50 | No |
| 05A7548-1D | Yes | Acceptable | 2+ | 35 | No |
| 05A7834-1D | Yes | Acceptable | 2+ | 60 | No |

(continued)

| Case ID | H&E (tumor present: yes/no) | Negative Control | Predominant Signal Intensity/Gestalt (0-3+) | % positive | Nuclear staining present (yes/no) |
|---|---|---|---|---|---|
| 05A17127-A | Yes | Acceptable | 1+ | 2 | No |
| 05A19004-1E | Yes | Acceptable | 1+ | 1 | Yes |
| 06A165-1G | Yes | Acceptable | 3+ | 5 | No |
| 06A177-1D | Yes | Acceptable | 0 | 0 | No |
| 06A14020-E | Yes | Acceptable | 1+ | 5 | No |
| 06A18238-A | Yes | Acceptable | 2+ | 15 | No |
| 06A18241-D | Yes | Acceptable | 1+ | 40 | No |
| 07A0882-1F | Yes | Acceptable | 1+ | 35 | No |
| 07A7276-E | Yes | Acceptable | 1+ | 4 | No |
| 07A7601-B | Yes | Acceptable | 2+ | 1 | No |
| 07A10206-1C | Yes | Acceptable | 2+ | 2 | No |
| 07A25413-E | Yes | Acceptable | 1+ | 10 | No |
| 07A27032-1B | Yes | Acceptable | 0 | 0 | No |
| 07A29654-C | Yes | Acceptable | 1+ | 35 | Yes |
| 08A26322-E | Yes | Acceptable | 2+ | 15 | No |
| 11A25489-D | Yes | Acceptable | 1+ | 60 | Yes (2+; 55%) |

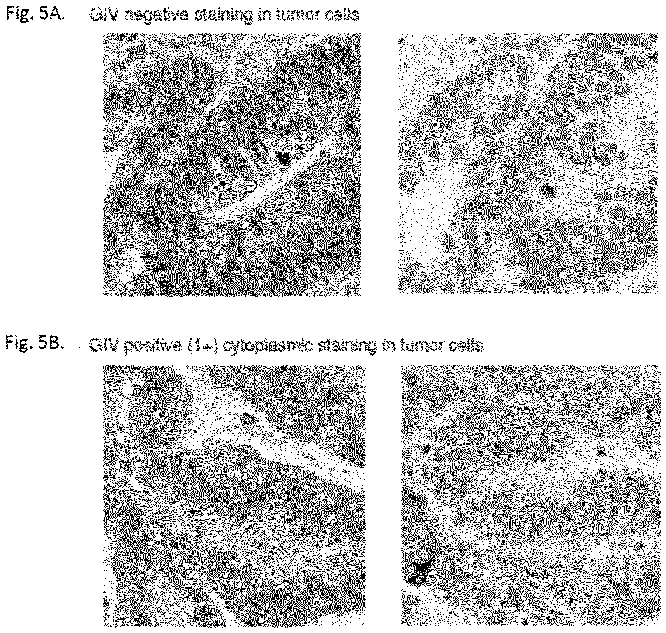

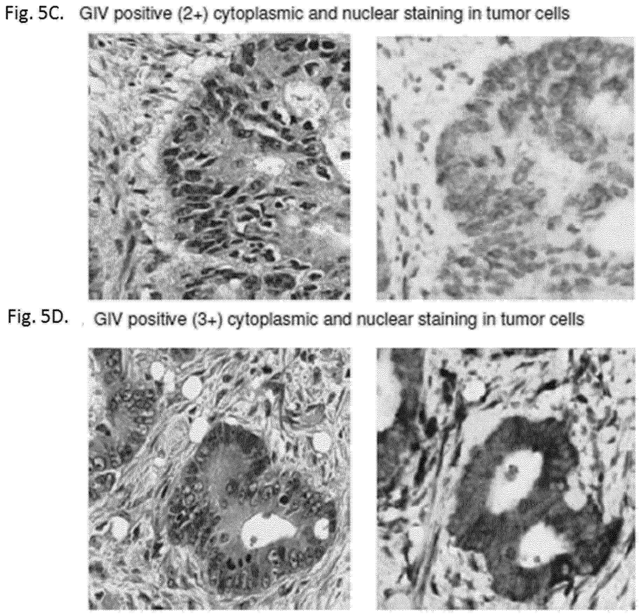

As shown in Table 6, GIV IHC was evaluable in all colorectal cancer cases. Stromal tissues consistently stained strongly positive, whereas tumor epithelia showed variable staining pattern (cytoplasmic and/or nuclear staining) with variable staining intensity and percentage of positive tumor cells. FIGS. 5A-5D show examples of IHC staining of GIV in human colorectal cancers.

**Example 8**

**Development of GIV-fl Scoring Algorithm**

This example describes methods used to develop a scoring algorithm for GIV IHC staining, based on the data described in Example 6 for the 192 CRC samples.

GIV IHC data were used to develop a scoring algorithm. Eleven scoring metrics were evaluated that incorporated the intensity of the staining and the extent of positive staining separately or in combination (Table 7). Seven of these required a binning of the percent staining into four categories and then these were assigned a rank number (0-3) that was either summed or multiplied by staining intensity. Positive and negative GIV was then based on cut-points from these summation products. The remaining four metrics were developed based on either staining intensity only or percent staining only and then two different types of cut-points for each of these metrics were used to define positive and negative GIV.

Table 7. Displays metrics and explanations of candidate methods for scoring GIV expression, assessed in terms of ability to discriminate between patients likely to experience a recurrence. A total of 11 scoring systems (5 scoring algorithms, each with multiple cut-points for positivity) were assessed in this analysis.

| Marker (Variable Names) | Staining Intensity | Percent Positive Staining Treatment | Method of Composition | Negative / Positive Definitions |
|---|---|---|---|---|
| **GIV** Intensity-Only Scoring Algorithms | Range: [0,1,2,3] | | | 0 / 1-3 |
| | | - | - | 0-1 / 2-3 |

(continued)

**Table 7**. Displays metrics and explanations of candidate methods for scoring GIV expression, assessed in terms of ability to discriminate between patients likely to experience a recurrence. A total of 11 scoring systems (5 scoring algorithms, each with multiple cut-points for positivity) were assessed in this analysis.

| Marker (Variable Names) | Staining Intensity | Percent Positive Staining Treatment | Method of Composition | Negative / Positive Definitions |
|---|---|---|---|---|
| **GIV** Composite Scoring Algorithms | Range: [0,1,2,3] | Binning Method #1: [0,1,2,3] 0: 0%-10% 1: 11%-25% 2: 26%-50% 3: 51%-100% | SUM (+) Range: [0,1,2,3,4,5,6] | 0-2 / 3-6 0-3 / 4-6 |
| | Range: [0,1,2,3] | Binning Method #2: [0,1,2,3] 0: 0%-10% 1: 11%-35% 2: 36%-50% 3: 51%-100% | SUM (+) Range: [0,1,2,3,4,5,6] | **0-2 / 3-6** 0-3 / 4-6 0-4 / 5-6 |
| | Range: [0,1,2,3] | Binning Method #2 (See above) | PRODUCT (*) Range: [0,1,2,3,4,6,9] | 0-3 / 4, 6, 9 0-4 / 6, 9 |
| **GIV** Percent-Only Scoring Algorithms | Range: [0%-100%] | Dichotomized: Median as cutpoint Q3 as cutpoint | - | 0%-10% / 11%+ 0%-35% / 36%+ |

In a statistical context, association is demonstrated by the difference in outcome for marker positive and marker negative patients. This was established by examining the separation of survival curves between GIV-fl positive and GIV-fl negative patients. This is different from statistical prediction; in general, association is a necessary but not sufficient, indication for predictive value (Pepe et al., Am J Epidemiol 159:882-90, 2004).

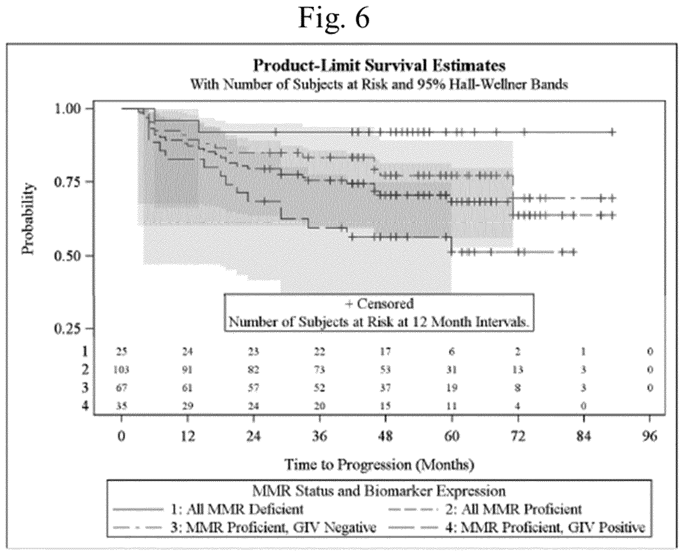

While all of the scoring metrics showed strong correlation between GIV expression and progression, only GIV-fl Extent and Intensity Score (bolded and underlined in Table 7) demonstrated a statistically significant association (p value = 0.0212) with the time-to-progression outcome in pMMR, chemo naive patients (FIG. 6). FIG. 6 shows the separation that is achieved stratifying by GIV-fl status in the pMMR population of stage II CRC samples using the GIV-fl Extent and Intensity Score scoring system. The lines from top to bottom show worsening prognosis, that is, less likelihood of survival over time. The top line shows dMMR samples, while the bottom shows pMMR and GIV positive samples. The survival curve for the pMMR population is shown for comparative purposes. The p-value for this log-rank test is 0.0212, which demonstrates statistical separation in the survival experiences between the GIV-fl positive and negative groups. This establishes that GIV-fl can provide predictive value.

Thus, in some examples, GIV-fl staining is assessed in a stage II (such as stage IIa) colon cancer sample that is pMMR, by first determining an extent of positive GIV-fl staining for the sample on a scale of 0 to 3, wherein extent of positive staining is assigned 0 if 0% to 10% of the total area of the sample is stained positive, wherein extent of positive staining is assigned 1 if 11 %-35% of the total area of the sample is stained positive, wherein extent of positive staining is assigned 2 if 36%-50% of the total area of the sample is stained positive, and wherein extent of positive staining is assigned 3 if 51%-100% of the total area of the sample is stained positive. The resulting value (0, 1, 2, or 3) is then added to the GIV-fl intensity of staining for the sample (an intensity score of on a scale of 0 (negative), 1 (weak), 2 (moderate), to 3 (strong)), to provide a GIV-fl Extent and Intensity Score value. So, if a patient has an extent of positive GIV-fl staining of 8% with a GIV-fl intensity of staining of 2 then they would receive a 0 for percent positivity, the GIV-fl Extent and Intensity Score value would be 0 + 2 = 2. If the GIV-fl Extent and Intensity Score value is 0-2, then the sample is assigned to be negative for GIV-fl staining, while a GIV-fl Extent and Intensity Score value of 3-6 is assigned to be positive for GIV-fl staining. Thus, each patient or sample is thus either a positive or negative based on a binned score for overall percent staining and the overall intensity.

A simpler scoring metric was developed that can be used instead of the GIV-fl Extent and Intensity Score scoring algorithm in Table 7. This method also relies on staining the sample using a GIV-fl Ab. In the simpler GIV-fl scoring metric, called the GIV-fl Predictive Score, a case or sample is considered GIV positive if (1) its overall percent staining is greater than 10% any staining intensity or (2) if the predominant staining intensity is 3+ with any percent. Thus, in some examples, GIV-fl staining is assessed in a pMMR stage II (such as stage IIa) colon cancer sample, by scoring the sample as GIV-fl positive if the overall percent of GIV-fl staining in the sample is greater than 10% (with any staining intensity), or if the predominant intensity of GIV-fl staining in the sample is 3+ (regardless of the extent of staining). In contrast, if the pMMR stage II colon cancer sample has less than 10% overall GIV-fl staining and has an predominant GIV-fl staining intensity of less than 3+, it is assigned GIV-fl negative.

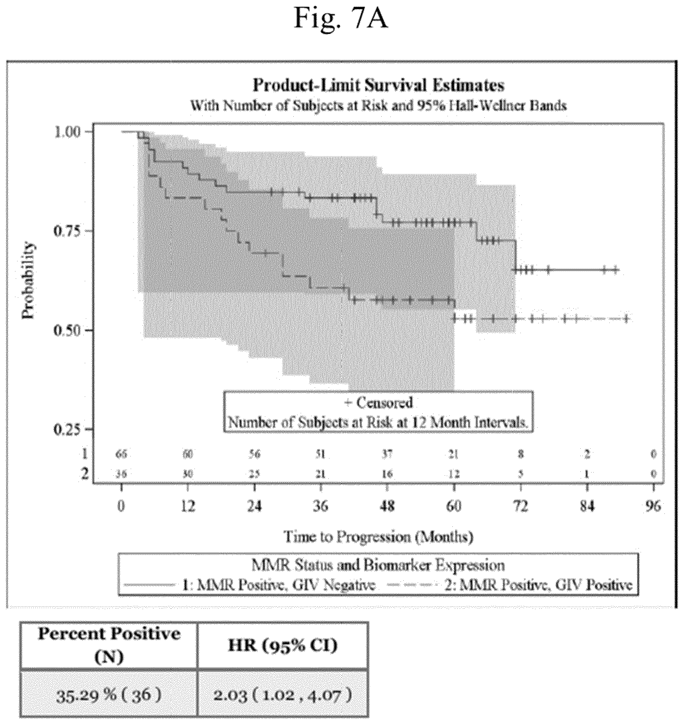

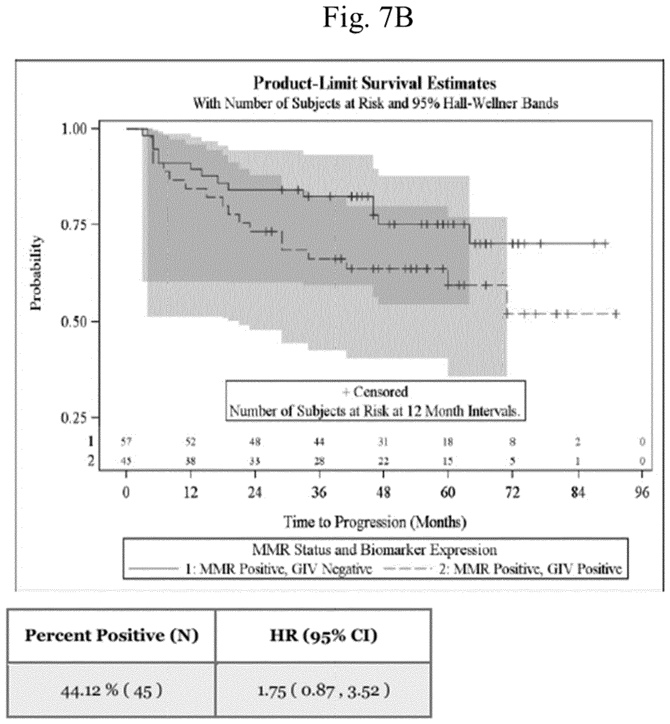

As shown in FIGS. 7A-7B, both scoring metrics (GIV-fl Extent and Intensity Score and GIV-fl Predictive Score) provided discrimination on survival, with hazard ratios (HR) of 2.03 and 1.75, respectively.

The new IHC assay can detect full length GIV protein (GIV-fl) in formalin fixed, paraffin-embedded tissue. 192 colorectal cases were first evaluated by IHC for MMR and then GIV proteins. Out of the 192 samples, 102 cases were pMMR, chemo naive patients, and only 32/102 patients showed disease recurrence (progression =yes). Based on the optimized scoring algorithms, 16/32 (50%; GIV-fl Extent and Intensity Score) or 18/32 (56%; GIV-fl Predictive Score) patients with recurrence had a GIV positive tumor (Table 8). Thus, if a stage II CRC (such as stage IIa) is pMMR and GIV-fl positive, this indicates that the patient is more likely to experience a progression or recurrence, for example within 12 to 96 months, such as within 12 months, within 24 months, within 36 months, within 48 months, within 60 months, within 72 months, within 84 months, or within 96 months. Recurrence can be a local recurrence (getting cancer again in the same area) or progression of the cancer to either local nodes (stage III), or distant nodes (stage IV, metastatic) or other sites (e.g., liver or lungs).

Table 8: Prognostic ability of GIV IHC in pMMR, chemo naive CRC patients using GIV-fl Extent and Intensity Score or GIV-fl predictive scoring algorithms

| GIV-fl Extent and Intensity Score | | Progression | |
| --- | --- | --- | --- |
| | | No | Yes |
| GIV Stain | Negative | 50 | 16 |
| | Postive | 20 | 16 |
| | | | |
| GIV-fl Predictive Score | | | |
| | | No | Yes |
| GIV Stain | Negative | 43 | 14 |
| | Postive | 27 | 18 |

The data demonstrates that detection of GIV by IHC can be used as a prognostic biomarker in defining individual risk for recurrence in patients with pMMR, stage II (such as stage IIa) colorectal cancers not previously treated with adjuvant chemotherapy.

**Example 9**

**Statistical Prediction Model of GIV-fl Expression and Time to Progression in Stage II CRC**

This example describes methods used to evaluate other markers in combination with pMMR and GIV expression as prognostic/predictive indicators for stage II CRC patients.

A statistical model was developed from the subset of chemo-naive patients from the cohort described in Example 7. The GIV-fl Predictive Score was carried forward for the statistical predictive modeling. This statistical model was then evaluated by applying the fitted statistical prediction model to the subset of patients receiving chemotherapy. The development of the statistical prediction model was undertaken in four steps: 1) establishing association between biomarker and outcome (progression free survival, PFS); 2) model selection; 3) assessment of statistical predictive ability; and 4) demonstrating individual-level predictive ability. Lastly, the validity of the model to inform individual patient risk; as well as demonstrate the ability of the statistical predictive model, including the biomarker GIV-fl, to predict treatment response was determined.

The cohort described in Example 7 included 103 pMMR, chemo-naïve patients, with 31% (32/103) of evaluable patients experiencing disease recurrence. Table 9 describes the distribution of clinical and pathologic variables that describe the 103 pMMR, chemo-naïve patients. This cohort was oversampled for recurrence, to ensure proper characterization of the outcome. For purposes of the scoring algorithm, all 103 patients were used. For the development of the statistical prediction model, the population was limited to stage IIa patients, as this subgroup of patients need further high-and-low risk classification, and clarification of decisions about adjuvant chemotherapy.

**Table 9.** Patient Characteristics

| Variable | N | Proportion Progressing |
|---|---|---|
| **LVI** | | |
| No | 75 | 0.27 |
| Not reported | 7 | 0.14 |
| Yes | 21 | 0.52 |
| **Age[a]** | | |
| [50.9,65.8) | 26 | 0.27 |
| [65.8,71.7) | 26 | 0.38 |
| [71.7,79.5) | 26 | 0.31 |
| [79.5,92.4] | 25 | 0.32 |
| **Number of Nodes** | | |
| <12 | 27 | 0.33 |
| ≥12 | 76 | 0.30 |
| **Sex** | | |
| F | 46 | 0.37 |
| M | 57 | 0.26 |
| **Tumor Differentiation** | | |
| Moderate | 84 | 0.27 |
| Poor | 18 | 0.44 |
| Well | 1 | 1.00 |
| **T Stage (TMN class)** | | |
| 3 | 91 | 0.29 |
| 4 | 12 | 0.50 |
| **Side** | | |
| left | 59 | 0.29 |
| right | 44 | 0.34 |
| **Overall** | 103 | 0.31 |
| [a]quartiles based on continuously expressed age at diagnosis | | |

**Scoring Algorithm**

Eleven scoring algorithms were evaluated that incorporated staining intensity and percent staining. This is similar to what was done in Example 7, expect that in Example 7, all 192 stage II CRC samples were analyzed, while here, only the subset of 103 chemo-naïve patients that were either tumor pathologic stage 3 or 4 (*e.g.*, T3/4) were analyzed. Some of these metrics were created by binning percent staining and intensity then obtaining a score (either multiplicative or additive) that included both of these measures. Other metrics used a method that optimized the percent staining, by finding the percent staining associated with the maximum rank test statistic, meaning the point at which high and low percent staining showed the largest difference between survival curves (Larson and Schumacher, Biometrics, 48, 73-85, 1992).

Table 10 summarizes the most robust metrics evaluated, and their performance in separating by progression free survival. The simple metric (GIV-fl Predictive Score) that incorporated an "optimal" opercent staining (> 10%) and also includes high intensity (3+), GIV-fl Predictive Score (see Example 7), and it is the scoring algorithm carried forward for the statistical predictive modeling. Forty-four percent (45/103) of the stage II patients (T3+T4) were GIV-fl positive; forty-seven percent (42/90) of the stage II patients were GIV-fl positive. The hazard ratio for the GIV-fl Predictive Score when applied to the stage II population alone was 2.56 (CI: 1.15, 5.87).

**Table 10.** Summary of Potential Scoring Algorithms, Based On 103 Stage II, pMMR, Chemo-Naïve Cases (Both T3 and T4).

| SCORING METRIC | % GIV Positive (n) | Hazard Ratio (95% CI) |
|---|---|---|
| *Composite Metrics: use both staining intensity (0-3) and percent staining (0%-100%). For 'Chundong, et. al.', 'Centile' and 'Composite' 1-3, an index based on binned percent staining was used and summed with staining intensity. Bins and cutpoints are defined below each metric.* | | |
| GIV-fl Predictive Score | 43.69 % (45) | 1.92 (0.94, |
| (>10% or 3+ intensity) | | 3.92) |
| Chundong et al., Anticancer Res 31: 1141-5,2011 (0: 0%, 1: 1-25%, 2: 26-50%, 3: >50%) Sum with intensity (neg: 0-3 / pos: 4-6) | 26.21 % (27) | 1.37(0.64, 2.91) |
| Centile (0: 0-2%, 1:3-9%, 2:10-34%, 3: >35%) Sum with intensity (neg: 0-3 / pos: 4-6) | 41.75 % (43) | 1.56 (0.77, 3.16) |
| Composite 1 (0: 0-10%, 1:11-25%, 2: 26-50%, 3:>50%) Sum with intensity (neg: 0-2 / pos: 3-6) | 38.83 % (40) | 1.84 (0.91, 3.73) |
| Composite 2 (this GIV-fl Extent and Intensity Score from Table 7) (0: 0-10%, 1:11-35%, 2:36-50%, 3: >50%) Sum with intensity (neg: 0-2 / pos: 3-6) | 34.95 % (36) | 2.19 (1.08, 4.43) |
| Composite 3 (0: 0-10%, 1:11-35%, 2: 36-50%, 3:>50%) Sum with intensity (neg: 0-3 / pos: 4-6) | 18.45 %(19) | 1.59 (0.71, 3.56) |
| *Percent-Only Metrics: using the percent staining only. Cutpoints are defined below each metric.* | | |
| Liu et al., Mol Biol Rep 39: 8717-22, 2012 (neg: 0% /pos: 1%+) | 84.47 % (87) | 1.23 (0.43, 3.51) |
| Median Percent (neg: 0%-10% / pos: 11%+) | 42.72 % (44) | 1.73 (0.85, 3.51) |
| Q3 Percent (neg: 0%-35% / pos: 36%+) | 20.39 % (21) | 1.63 (0.75, 3.54) |

**Establishing Statistical Association Between Marker and Progression**

In a statistical context, association is demonstrated by the difference in outcome for marker positive and marker negative patients. This was established herein by examining the separation of survival curves between GIV-fl positive and GIV-fl negative patients. This is different from statistical prediction; in general, association is a necessary but not sufficient, indication for predictive value (Pepe, Am J Epidemiol 159:882-90, 2004).

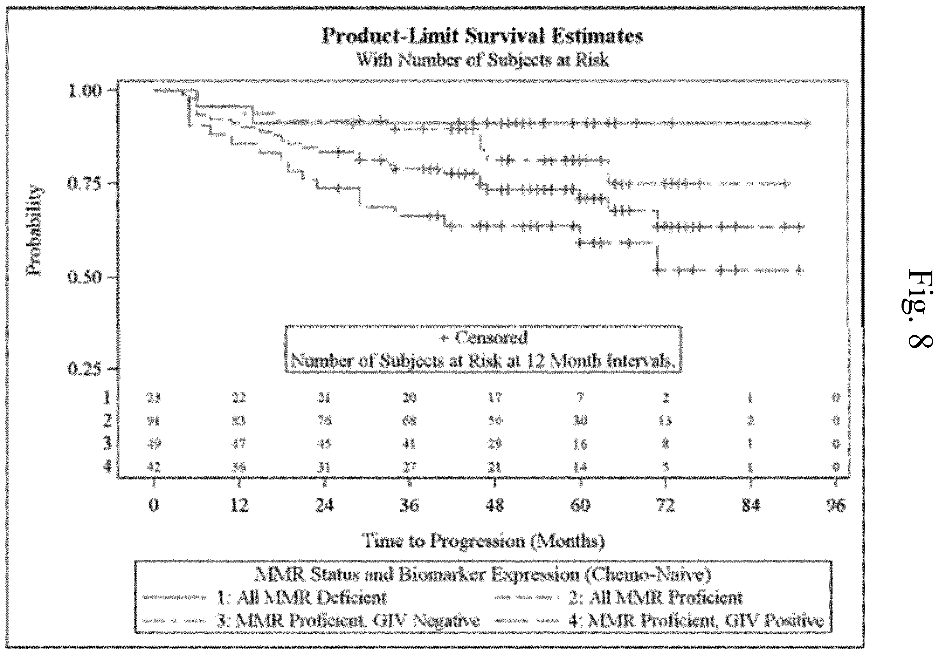

FIG. 8 shows the separation that is achieved stratifying by GIV-fl status in the pMMR population for the 103 chemo-naïve patients using the GIV-fl Predictive Score algorithm. The survival curve for the dMMR population is shown for comparative purposes. The p-value for this log-rank test is 0.0209, which demonstrates statistical separation in the survival experiences between the GIV-fl positive and negative groups. This demonstrates that GIV-fl provides predictive value. Forty percent (18/45) of the GIV-fl positive patients experienced a recurrence; twenty-six percent (14/57) GIV-fl negative patients recurred.

## Variable Selection and Cox Proportional Model

To identify variables, in addition to GIV-fl, to include in the statistical prediction model, it was first determined how many degrees of freedom available to allocate to a model. For survival analyses, it can generally fit up to the number of events divided by 10 or 20 without over-fitting. Over-fitting manifests in statistical prediction as performance being over-estimated, this would mean that when the statistical model is applied in practice it would not perform as well as expected. For this particular sample, it was expected to be able to fit approximately 2 degrees of freedom without model over-fitting. Variables deemed more important in the statistical model using the partial $\chi^2$ (adjusted for the degrees of freedom that each variable contributes) were identified. These test statistics are based on comparing the deviance, a statistical measure of goodness-of-fit that compares the difference in likelihoods from the full model compared to the model with each variable removed.

More critical variables, with respect to describing progression-free survival, have large partial $\chi^2$, while less critical variables have smaller values. If the sample size is large enough, it is generally better to use all available clinical and descriptive information that is available; however, when choosing between potential important variables, choosing on the basis of partial $\chi^2$ is preferred to a selection method that relies on step-wise selection methods.

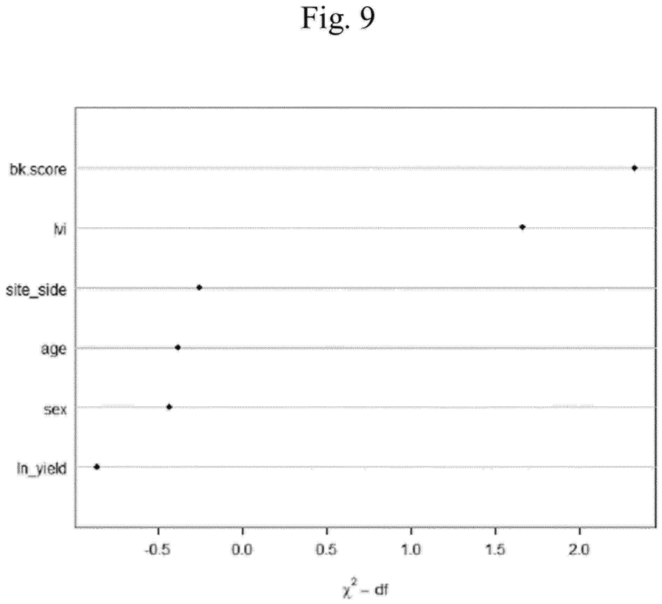

As shown in FIG. 9, there is a clear delineation between the first two variables (GIV-fl positive and lymphovascular invasion (LVI), the two dots on the upper right), and the other clnical variables (the dots on the left). This indicates that a predictive model with GIV-fl and LVI can be used to determine which stage II (such as stage IIa) pMMR CRCs will benefit from chemotherapy or biotherapy.

Table 11 is a summary that displays results from the Cox proportional hazards models for single variable, multivariable (includes all possible predictors listed in Table 11), and a final model with only GIV-fl and LVI variables. The hazard ratios were consistent (with respect to direction of effect) for all models, with more LVI and high GIV-fl levels associated with higher probability of recurrence. Table 11 is modeled in a similar fashion to the 12-gene recurrence score (Genomic Health, CA) validation studies using the QUASAR and CALGB study cohorts (Gray et al., J. Clin. Oncol. 29:4611-4619, 2011; Venook et al., J. Clin. Oncol. 31:1775-81, 2013); directionality of most these variables are similar to what was seen in these cohorts. Subtle differences (*e.g.*, higher risk of recurrence in females and right-sided tumors) may be due to the small sample size in this cohort, these variables did not demonstrate statistically significant differences in progression-free survival (PFS).

Table 11. Hazard ratios, and P-Values For Cox Proportional Models (pMMR, Chemo-Naïve Patients, Except Where Noted Otherwise).

| Variable | N | Single Covariate | | | GIV & Clinicopathologic Variables | | | Proposed Model: LVI & GIV | | | Direction |
|---|---|---|---|---|---|---|---|---|---|---|---|
| | | HR | 95% CI | P | HR | 95% CI | P | HR | 95% CI | P | |
| Dichotomous | | | | | | | | | | | |
| Lymphovascular Invasion[1] | 85 | 2.62 | 1.16 to 5.92 | 0.0208 | 2.31 | 0.95 to 5.61 | 0.0645 | 2.07 | 0.90 to 4.76 | 0.0872 | Invasion is worse |
| T Stage: T4 v T3[2] | 103 | 2.39 | 0.98 to 5.83 | 0.0553 | N/A | | | N/A | | | T Stage 4 is worse |
| Site Side | 91 | 1.26 | 0.58 to 2.73 | 0.5553 | 1.48 | 0.60 to 3.67 | 0.3918 | N/A | | | Right side is worse |
| Sex: female vs. male | 91 | 1.53 | 0.71 to 3.14 | 0.2793 | 1.40 | 0.58 to 3.34 | 0.4512 | N/A | | | Female is worse |
| Lymph Node Yield: <12 v ≥12 | 91 | 0.77 | 0.34 to 1.73 | 0.5252 | 1.17 | 0.48 to 2.85 | 0.7290 | N/A | | | <12 is worse |
| MMR Status[3] | 114 | 3.45 | 0.82 to 14.56 | 0.0916 | N/A | | | N/A | | | MMR Proficient is worse |
| MMR Status[4] | 128 | 4.18 | 1.00 to 17.45 | 0.0499 | N/A | | | N/A | | | MMR Proficient is worse |
| GIV Status | 91 | 2.49 | 1.11 to 5.58 | 0.0273 | 2.28 | 0.94 to 5.53 | 0.0684 | 2.56 | 1.12 to 5.87 | 0.0265 | GIV Positive is worse |
| Continuous | | | | | | | | | | | |
| Age | 91 | 1.02 | 0.98 to 1.06 | 0.4261 | 1.02 | 0.97 to 1.07 | 0.4312 | N/A | | | Older is worse |

1. Analysis as a single covariate did not include cases with LVI recorded as 'Not reported'.

2. Analysis included both T3 and T4 (Stage IIa and IIb), pMMR cases.

3. Analysis included both pMMR and dMMR, T3 (Stage IIa) cases.

4. Analysis included pMMR and dMMR cases that were both T3 and T4 (Stage IIa and IIb).

**Assessment of Predictive Ability**

The predictive ability of the statistical prediction model was assessed in two ways. First, how well the model classified patients into high and low risk, using the area under the ROC curve (AUC), was evaluated. Internal validation of the model(s) was determined by measuring bias in the AUC that is due to model over-fitting; bias is estimated by using boot-strap resampling. Boot-strap resamples are random samples that are taken with replacement. For this problem, 200 resamples were used, and for each of these resamples, a measure of the AUC was obtained. Over-fitting is estimated as optimism that is seen with fitted models compared to the resamples; the adjusted AUC is a way of adjusting for the potential over-optimistic AUC that could be seen when more variables are included than the sample size can adequately fit.

The models compared are the model proposed as the most useful statistical predictive model for this cohort: (1) a model that includes only the clinical/descriptive variables, as this model theoretically contains the information that is currently used in practice to evaluate patient risk; and (2) the full model, a model that includes both the marker and the current practice variables; and the GIV-fl (marker) model alone (as a baseline). It is expected that the full model will outperform all other models, as it uses all patient information. However, for these data performance of this statistical model may be difficult to assess due to over-fitting.

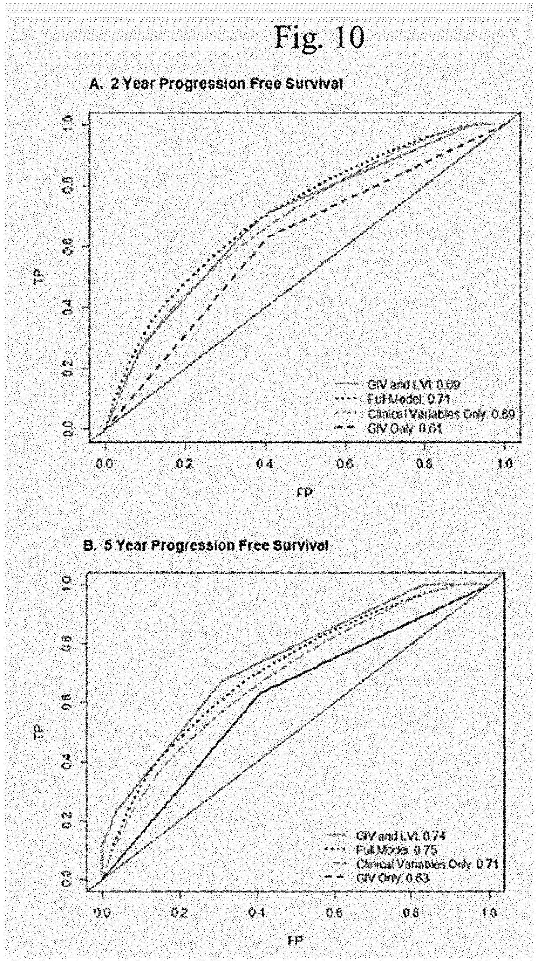

ROC curves for 2-year and 5-year PFS are shown in FIGS. 10A and 10B. The AUC shown in the figure legends are not adjusted for potential over-fitting, rather, these are shown in Table 12. The larger the AUC the better the sensitivity and specificity (also called the true positive and true negative value) of the marker. The AUC is highest at Max (sens,spec) - and generally "good" AUC values are greater than 0.70. Table 12 shows that when more than the two variables are added into the model, then the difference between the unadjusted and adjusted AUC is larger. Specifically, both the full model (all the clinical/descriptive variables and the GIV-fl marker) and the clinical model (only the clinical/descriptive variables) have much better unadjusted AUC values; however when adjusted for over-fitting, the LVI + GIV-fl model performs equally as well as the full model for both 2-year and 5-year PFS.

**Table 12.** Summary of AUC Values For Comparative Statistical Prediction Models

| Model | 2 Year PFS | | 5 year PFS | |
|---|---|---|---|---|
| | Unadjusted | Adjusted[a] | Unadjusted | Adjusted[a] |
| GIV-fl Only | 0.61 | 0.62 | 0.63 | 0.62 |
| LVI +GIV-fl | 0.69 | 0.68 | 0.74 | 0.69 |
| All Clinical Variables (those in Table 9) | 0.65 | 0.62 | 0.71 | 0.65 |
| Full Model (all predictors + GIV-fl) | 0.71 | 0.68 | 0.74 | 0.69 |
| [a]values after adjustment for bias estimated from 200 bootstrap resamples | | | | |

**Individual Level Predictive Ability**

Although AUC, sensitivity, specificity, Brier score, and other metrics are measures of statistical predictive ability, what is often missing is the integration of these measures with measures of prediction. The method described in Pepe (J. Epidemiol. 167:362, 2008), called the predictiveness curve, was used to combine these two measures of classification and risk prediction. The y-axis of the predictiveness curve is the predicted outcome for each patient (here the hazard ratio, on the log scale), and the x-axis is the cumulative risk from the classification. These individual-level probabilities for the GIV-fl marker were color-coded (red GIV-fl negative, blue GIV-fl positive).

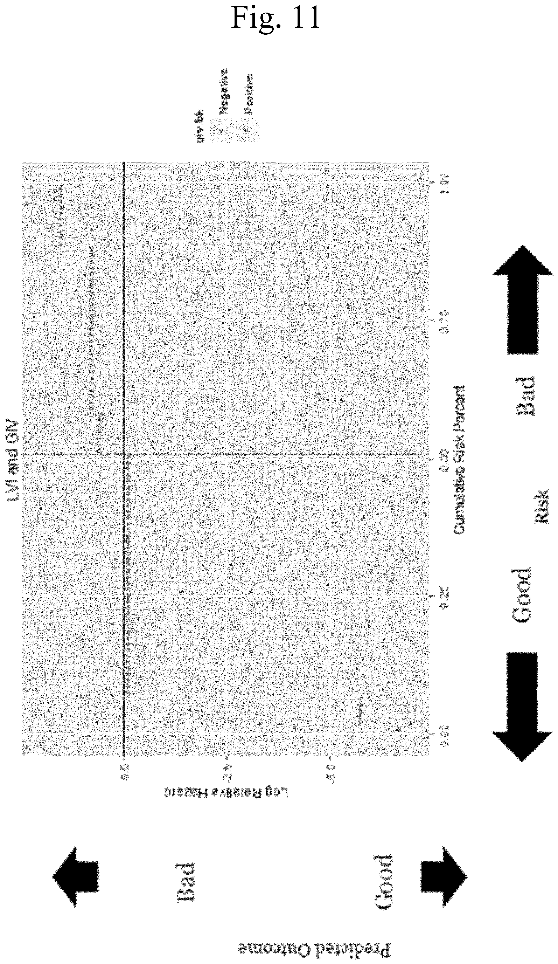

FIG. 11 shows the predictiveness plot for the stage IIa chemo-naïve CRC patients based on the GIV-fl and LVI statistical model. The hazard scale is on the log-scale for ease in interpretation, since the ratio scale is not symmetric; this shifts the scale of "significance" from one to zero, so that values above zero demonstrate a higher probability of progression, and values below zero show a lower probability of progression. For this statistical model, just below 50% of patients have a higher risk of progression, and the majority of those patients are GIV-fl positive. There are 5 patients at the low end of the risk/log hazard scale and yet are GIV-fl positive, and 6 patients with high risk of progression that are GIV-fl negative; but in general, GIV-fl positive patients have a high probability of progression, and are classified as high-risk, compared to GIV-fl negative patients who generally have low-risk and low probability of progression.

*Model Validity*

The ability of the model to predict in a subsample of patients for whom the prediction is intended was determined. The GIV-fl and LVI statistical prediction model was applied to patients from the cohort that underwent adjuvant chemotherapy (Tables 13 and 14). The expectation is that patients who were predicted to have benefitted from adjuvant chemotherapy actually did benefit. Because this is a subsampled population of the cohort, there is no external model of validity, so the focus was on the predictive value of the statistical model (and marker). The maximum of both sensitivity and specificity was used as the cut-point for the predicted probabilities when the fitted statistical prediction model is applied to the chemo-treated patient population. Each subject was therefore classified into high-risk and low-risk based on these cut-points, testing the assumption that high-risk patients (predicted probability $\geq 0.22$ for 2-year PFS and 0.24 for 5-year PFS) are likely to have high positive predictive value.

Tables 15 and 16 show the estimated benefit based on the predicted probabilities of the GIV-fl and LVI statistical model. The model predicted that 20/37 people should have benefited from chemotherapy at 2-years (Table 13). In fact, 15/20 did respond to chemotherapy (Positive Predictive Value (PPV) = 0.75). Furthermore, it was predicted that 25/37 people should have benefited from chemotherapy (Table 14) at 5-years. In fact, 20/25 did respond to chemotherapy (PPV = 0.80). These values indicate a moderately high level of predictive value for classifying patients who would benefit from adjuvant chemotherapy.

**Table 13.** Cross-Tabulation of Predicted Versus Observed 2-Year PFS

| 2-Year PFS (Predicted) | 2-Year PFS (Observed) | | |
| --- | --- | --- | --- |
| | No Recurrence | Recurrence | Total |
| Low Risk | 15 | 2 | 17 |
| High Risk | **15** | 5 | 20 |
| Total | 30 | 7 | 37 |

**Table 14.** Cross-Tabulation of Predicted Versus Observed 5-Year PFS

| 5-Year PFS (Predicted) | 5-Year PFS (Observed) | | |
| --- | --- | --- | --- |
| | No Recurrence | Recurrence | Total |
| Low Risk | 10 | 2 | 12 |
| High Risk | 20 | 5 | 25 |
| Total | 30 | 7 | 37 |

The positive predicted value of the full model (all predictors + GIV-fl labeling) was determined, and is shown in Tables 15 and 16. Tables 15 and 16 show the predictive utility for stage IIa (T3) patients using a statistical predictive model for GIV-fl and all clinicopathologic variables. As shown in Table 15, the model predicted that 12/37 people should have benefited from chemotherapy, 10/12 did respond to chemotherapy (PPV = 0.83). As shown in Table 16, the model predicted that 19/37 people should have benefited from chemotherapy, 16/19 did respond to chemotherapy (PPV = 0.84). The resulting values of 0.83 and 0.84 for 2-year and 5-year PFS, respectively, demonstrate high predictive utility.

**Table 15.** Cross-Tabulation of Predicted Versus Observed 2-Year PFS

| 2-Year PFS (Predicted) | 2-Year PFS (Observed) | | |
| --- | --- | --- | --- |
| | No Recurrence | Recurrence | Total |
| Low Risk | 20 | 5 | 25 |
| High Risk | **20** | 2 | 12 |
| Total | 30 | 7 | 37 |

**Table 16.** Cross-Tabulation of Predicted Versus Observed 5-Year PFS

| 5-Year PFS (Predicted) | 2-Year PFS (Observed) | | |
| --- | --- | --- | --- |
| | No Recurrence | Recurrence | Total |
| Low Risk | 14 | 4 | 18 |

(continued)

| 5-Year PFS (Predicted) | 2-Year PFS (Observed) | | |
|---|---|---|---|
| | No Recurrence | Recurrence | Total |
| High Risk | **16** | 3 | 19 |
| Total | 30 | 7 | 37 |

In summary, GIV-fl when used in conjunction with other clinicopathologic variables such as LVI, age, number of lymph nodes positive, sex, tumor differentiation, T stage, or on which side the tumor is present, can be used to classify chemo-naive stage II patients into high and low risk populations. Those classified using these methods as high risk can be assigned to receive chemotherapy, while those classified using these methods as low risk can be assigned to not receive chemotherapy.

In one example, the model consisted of GIV-fl status and LVI. One skilled in the art will appreciate that other clinicopathologic variables such as one or more of age, number of lymph nodes positive, sex, tumor differentiation, T stage, or on which side the tumor is present, can be included in the model. When this statistical prediction model was applied to a chemotreated subgroup, the model had high positive predicted value (PPV). This high PPV, along with statistical model performance (*e.g.*, AUC) gives evidence of prognostic utility.

### *Model validation using BioGrid 2*

Tables 17 and 18 provide data summaries of two cohorts (termed BioGrid 1 and BioGrid 2).

**Table 17 (BioGrid 1)**

| Variable | Categories | Recurrence | | No Recurrence | | Total | |
|---|---|---|---|---|---|---|---|
| | | N | % | N | % | N | % |
| MMR | Deficient | 3 | 6.2% | 36 | 25.5% | 39 | 20.6% |
| | Proficient | 45 | 93.8% | 105 | 74.5% | 150 | 79.4% |
| Chemo | No | 34 | 70.8% | 91 | 64.5% | 125 | 66.1% |
| | Yes | 14 | 29.2% | 50 | 35.5% | 64 | 33.9% |
| LVI | No | 26 | 54.2% | 99 | 70.2% | 125 | 66.1% |
| | Yes | 19 | 39.6% | 31 | 22% | 50 | 26.5% |
| | Not Reported/NA | 3 | 6.2% | 11 | 7.8% | 14 | 7.4% |
| Number of nodes | [2.12) | 15 | 31.2% | 35 | 24.8% | 50 | 26.5% |
| | [12,15) | 11 | 22.9% | 29 | 20.6% | 40 | 21.2% |
| | [15,21) | 10 | 20.8% | 38 | 27% | 48 | 25.4% |
| | [21,56] | 12 | 25% | 39 | 27.7% | 51 | 27% |
| Ln yield | <12 | 15 | 31.2% | 35 | 24.8% | 50 | 26.5% |
| | >=12 | 33 | 68.8% | 106 | 75.2% | 139 | 73.5% |
| Sex | F | 24 | 50% | 71 | 50.4% | 95 | 50.3% |
| | M | 24 | 50% | 70 | 49.6% | 94 | 49.7% |
| Tumor Diff. | Poor-Moderate | 13 | 27.1% | 28 | 19.9% | 41 | 21.7% |
| | Moderate-Well | 35 | 72.9% | 110 | 78% | 145 | 76.7% |
| | Not Reported / NA | 0 | 0% | 3 | 2.1% | 3 | 1.6% |
| T stage | 3 | 36 | 75% | 123 | 87.2% | 159 | 84.1% |
| | 4 | 12 | 25% | 18 | 12.8% | 30 | 15.9% |
| Site side | left | 25 | 52.1% | 68 | 48.2% | 93 | 49.2% |
| | right | 23 | 47.9% | 73 | 51.8% | 96 | 50.8% |
| rfs | | 19,7 | (16.5) | | 62(20) | 51.2 | (26.6) |
| Total | | 48 | 25.4% | 141 | 74.6% | 189 | |

### Table 18 (BioGrid 2)

| Variable | Categories | Recurrence N | Recurrence % | No Recurrence N | No Recurrence % | Total N | Total % |
|---|---|---|---|---|---|---|---|
| MMR | Deficient | 1 | 2.9% | 60 | 21.1% | 61 | 19.1% |
|  | Proficient | 33 | 97.1% | 225 | 78.9% | 258 | 80.9% |
| Chemo | No | 25 | 73.5% | 240 | 84.2% | 265 | 83.1% |
|  | Yes | 9 | 26.5% | 43 | 15.1% | 52 | 16.3% |
|  | Missing | 0 | 0% | 2 | 0.7% | 2 | 0.6% |
| LVI | No | 13 | 38.2% | 206 | 72.3% | 219 | 68.7% |
|  | Yes | 18 | 52.9% | 63 | 22.1% | 81 | 25.4% |
|  | Not Reported/NA | 3 | 8.8% | 16 | 5.6% | 19 | 6% |
| Number of nodes | [2,12) | 6 | 17.6% | 54 | 18.9% | 60 | 18.8% |
|  | [12,15) | 7 | 20.6% | 47 | 16.5% | 54 | 16.9% |
|  | [15,21) | 5 | 14.7% | 92 | 32.3% | 97 | 30.4% |
|  | [21,56] | 15 | 44.1% | 89 | 31.2% | 104 | 32.6% |
|  | Missing | 1 | 2.9% | 3 | 1.1% | 4 | 1.3% |
| Ln yield | <12 | 6 | 17.6% | 56 | 19.6% | 62 | 19.4% |
|  | >=12 | 28 | 82.4% | 229 | 80.4% | 257 | 80.6% |
| Sex | F | 11 | 32.4% | 126 | 44.2% | 137 | 42.9% |
|  | M | 23 | 67.6% | 159 | 55.8% | 182 | 57.1% |
| Tumor Diff. | Poor-Moderate | 6 | 17.6% | 65 | 22.8% | 71 | 22.3% |
|  | Moderates-Well | 28 | 82.4% | 216 | 75.8% | 244 | 76.5% |
|  | Not Reported/NA | 0 | 0% | 4 | 1.4% | 4 | 1.3% |
| T stage | 3 | 23 | 67.6% | 248 | 87% | 271 | 85% |
|  | 4 | 11 | 32.4%. | 37 | 13% | 48 | 15% |
| Site side | left | 20 | 58.8% | 140 | 49.1% | 160 | 50.2% |
|  | right | 14 | 41.2% | 145 | 50.9% | 159 | 49.8% |
| rfs |  | 21.2 | (15.9) | 52.7 | (37.3) | 49.4 (37) |  |
| Total |  | 34 | 10.7% | 285 | 89.3% | 319 |  |

### *Hazard ratio of BioGrid 1 and 2*

Tables 19, 20, and 21 present hazard ratios (HR) and the associated 95% confidence intervals and p values for various models.

Table 19 shows HR of high risk vs. low risk groups that are stratified by the risk models. All risk stratifications are based on the model developed in a training set developed in BioGrid 1.

### Table 19: Hazard Ratio for Risk Models

| Model | Chemo | B1 HR | B1 P value | B2 HR | B2 P value |
|---|---|---|---|---|---|
| GIV | Naïve | 2.37 (1.05, 5.32) | 0.037 | 2.36 (0.84, 6.61) | 0.104 |
| GIV | Treated | 2.48 (0.48, 12.78) | 0.279 | 2.26 (0.37, 13.58) | 0.375 |
| GIV + LVI | Naive | 3.74 (1.50, 9.32) | 0.005 | 7.83 (1.03, 59.54) | 0.047 |
| GIV + LVI | Treated | Inf | 0.999 | Inf | 0.999 |

Tables 20 and 21 show HR of each variable in both single variable models and the multivariable model including GIV and LVI.

Table 20 demonstrates the Hazard ratio for GIV and clinical variables in BioGrid 1, pMMR, T3, chemo-naïve population. Col. 3-5 show HR, CI, and P value for single variable models. Col. 6-8 show HR, CI, and P value for each variable in GIV+ LVI model.

**Table 20: Hazard Ratio for BioGrid 1**

|  | N | HR | 95% CI | p-value | HR2 | 95% CI2 | p-value 2 |
|---|---|---|---|---|---|---|---|
| 1vi | 83 | 2.54 | 1.12 to 5.74 | 0.03 | 2.09 | 0.9 to 4.86 | 0.09 |
| Giv | 89 | 2.37 | 1.05 to 5.32 | 0.04 | 2.49 | 1.07 to 5.78 | 0.03 |
| Site side | 89 | 1.30 | 0.6 to 2.8 | 0.51 |  |  |  |
| Sex | 89 | 1.46 | 0.67 to 3.15 | 0.33 |  |  |  |
| age | 89 | 1.01 | 0.97 to 1.06 | 0.11 |  |  |  |

Table 21 demonstrates the Hazard ratio for GIV and clinical variables in BioGrid 1, pMMR, T3, chemo-naïve population. Col. 3-5 show HR, CI, and P value for single variable models. Col. 6-8 show HR, CI, and P value for each variable in GIV+ LVI model.

**Table 21: Hazard Ratio for BioGrid 2**

|  | N | HR | 95% CI | p-value | HR2 | 95% CI2 | p-value 2 |
|---|---|---|---|---|---|---|---|
| lvi | 175 | 3.36 | 1.22 to 9.27 | 0.02 | 3.44 | 1.24 to 9.49 | 0.02 |
| Giv | 188 | 2.36 | 0.84 to 6.61 | 0.1 | 2.21 | 0.7 to 6.98 | 0.18 |
| Site side | 188 | 0.87 | 0.34 to 2.25 | 0.78 |  |  |  |
| Sex | 188 | 0.58 | 0.19 to 1.75 | 0.33 |  |  |  |
| age | 188 | 1.04 | 0.99 to 1.09 | 0.11 |  |  |  |

### AUC of BioGrid 2

Table 22 provides the area under the ROC curve (AUC) for various models. All models were developed in the training set developed in BioGrid 1 and then applied to BioGrid 2/pMMR/chemo-naive data.

**Table 22**

|  | T3 | T3+T4 |
|---|---|---|
| Clinical | 0.639 | 0.656 |
| GIV | 0.596 | 0.577 |
| LVI | 0.628 | 0.652 |
| GIV + LVI | 0.682 | 0.677 |
| GIV + Clinical | 0.697 | 0.695 |

### Risk prediction of BioGrid 1 and BioGrid 2

Tables 23-26 provide 2 by 2 tables of observed recurrence vs. 5 year risk prediction. All risk predictions are based on the model developed in the training set.

**Table 23: Risk prediction, GIV+LVI, BioGrid 1 / chemo-naive**

|  | No Recurrence | Recurrence |
|---|---|---|
| Low Risk | 34 | 5 |
| High Risk | 26 | 18 |

**Table 24: Risk prediction, GIV+LVI, BioGrid 1 / chemo-treated**

|  | No Recurrence | Recurrence |
|---|---|---|
| Low Risk | 10 | 0 |
| High Risk | 20 | 5 |

**Table 25: Risk prediction, GIV+LVI, BioGrid 2/chemo-naïve**

|  | No Recurrence | Recurrence |
|---|---|---|
| Low Risk | 62 | 1 |
| High Risk | 99 | 13 |

**Table 26: Risk prediction, GIV+LVI, BioGrid 3/chemo-treated**

|  | No Recurrence | Recurrence |
|---|---|---|
| Low Risk | 12 | 0 |
| High Risk | 11 | 5 |

Table 27 provides the PPV and miscalculation rates. PPV = Number of high risk and no recurrence / number of high risk.

**Table 27**

| Model | Cohort | Chemo | PPV | Misclassification |
|---|---|---|---|---|
| GIV + LVI | BioGrid 1 | Naïve | 0.59 | 0.37 |
| GIV + LVI | BioGrid 1 | Treated | 0.80 | 0.57 |
| GIV + LVI | BioGrid 2 | Naive | 0.88 | 0.57 |
| GIV + LVI | BioGrid 2 | Treated | 0.69 | 0.39 |

***Kaplan-Meier Curves***

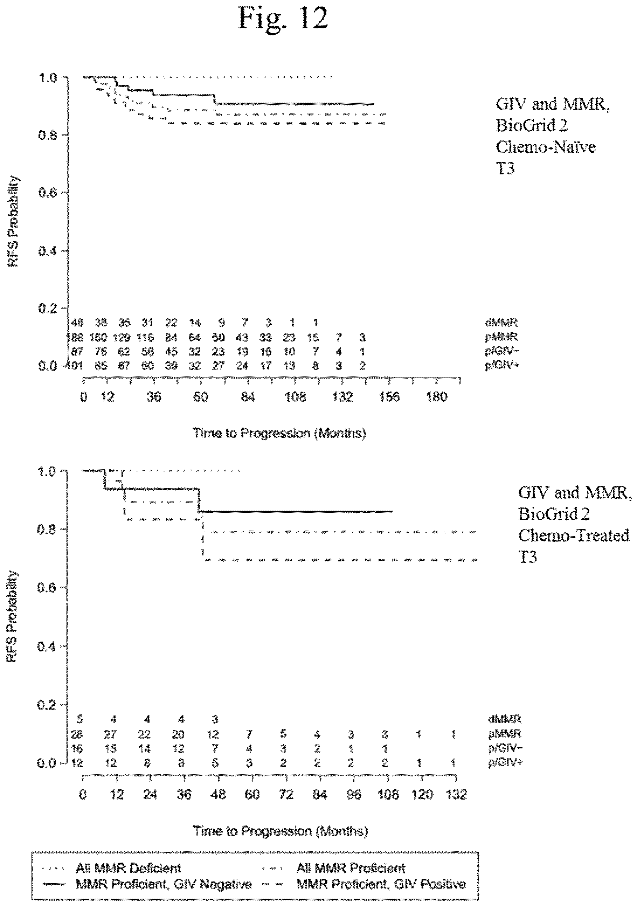

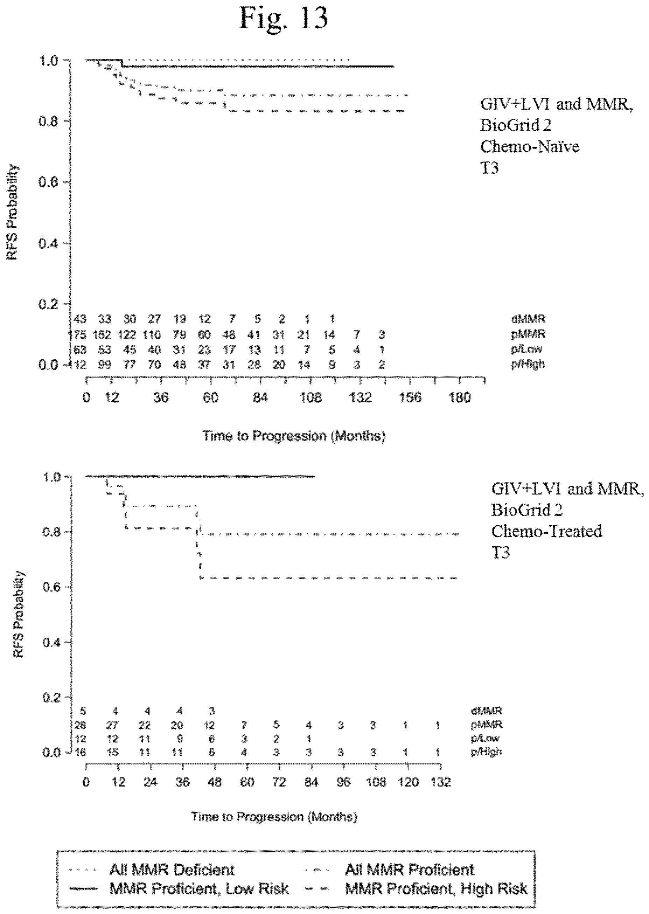

Figures 12-13 provide Kaplan-Meier curves for various marker/LVI combinations from BioGrid 2. All risk stratifications are based on the model developed in the training set above.

As can be seen from the foregoing figures and tables, GIV has been validated across two independent cohorts as a reproducibly prognostic and predictive biomarker in Stage II CRC.

In view of the many possible embodiments to which the principles of the disclosure may be applied, it should be recognized that the illustrated examples are only examples of the disclosure and should not be taken as limiting the scope of the invention. Rather, the scope of the invention is defined by the following claims. We therefore claim as our invention all that comes within the scope of these claims.

## Claims

1. A method for analyzing a stage II mis-match repair proficient (pMMR) colorectal cancer (CRC) sample obtained from a subject, comprising:

   contacting a sample comprising the stage II pMMR CRC with a G-alpha interacting vesicle associated protein-full length (GIV-fl) protein specific binding agent, wherein the GIV-fl protein specific binding agent comprises a GIV-fl antibody, or contacting RNA isolated from a sample with a nucleic acid probe specific for G-alpha interacting vesicle associated protein-full length (GIV-fl) mRNA;

scoring expression of GIV-fl protein or mRNA in the sample to determine a GIV-fl status of the sample; determining the lymphovascular invasion (LVI) status of the CRC in the subject with a specific-binding agent that permits for a determination of LVI wherein the specific-binding agent that permits for a determination of LVI comprises an antibody specific for CD34 or lymphatic endothelium; and analyzing the sample based on the GIV-fl status and LVI status,

wherein the method further comprises determining the mis-match repair (MMR) status of the sample with a the mismatch repair protein specific-binding agent, wherein the mis-match repair protein specific-binding agent comprises one or more antibodies specific for mutL homolog 1 (MLH1); postmeiotic segregation increased 2 (PMS2); MutS protein homolog 2 Msh2 (MSH2), and/or MutS protein homolog 6 (MSH6); and wherein the method is a method of predicting the likely progression free survival (PFS) of the subject, wherein more LVI and high GIV-fl levels are associated with a higher probability of recurrence.

2. The method of claim 1, further comprising:

determining one or more characteristics of the subject, wherein the one or more characteristics include age of the subject at diagnosis, number of lymph nodes that are positive for CRC; sex of the subject, state of tumor differentiation, T stage of the cancer; and on which side the colon cancer was present; and analyzing the sample based on the GIV-fl status, LVI status, and the one or more characteristics of the subject.

3. The method of claim 1 or 2, further comprising:

inputting the GIV-fl status, LVI status, and one or more of the subject's characteristics into a computer; and generating an output from the computer, thereby analyzing the sample.

4. The method of any of claims 1 to 3, wherein scoring expression of GIV-fl protein comprises:

a. determining an extent of positive GIV-fl staining for the sample on a scale of 0 to 3, wherein extent of positive staining is assigned 0 if 0% to 10% of the total area of the sample is stained positive, wherein extent of positive staining is assigned 1 if 11 %-35% of the total area of the sample is stained positive, wherein extent of positive staining is assigned 2 if 36%-50% of the total area of the sample is stained positive, and wherein extent of positive staining is assigned 3 if 51%-100% of the total area of the sample is stained positive;
b. determining a GIV-fl intensity of staining for the sample on a scale of 0 (negative), 1 (weak), 2 (moderate), to 3 (strong); GIV-fl Extent and Intensity Score
c. summing the extent of positive GIV-fl staining and the GIV-fl intensity of staining, thereby generating a GIV-fl score value from 0 to 6; and
d. determining that the sample is GIV-fl negative if the GIV-fl score value is 0-2 or determining that the sample is GIV-fl positive if the GIV-fl score value is 3-6.

5. The method of claim any of claims 1 to 3, wherein scoring expression of GIV-fl protein comprises:

a. determining if a total area of GIV-fl staining for the sample is greater than 10% or determining if GIV-fl staining intensity is 3+ (strong); and
b. determining that the sample is GIV-fl positive if the total area of GIV-fl staining for the sample is greater than 10% with any staining intensity or if the GIV-fl staining intensity is 3+ with any percent; or determining that the sample is GIV-fl negative if the total area of GIV-fl staining with a staining intensity of 0, 1+, or 2+ for the sample is less than 10%.

6. The method of any of claims 1 to 7, wherein the method is a method of distinguishing between a subject who is likely to respond to treatment with chemotherapy or biotherapy from a subject who is not likely to respond to treatment with chemotherapy or biotherapy,

- particularly further comprising selecting the subject for treatment with the chemotherapy or biotherapy if the subject is identified as a subject who is likely to respond to treatment with chemotherapy or biotherapy; and/or
- particularly wherein the chemotherapy or biotherapy comprises 5-fluouracil, leucovorin, panitumumab (VECTIBIX®), cetuximab (ERBITUX®), bevacizumab (AVASTIN®), ziv-aflibercept (ZALTRAP®), irinotecan (CAMPTOSAR®), oxaliplatin (ELOXATIN®), or combinations thereof

7. The method of any of claims 1 to 6,

a) wherein the subject is a chemo-naïve subject; and/or
b) wherein the sample comprises a surgical resection specimen, tissue biopsy or fine needle aspirate, particularly wherein the tissue biopsy comprises a tissue section; and/or
c) wherein the sample is a fixed sample; and/or
d) wherein the sample is a formalin fixed, paraffin embedded (FFPE) sample; and/or
e) wherein the sample is a stage IIa pMMR CRC sample or a stage IIb pMMR CRC sample; and/or
f) wherein contacting the sample with the GIV-fl protein specific binding agent is performed with an automated tissue stainer; and/or
g) wherein scoring expression of GIV-fl protein comprises visual inspection or image analysis of a corresponding digital image, particularly wherein the visual inspection is performed utilizing light microscopy; and/or
h) wherein scoring expression of GIV-fl protein comprises visual inspection of a total area of the sample; and/or
i) wherein scoring expression of GIV-fl protein in the sample comprises direct or indirect detection of binding of the GIV-fl protein specific binding agent to the sample; and/or
j) wherein one or more steps are performed by a suitably-programmed computer.

8. A kit for performing the method according to claim 1 comprising:
a GIV-fl protein specific-binding agent comprising a GIV-fl antibody, or a nucleic acid probe specific for the GIV-fl mRNA and at least one pair of primers specific for GIV-fl gene sequence;

a specific-binding agent that permits for a determination of LVI comprising an antibody specific for CD34 or lymphatic endothelium;
a mis-match repair protein specific-binding agent comprising one or more antibodies specific for mutL homolog 1 (MLH1); postmeiotic segregation increased 2 (PMS2); MutS protein homolog 2 Msh2 (MSH2), and/or MutS protein homolog 6 (MSH6); and optionally
microscope slides;
labeled secondary antibodies; and
buffers for IHC.

**Patentansprüche**

1. Verfahren zum Analysieren einer Probe von Stadium II mismatch-Reparatur-kompetentem (pMMR; *mismatch-repair proficient*) Dickdarmkrebs (CRC), die von einem Subjekt erhalten wird, umfassend:

Inkontaktbringen einer Probe, die den Stadium II pMMR CRC umfasst, mit einem G-alpha interagierenden Vesikel assoziierten Protein-Volllängen (GIV-fl) Protein-spezifischen Binde-Agens, wobei das GIV-fl Proteinspezifische Binde-Agens einen GIV-fl-Antikörper umfasst, oder Inkontaktbringen von RNA, die aus einer Probe isoliert ist mit einer Nukleinsäuresonde, die spezifisch ist für G-alpha interagierende Vesikelassoziierte Protein-Volllängen- (GIV-fl) mRNA;
Bewerten der Expression von GIV-fl-Protein oder mRNA in der Probe, um einen GIV-fl-Status der Probe zu bestimmen;
Bestimmen des lymphovaskulären Invasions- (LVI) Status des CRC in dem Subjekt mit einem spezifisch-bindenden Agens, das die Bestimmung von LVI erlaubt, wobei das spezifisch-bindende Agens, das die Bestimmung von LVI erlaubt, einen Antikörper umfasst, der spezifisch ist für CD34 oder lymphatisches Endothel; und Analysieren der Probe basierend auf dem GIV-fl-Status und LVI-Status, wobei das Verfahren weiterhin umfasst das Bestimmen des mismatch-Reparatur (MMR)-Status der Probe mit einem mismatch-Reparatur-Protein-spezifischen Binde-Agens, wobei das mismatch-Reparatur-Proteinspezifische Binde-Agens mehrere Antikörper umfasst, die spezifisch sind für mutL Homolog 1 (MLH1); *postmeiotic segregation increased 2* (PMS2); MutS Protein Homolog 2 Msh2 (MSH2) und/oder MutS Protein Homolog 6 (MSH6); und wobei das Verfahren ein Verfahren ist zum Voraussagen des wahrscheinlichen progressionsfreien Überlebens (PFS) des Subjekts, wobei mehr LVI und hohe GIV-fl-Spiegel mit einer höheren Wahrscheinlichkeit des Wiederauftretens assoziiert sind.

2. Verfahren nach Anspruch 1, weiterhin umfassend:

Bestimmen von einem oder mehreren Charakteristika des Subjekts, wobei die ein oder mehreren Charakteristika das Alter des Subjekts bei der Diagnose, die Anzahl der Lymphknoten, die positiv für CRC sind, das Geschlecht des Subjekts, den Zustand der Tumordifferenzierung, das T-Stadium des Krebses und auf welcher Seite der Darmkrebs vorhanden war, beinhalten; und

Analysieren der Probe basierend auf dem GIV-fl-Status, dem LVI-Status und den ein oder mehreren Charakteristika des Subjekts.

3. Verfahren nach Anspruch 1 oder 2, weiterhin umfassend:

Eingeben des GIV-fl-Status, des LVI-Status und der ein oder mehreren Charakteristika des Subjekts in einen Computer; und
Generieren einer Ausgabe aus dem Computer, dadurch Analysieren der Probe.

4. Verfahren nach einem beliebigen der Ansprüche 1 bis 3, wobei das Bewerten der Expression von GIV-fl-Protein umfasst:

a. Bestimmen eines Ausmaßes von positiver GIV-fl-Färbung für die Probe auf einer Skala von 0 bis 3, wobei dem Ausmaß der positiven Färbung 0 zugewiesen wird, wenn 0% bis 10% der gesamten Fläche der Probe positiv gefärbt ist, wobei dem Ausmaß der positiven Färbung 1 zugewiesen wird, wenn 11%-35% der gesamten Fläche der Probe positiv gefärbt ist, wobei dem Ausmaß der positiven Färbung 2 zugewiesen wird, wenn 36% bis 50% der gesamten Fläche der Probe positiv gefärbt ist und wobei dem Ausmaß der positiven Färbung 3 zugewiesen wird, wenn 51% bis 100% der gesamten Fläche der Probe positiv gefärbt ist;
b. Bestimmen einer GIV-fl-Intensität der Färbung für die Probe auf einer Skala von 0 (negativ), 1 (schwach), 2 (moderat) bis 3 (stark); GIV-fl-Ausmaß und -Intensitäts-Punktezahl (*score*)
c. Aufsummieren des Ausmaßes der positiven GIV-fl-Färbung und der GIV-fl-Intensität der Färbung, dadurch Generieren eines GIV-fl-Punktezahlwerts von 0 bis 6; und
d. Bestimmen, dass die Probe GIV-fl-negativ ist, wenn der GIV-fl-Punktezahlwert 0-2 ist oder Bestimmen, dass die Probe GIV-fl-positiv ist, wenn der GIV-fl-Punktezahlwert 3-6 ist.

5. Verfahren nach einem beliebigen der Ansprüche 1 bis 3, wobei das Bewerten der Expression von GIV-fl-Protein umfasst:

a. Bestimmen, ob eine gesamte Fläche der GIV-fl-Färbung für die Probe größer als 10% ist oder Bestimmen, ob die GIV-fl-Färbungsintensität 3+ (stark) ist; und
b. Bestimmen, dass die Probe GIV-fl-positiv ist, falls die gesamte Fläche der GIV-fl-Färbung für die Probe größer als 10% ist mit irgendeiner beliebigen Färbeintensität oder ob die GIV-fl-Färbeintensität 3+ ist mit einem beliebigen Prozentwert; oder Bestimmen, dass die Probe GIV-fl-negativ ist, wenn die gesamte Fläche der GIV-fl-Färbung mit einer Färbeintensität von 0, 1+ oder 2+ für die Probe weniger als 10% ist.

6. Verfahren nach einem beliebigen der Ansprüche 1 bis 7, wobei das Verfahren ein Verfahren ist zur Unterscheidung zwischen einem Subjekt, für das es wahrscheinlich ist, dass es auf eine Behandlung mit Chemotherapie oder Biotherapie anspricht von einem Subjekt, für das es nicht wahrscheinlich ist, dass es auf eine Behandlung mit Chemotherapie oder Biotherapie anspricht,

- insbesondere ferner umfassend Auswählen des Subjekts für die Behandlung mit der Chemotherapie oder Biotherapie, falls das Subjekt als ein Subjekt identifiziert wird, für das es wahrscheinlich ist, dass es auf die Behandlung mit Chemotherapie oder Biotherapie anspricht; und/oder
- insbesondere wobei die Chemotherapie oder Biotherapie 5-Fluouracil, Leucovorin, Panitumumab (Vectibix®), Cetuximab (Erbitux®), Bevacizumab (Avastin®), Ziv-aflibercept (Zaltrap®), Irinotecan (Camptosar®), Oxaliplatin (Eloxatin®), oder Kombinationen davon umfasst.

7. Verfahren nach einem beliebigen der Ansprüche 1 bis 6,

a) wobei das Subjekt ein chemo-naives Subjekt ist; und/oder
b) wobei die Probe ein Präparat einer chirurgischen Entfernung, eine Gewebebiopsie oder ein Aspirat einer feinen Nadel ist, insbesondere wobei die Gewebebiopsie einen Gewebeschnitt umfasst; und/oder
c) wobei die Probe eine fixierte Probe ist; und/oder
d) wobei die Probe eine Formalin-fixierte, Paraffin-eingebettete (FFPE) -Probe ist; und/oder
e) wobei die Probe eine Stadium IIa pMMR CRC-Probe oder eine Stadium IIb pMMR CRC-Probe ist; und/oder

f) wobei das Inkontaktbringen der Probe mit dem GIV-fl-Protein-spezifischen Binde-Agens mit einem automatischen Gewebefärber durchgeführt wird; und/oder

g) wobei das Bewerten der Expression des GIV-fl-Proteins die visuelle Untersuchung oder Bildanalyse eines entsprechenden digitalen Bildes umfasst, insbesondere wobei die visuelle Untersuchung unter Verwendung von Lichtmikroskopie durchgeführt wird; und/oder

h) wobei das Bewerten der Expression des GIV-fl-Proteins die visuelle Untersuchung der gesamten Fläche der Probe umfasst; und/oder

i) wobei das Bewerten der Expression des GIV-fl-Proteins in einer Probe die direkte oder indirekte Detektion des Bindens des GIV-fl-Protein-spezifischen Binde-Agens an die Probe umfasst; und/oder

j) wobei ein oder mehrere Schritte durch einen geeignet programmierten Computer durchgeführt werden.

8. Kit zum Durchführen des Verfahrens gemäß Anspruch 1, umfassend:

ein GIV-fl-Protein-spezifisches Binde-Agens umfassend einen GIV-fl-Antikörper, oder eine Nukleinsäuresonde, die spezifisch für die GIV-flmRNA ist und mindestens ein Primerpaar, das spezifisch für die GIV-fl-Gensequenz ist;

ein spezifisch-bindendes Agens, das die Bestimmung von LVI erlaubt, umfassend einen Antikörper, der spezifisch ist für CD34 oder lymphatisches Endothel;

ein *mismatch*-Reparatur-Protein-spezifisches Binde-Agens umfassend ein oder mehrere Antikörper, die spezifisch sind für mutL Homolog 1 (MLH1); *postmeiotic segregation increased 2* (PMS2); MutS Protein Homolog 2 Msh2 (MSH2) und/oder MutS Protein Homolog 6 (MSH6); und optional Mikroskop-Objektträger;

markierte sekundäre Antikörper; und

Puffer für IHC.

**Revendications**

1. Procédé pour l'analyse d'un échantillon de cancer colorectal (CRC) de stade II compétent pour la réparation des mésappariements (pMMR) obtenu à partir d'un sujet, comprenant :

la mise en contact d'un échantillon comprenant le CRC pMMR de stade II avec un agent de liaison spécifique d'une protéine pleine longueur de protéine associée au vésicule interagissant avec G-alpha (GIV-fl), dans lequel l'agent de liaison spécifique de la protéine GIV-fl comprend un anticorps anti-GIV-fl, ou la mise en contact d'un ARN isolé à partir d'un échantillon avec une sonde d'acide nucléique spécifique de l'ARNm de protéine pleine longueur associée au vésicule interagissant avec G-alpha (GIV-fl);

l'évaluation de l'expression de la protéine ou de l'ARNm GIV-fl dans l'échantillon pour déterminer un statut GIV-fl de l'échantillon ;

la détermination du statut d'invasion lymphovasculaire (LVI) du CRC dans le sujet avec un agent de liaison spécifique qui permet une détermination de la LVI dans lequel l'agent de liaison spécifique qui permet une détermination de la LVI comprend un anticorps spécifique de CD34 ou de l'endothélium lymphatique ; et

l'analyse de l'échantillon sur la base du statut GIV-fl et du statut LVI,

dans lequel le procédé comprend en outre la détermination du statut de réparation des mésappariements (MMR) de l'échantillon avec un agent de liaison spécifique de protéine de réparation des mésappariements,

dans lequel l'agent de liaison spécifique de protéine de réparation des mésappariements comprend un ou plusieurs anticorps spécifiques de l'homologue 1 de mutL (MLH1); la protéine 2 de ségrégation post-méiotique plus importante (PMS2) ; l'homologue 2 de la protéine MutS Msh2 (MSH2), et/ou l'homologue 6 de la protéine MutS (MSH6) ; et dans lequel le procédé est un procédé de prédiction de la survie sans progression probable (PFS) du sujet, dans lequel davantage de LVI et des niveaux élevés de GIV-fl sont associés à une probabilité plus élevée de récidive.

2. Procédé selon la revendication 1, comprenant en outre :

la détermination d'une ou de plusieurs caractéristiques du sujet, dans laquelle la ou les caractéristiques comprennent l'âge du sujet au moment du diagnostic, le nombre de ganglions lymphatiques qui sont positifs pour le CRC ; le sexe du sujet, l'état de différenciation tumorale, le stade T du cancer ; et le côté sur lequel le cancer du côlon était présent ; et

l'analyse de l'échantillon sur la base du statut GIV-fl, du statut LVI, et de la ou des caractéristiques du sujet.

**3.** Procédé selon la revendication 1 ou 2, comprenant en outre :

l'entrée du statut GIV-fl, du statut LVI et de la ou des caractéristiques du sujet dans un ordinateur ; et
la génération d'une sortie de l'ordinateur, analysant ainsi l'échantillon.

**4.** Procédé selon l'une quelconque des revendications 1 à 3, dans lequel l'évaluation de l'expression de la protéine GIV-fl comprend :

a. la détermination d'une étendue de la coloration positive pour la GIV-fl pour l'échantillon sur une échelle de 0 à 3, dans laquelle on attribue 0 à une étendue de coloration positive si 0 % à 10 % de la surface totale de l'échantillon est colorée positive, dans laquelle on attribue 1 à une étendue de coloration positive si 11 % à 35 % de la surface totale de l'échantillon est colorée positive, dans laquelle on attribue 2 à une étendue de coloration positive si 36 % à 50 % de la surface totale de l'échantillon est colorée positive, et dans laquelle on attribue 3 à une étendue de coloration positive si 51 % à 100 % de la surface totale de l'échantillon est colorée positive ;
b. la détermination qu'une intensité de coloration de GIV-fl pour l'échantillon sur une échelle de 0 (négative), 1 (faible), 2 (modérée), à 3 (forte) ; d'un score d'étendue et d'intensité de GIV-fl
c. la sommation de l'étendue de la coloration positive pour la GIV-fl et de l'intensité de la coloration de GIV-fl, générant ainsi une valeur de score GIV-fl comprise entre 0 et 6 ; et
d. la détermination que l'échantillon est négatif pour la GIV-fl si la valeur du score GIV-fl est comprise entre 0 et 2, ou la détermination que l'échantillon est positif pour la GIV-fl si la valeur du score GIV-fl est comprise entre 3 et 6.

**5.** Procédé selon l'une quelconque des revendications 1 à 3, dans lequel l'évaluation de l'expression de la protéine GIV-fl comprend :

a. la détermination si la surface totale de la coloration de GIV-fl pour l'échantillon est supérieure à 10 % ou la détermination si l'intensité de la coloration de GIV-fl est de 3+ (forte) ; et
b. la détermination que l'échantillon est positif pour la GIV-fl si la surface totale de la coloration de GIV-fl pour l'échantillon est supérieure à 10 % avec n'importe quelle intensité de coloration ou si l'intensité de la coloration de GIV-fl est de 3+ avec un pourcentage quelconque ; ou la détermination que l'échantillon est négatif pour la GIV-fl si la surface totale de la coloration de GIV-fl avec une intensité de coloration de 0, 1+, ou 2+ pour l'échantillon est inférieure à 10 %.

**6.** Procédé selon l'une quelconque des revendications 1 à 7, dans lequel le procédé est un procédé permettant de distinguer un sujet susceptible de répondre à un traitement par chimiothérapie ou biothérapie d'un sujet non susceptible de répondre à un traitement par chimiothérapie ou biothérapie,

- en particulier, comprenant en outre la sélection du sujet pour un traitement par la chimiothérapie ou la biothérapie si le sujet est identifié en tant que sujet susceptible de répondre à un traitement par chimiothérapie ou biothérapie ; et/ou
- en particulier, dans lequel la chimiothérapie ou la biothérapie comprend le 5-fluouracil, la leucovorine, le panitumumab (VECTIBIX®), le cétuximab (ERBITUX®), le bévacizumab (AVASTIN®), le ziv-aflibercept (ZALTRAP®), l'irinotécan (CAMPTOSAR®), l'oxaliplatine (ELOXATIN®) ou leurs combinaisons.

**7.** Procédé selon l'une quelconque des revendications 1 à 6,

a) dans lequel le sujet est un sujet chimio-naïf ; et/ou
b) dans lequel l'échantillon comprend un spécimen de résection chirurgicale, une biopsie tissulaire ou un aspirat par aiguille fine, en particulier dans lequel la biopsie tissulaire comprend une coupe tissulaire ; et/ou
c) dans lequel l'échantillon est un échantillon fixé; et/ou
d) dans lequel l'échantillon est un échantillon fixé au formol et inclus dans de la paraffine (FFPE) ; et/ou
e) dans lequel l'échantillon est un échantillon de CRC de stade IIa pMMR ou un échantillon de CRC de stade IIb pMMR ; et/ou
f) dans lequel la mise en contact de l'échantillon avec l'agent de liaison spécifique de la protéine GIV-fl est effectuée avec un appareil de coloration tissulaire automatisé ; et/ou
g) dans lequel l'évaluation de l'expression de la protéine GIV-fl comprend une inspection visuelle ou une analyse d'image d'une image numérique correspondante, en particulier dans lequel l'inspection visuelle est effectuée à l'aide d'une microscopie optique ; et/ou

h) dans lequel l'évaluation de l'expression de la protéine GIV-fl comprend une inspection visuelle d'une surface totale de l'échantillon ; et/ou

i) dans lequel l'évaluation de l'expression de la protéine GIV-fl dans l'échantillon comprend la détection directe ou indirecte de la liaison de l'agent de liaison spécifique de la protéine GIV-fl à l'échantillon ; et/ou

j) dans lequel une ou plusieurs étapes sont effectuées par un ordinateur programmé de manière appropriée.

8. Kit pour la mise en oeuvre du procédé selon la revendication 1 comprenant :

un agent de liaison spécifique de la protéine GIV-fl comprenant un anticorps anti-GIV-fl, ou une sonde d'acide nucléique spécifique de l'ARNm de GIV-fl et au moins une paire d'amorces spécifiques de la séquence génétique de GIV-fl ;

un agent de liaison spécifique qui permet une détermination de la LVI comprenant un anticorps spécifique de CD34 ou de l'endothélium lymphatique ;

un agent de liaison spécifique de protéine de réparation des mésappariements comprenant un ou plusieurs anticorps spécifiques de l'homologue 1 de mutL (MLH1); la protéine 2 de ségrégation post-méiotique plus importante (PMS2) ; l'homologue 2 de la protéine MutS Msh2 (MSH2), et/ou l'homologue 6 de la protéine MutS (MSH6) ; et éventuellement

des lames de microscope ;

des anticorps secondaires marqués ; et

des tampons pour IHC.

Fig. 1

Fig. 2A.  Normal colon          IBL-GIV                    SP173-GIV

Fig. 2B.  Colon cancer          IBL-GIV                    SP173-GIV

IBL-GIV                    SP173-GIV

# Fig. 2C

# Fig. 3

**A)**

| 5.0 µg/ml | 2.5 µg/ml | 1.0 µg/ml |

**B)**

| 5.0 µg/ml | 2.5 µg/ml | 1.0 µg/ml |

**C)**

| 5.0 µg/ml | 2.5 µg/ml | 1.0 µg/ml |

# Fig. 4

A) 10.0 µg/ml    5.0 µg/ml

B) 10.0 µg/ml    5.0 µg/ml

Fig. 5A.  GIV negative staining in tumor cells

Fig. 5B.  GIV positive (1+) cytoplasmic staining in tumor cells

Fig. 5C. GIV positive (2+) cytoplasmic and nuclear staining in tumor cells

Fig. 5D. GIV positive (3+) cytoplasmic and nuclear staining in tumor cells

# Fig. 6

Product-Limit Survival Estimates
With Number of Subjects at Risk and 95% Hall-Wellner Bands

# Fig. 7A

**Product-Limit Survival Estimates**
With Number of Subjects at Risk and 95% Hall-Wellner Bands

| Percent Positive (N) | HR (95% CI) |
|---|---|
| 35.29 % ( 36 ) | 2.03 ( 1.02 , 4.07 ) |

# Fig. 7B

**Product-Limit Survival Estimates**
With Number of Subjects at Risk and 95% Hall-Wellner Bands

| Percent Positive (N) | HR (95% CI) |
|---|---|
| 44.12 % ( 45 ) | 1.75 ( 0.87 , 3.52 ) |

# Fig. 8

**Product-Limit Survival Estimates**
With Number of Subjects at Risk

# Fig. 9

# Fig. 10

A. 2 Year Progression Free Survival

B. 5 Year Progression Free Survival

# Fig. 11

# Fig. 12

GIV and MMR,
BioGrid 2
Chemo-Naïve
T3

| | | | | | | | | | | | | | |
|---|---|---|---|---|---|---|---|---|---|---|---|---|---|
| 48 | 38 | 35 | 31 | 22 | 14 | 9 | 7 | 3 | 1 | 1 | | | dMMR |
| 188 | 160 | 129 | 116 | 84 | 64 | 50 | 43 | 33 | 23 | 15 | 7 | 3 | pMMR |
| 87 | 75 | 62 | 56 | 45 | 32 | 23 | 19 | 16 | 10 | 7 | 4 | 1 | p/GIV− |
| 101 | 85 | 67 | 60 | 39 | 32 | 27 | 24 | 17 | 13 | 8 | 3 | 2 | p/GIV+ |

Time to Progression (Months)

GIV and MMR,
BioGrid 2
Chemo-Treated
T3

| | | | | | | | | | | | | dMMR |
|---|---|---|---|---|---|---|---|---|---|---|---|---|
| 5 | 4 | 4 | 4 | 3 | | | | | | | | dMMR |
| 28 | 27 | 22 | 20 | 12 | 7 | 5 | 4 | 3 | 3 | 1 | 1 | pMMR |
| 16 | 15 | 14 | 12 | 7 | 4 | 3 | 2 | 1 | 1 | | | p/GIV− |
| 12 | 12 | 8 | 8 | 5 | 3 | 2 | 2 | 2 | 2 | 1 | 1 | p/GIV+ |

Time to Progression (Months)

···· All MMR Deficient    – · – All MMR Proficient
—— MMR Proficient, GIV Negative   – – MMR Proficient, GIV Positive

# Fig. 13

GIV+LVI and MMR,
BioGrid 2
Chemo-Naïve
T3

RFS Probability — Time to Progression (Months)

| dMMR | 43 | 33 | 30 | 27 | 19 | 12 | 7 | 5 | 2 | 1 | 1 | | |
| pMMR | 175 | 152 | 122 | 110 | 79 | 60 | 48 | 41 | 31 | 21 | 14 | 7 | 3 |
| p/Low | 63 | 53 | 45 | 40 | 31 | 23 | 17 | 13 | 11 | 7 | 5 | 4 | 1 |
| p/High | 112 | 99 | 77 | 70 | 48 | 37 | 31 | 28 | 20 | 14 | 9 | 3 | 2 |

GIV+LVI and MMR,
BioGrid 2
Chemo-Treated
T3

RFS Probability — Time to Progression (Months)

| dMMR | 5 | 4 | 4 | 4 | 3 | | | | | | | |
| pMMR | 28 | 27 | 22 | 20 | 12 | 7 | 5 | 4 | 3 | 3 | 1 | 1 |
| p/Low | 12 | 12 | 11 | 9 | 6 | 3 | 2 | 1 | | | | |
| p/High | 16 | 15 | 11 | 11 | 6 | 4 | 3 | 3 | 3 | 3 | 1 | 1 |

Legend:
- ···· All MMR Deficient
- ─·─ All MMR Proficient
- ── MMR Proficient, Low Risk
- ── ── MMR Proficient, High Risk

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4745055 A
- US 4444487 A
- WO 8803565 A
- EP 256654 A
- EP 120694 A
- EP 125023 A
- US 5210015 A
- US 8625930 B
- US 8609023 B
- US 8290236 B

- US 8537181 B
- US 8515683 B
- US 8428887 B
- US 20120171668 A
- US 6248535 B
- US 8586311 B
- US 20030228689 A
- US 6283761 B
- US 61940705 B

### Non-patent literature cited in the description

- **MULLER et al.** *Nature,* 2001, vol. 410 (6824), 50-6
- **LARUE ; BELLACOSA.** *Oncogene,* 2005, vol. 24 (50), 7443-54
- **QIAO et al.** *Cancer Res,* 2007, vol. 67 (11), 5293-9
- **FIDLER.** *Nat Rev Cancer,* 2003, vol. 3 (6), 453-8
- **QIAO et al.** *Cell Cycle,* 2008, vol. 7 (19), 2991-6
- **GARCIA-MARCOS et al.** *FASEB J,* 2011, vol. 25 (2), 590-9
- **CHEN et al.** *J. Mol. Biol.,* 1999, vol. 293, 865-881
- **FAOULKNER et al.** *Nature,* 1982, vol. 298, 286
- **MORRISON.** *J. Immunol.,* 1979, vol. 123, 793
- **MORRISON et al.** *Ann Rev. Immunol.,* 1984, vol. 2, 239
- **GARCIA-MARCOS et al.** *Proc Natl Acad Sci USA,* 2009, vol. 106, 3178-83
- **ENOMOTO et al.** *Dev Cell,* 2005, vol. 9, 389-402
- **GHOSH et al.** *J Cell Biol,* 2008, vol. 182, 381-93
- **JIANG et al.** *Cancer Res,* 2008, vol. 68 (5), 1310-8
- **KITAMURA et al.** *Nat Cell Biol,* 2008, vol. 10 (3), 329-37
- **ANAI et al.** *J Biol Chem,* 2005, vol. 280, 18525-35
- **GHOSH et al.** *Mol. Biol. Cell,* 2010, vol. 21, 2338-54
- **SADHU et al.** *J. Biosci.,* 1984, vol. 6, 817-821

- **SAMBROOK et al.** Molecular Cloning. Cold Spring Harbor Laboratory, 1989
- **LANDEGENT et al.** *Hum. Genet.,* 1987, vol. 77, 366-370
- **LICHTER et al.** *Hum. Genet.,* 1988, vol. 80, 224-234
- **PINKEL et al.** *Proc. Natl. Acad. Sci. USA,* 1988, vol. 85, 9138-9142
- **HOLLAND et al.** *Proc. Natl. Acad. Sci.,* 1991, vol. 88, 7276-7280
- **HARLOW ; LANE.** Using Antibodies: A Laboratory Manual. CSHL, 1999
- **GARCIA-MARCOS et al.** *Proc Natl Acad Sci USA,* 2009, vol. 106 (9), 3178-83
- **GARCIA-MARCOS et al.** *J Biol Chem.,* 2010, vol. 285, 12765-77
- **ENOMOTO et al.** *Dev Cell,* 2005, vol. 9 (3), 389-402
- **GHOSH et al.** *J Cell Biol,* 2008, vol. 182 (2), 381-93
- **ANAI et al.** *J Biol Chem,* 2005, vol. 280 (18), 18525-35
- **ENOMOTO et al.** *Ann N Y Acad Sci,* 2006, vol. 1086, 169-84
- **WENG et al.** *Cancer Sci.,* 2010, vol. 101, 836-42
- **GARCIA-MARCOS et al.** *Faseb J.,* 2011, vol. 25, 590-99
- Production of Polyclonal Antisera. **GREEN et al.** Immunochemical Protocols. Humana Press, 1992, 1-5
- **COLIGAN et al.** Production of Polyclonal Antisera in Rabbits, Rats, Mice and Hamsters. *Current Protocols in Immunology,* 1992

- **KOHLER ; MILSTEIN.** *Nature,* 1975, vol. 256, 495
- **COLIGAN ; HARLOW et al.** Antibodies: a Laboratory Manual. Cold Spring Harbor Pub, 1988, 726
- **CARSON.** Histotechology: A Self-Instructional Text. ASCP Press, 1997
- **E. W. MARTIN.** Remington's Pharmaceutical Sciences. Mack Publishing Co, 1975
- **COX.** *Journal of the Royal Statistical Society,* 1972, vol. 34 (2), 187-220
- **T MANIATIS ; E F FRITSCH ; J SAMBROOK.** Molecular Cloning. A Laboratory Manual. Cold Spring Harbor Laboratory, 1982
- **PEPE et al.** *Am J Epidemiol,* 2004, vol. 159, 882-90
- **LARSON ; SCHUMACHER.** *Biometrics,* 1992, vol. 48, 73-85
- **CHUNDONG et al.** *Anticancer Res,* 2011, vol. 31, 1141-5
- **LIU et al.** Percent-Only Metrics: using the percent staining only. Cutpoints are defined below each metric. *Mol Biol Rep,* 2012, vol. 39, 8717-22
- **PEPE.** *Am J Epidemiol,* 2004, vol. 159, 882-90
- **GRAY et al.** *J. Clin. Oncol.,* 2011, vol. 29, 4611-4619
- **VENOOK et al.** *J. Clin. Oncol.,* 2013, vol. 31, 1775-81
- **PEPE.** *J. Epidemiol.,* 2008, vol. 167, 362